# EUROPEAN PATENT APPLICATION

(11) **EP 3 150 637 A1**
(43) Date of publication of application: **05.04.2017**
(21) Application number: 15188060.6
(22) Date of filing: 02.10.2015
(51) Int. Cl.: C07K 16/46, C07K 16/22, C07K 16/24, C07K 16/28

(54) **MULTISPECIFIC ANTIBODIES**

(71) Applicant: F. Hoffmann-La Roche AG, 4070 Basel (CH)
(72) Inventor: The designation of the inventor has not yet been filed
(74) Representative: Burger, Alexander

(57) **Abstract**

The present disclosure relates to multispecific antibodies, methods for their production, pharmaceutical compositions containing said antibodies and uses thereof.

## Description

### FIELD OF THE INVENTION

The present invention relates to novel multispecific antibodies, methods for their production, pharmaceutical compositions containing said antibodies and uses thereof.

### BACKGROUND OF THE INVENTION

Engineered proteins, such as bi- or multispecific antibodies capable of binding two or more antigens are known in the art. Such multispecific binding proteins can be generated using cell fusion, chemical conjugation, or recombinant DNA techniques.

A wide variety of recombinant multispecific antibody formats have been developed in the recent past, e.g. tetravalent bispecific antibodies by fusion of, e.g. an IgG antibody format and single chain domains (see e.g. Coloma, M.J., et. al., Nature Biotech. 15 (1997) 159-163; WO 2001/077342; and Morrison, S.L., Nature Biotech. 25 (2007) 1233-1234).

Also several other new formats, wherein the antibody core structure (IgA, IgD, IgE, IgG or IgM) is no longer retained, have been developed; such as dia-, tria- or tetrabodies, minibodies and several single chain formats (scFv, Bis-scFv), which are capable of binding two or more antigens (Holliger, P., et. al, Nature Biotech. 23 (2005) 1126-1136; Fischer, N., and Léger, O., Pathobiology 74 (2007) 3-14; Shen, J., et. al., J. Immunol. Methods 318 (2007) 65-74; Wu, C., et al., Nature Biotech. 25 (2007) 1290-1297).

All such formats use linkers either to fuse the antibody core (IgA, IgD, IgE, IgG or IgM) to a further binding protein (e.g. scFv) or to fuse e.g. two Fab fragments or scFv (Fischer, N., and Léger, O., Pathobiology 74 (2007) 3-14). While it is obvious that linkers have advantages for the engineering of bispecific antibodies, they may also cause problems in therapeutic settings. Indeed, these foreign peptides might elicit an immune response against the linker itself or the junction between the protein and the linker. Furthermore, the flexible nature of these peptides makes them more prone to proteolytic cleavage, potentially leading to poor antibody stability, aggregation and increased immunogenicity. In addition one may want to retain effector functions, such as e.g. complement-dependent cytotoxicity (CDC) or antibody dependent cellular cytotoxicity (ADCC), which are mediated through the Fc-part by maintaining a high degree of similarity to naturally occurring antibodies.

Thus, ideally, one should aim at developing bispecific antibodies that are very similar in general structure to naturally occurring antibodies (like IgA, IgD, IgE, IgG or IgM) with minimal deviation from human sequences.

In one approach bispecific antibodies that are very similar to natural antibodies have been produced using the quadroma technology (see Milstein, C., and Cuello, A.C., Nature 305 (1983) 537-540) based on the somatic fusion of two different hybridoma cell lines expressing murine monoclonal antibodies with the desired specificities of the bispecific antibody. Because of the random pairing of two different antibody heavy and light chains within the resulting hybrid-hybridoma (or quadroma) cell line, up to ten different antibody species are generated of which only one is the desired, functional bispecific antibody. Due to the presence of mispaired byproducts, and significantly reduced production yields, sophisticated purification procedures are required (see e.g. Morrison, S.L., Nature Biotech. 25 (2007) 1233-1234). In general the same problem of mispaired byproducts remains if recombinant expression techniques are used.

An approach to circumvent the problem of mispaired byproducts, which is known as "knob-into-hole technology", aims at forcing the pairing of two different antibody heavy chains by introducing mutations into the CH3 domains to modify the contact interface. On one chain bulky amino acids were replaced by amino acids with short side chains to create a "hole". Conversely, amino acids with large side chains were introduced into the other CH3 domain, to create a "knob". By coexpressing these two heavy chains (and two identical light chains, which have to be appropriate for both heavy chains), high yields of heterodimer formation ("knob-hole") versus homodimer formation ("hole-hole" or "knob-knob") was observed (Ridgway, J.B., et al., Protein Eng. 9 (1996) 617-621; and WO 96/027011). The percentage of heterodimer could be further increased by remodeling the interaction surfaces of the two CH3 domains using a phage display approach and the introduction of a disulfide bridge to stabilize the heterodimers (Merchant, A.M., et al., Nature Biotech. 16 (1998) 677-681; Atwell, S., et al., J. Mol. Biol. 270 (1997) 26-35). New approaches for the knob-into-hole technology are described in e.g. in EP 1 870 459 A1. Although this format appears very attractive, no data describing progression towards the clinic are currently available. One important constraint of this strategy is that the light chains of the two parent antibodies have to be identical to prevent mispairing and formation of inactive molecules. Thus this technique is not appropriate as a basis for easily developing recombinant, tri-or tetraspecific antibodies against three or four antigens starting from two antibodies against the first and the second antigen, as either the heavy chains of these antibodies and/or the identical light chains have to be optimized first and then further antigen binding peptides against the third and fourth antigen have to be added.

WO 2006/093794 relates to heterodimeric protein binding compositions. WO 99/37791 describes multipurpose antibody derivatives. Morrison, S.L., et al., J. Immunol. 160 (1998) 2802-2808 refers to the influence of variable region domain exchange on the functional properties of IgG.

WO 2013/02362 relates to heterodimerized polypeptides. WO 2013/12733 relates to polypeptides comprising heterodimeric Fc regions. WO 2012/131555 relates to engineered hetero-dimeric immunoglobulins. EP 2647707 relates to engineered hetero-dimeric immunoglobulins.

WO 2009/080251, WO 2009/080252, WO 2009/080253, WO 2009/080254 and Schaefer, W. et al, PNAS, 108 (2011) 11187-1191 relate to bivalent, bispecific IgG antibodies with a domain crossover.

The multispecific antibodies with a CL-CH1 replacement in one binding arm (CrossMAb^{CH1-CL)}, which are described in WO 2009/080253 and Schaefer, W. et al, PNAS, 108 (2011) 11187-1191 clearly reduce the byproduct formation caused by a mismatch of a light chain of a first antibody that specifically binds to a first antigen with the wrong heavy chain of a second antibody that specifically binds to a second antigen (when compared to approaches without such domain exchanges). However their preparation is not completely free of side products The side product profile depends on the structure of the multispecific antibody with a CL-CH1 replacement in one binding arm. Additionally their thermal stability can be still improved

Therefore there is still a need for approaches to further reduce unwanted side products an improved purity and to improve thermal stability of multispecific antibodies.

### SUMMARY OF THE INVENTION

The present invention relates to a multispecific antibody, comprising a first light chain and a first heavy chain derived from a first antibody which specifically binds to a first antigen; and a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
- wherein in the constant domain CL of the first light chain the amino acid at position 124 (numbering according to Kabat) is substituted by an amino acid selected from K, R and H; and wherein in the constant domain CH1 of the first heavy chain the amino acid at position 147 (numbering according to EU index of Kabat) is substituted by an amino acid selected from E or D; or
- wherein in the constant domain CL of the second heavy chain the amino acid at position 124 (numbering according to Kabat) is by an amino acid selected from K, R and H; and wherein in the constant domain CH1 of the second light chain the amino acid at position 147 (numbering according to EU index of Kabat) is substituted by an amino acid selected from E or D.

One embodiment of the invention relates to a multispecific antibody,
- wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D, and wherein in the constant domain CL of the second heavy chain the amino acid at position 124 (numbering according to Kabat) is substituted by an amino acid selected from E and D; or
- wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D and wherein in the constant domain CL of the first light chain the amino acid at position 124 (numbering according to Kabat) is substituted by an amino acid selected from E and D.

Another aspect of the invention is a method for the preparation of a multispecific antibody according to the invention, comprising the steps of
- transforming a host cell with vectors comprising nucleic acids encoding
   a) the first light chain as defined for a multispecific antibody according to the invention derived from a first antibody which specifically binds to a first antigen;
   b) the first heavy chain as defined for a multispecific antibody according to the invention derived from a first antibody which specifically binds to a first antigen;
   c) the second light chain as defined for a multispecific antibody according to the invention derived from a second antibody which specifically binds to a second antigen; and
   d) the second heavy chain as defined for a multispecific antibody according to the invention derived from a second antibody which specifically binds to a second antigen,
- culturing said host cell under conditions that allow synthesis of said multispecific antibody; and
- recovering said multispecific antibody from said host cell culture.

Another aspect of the invention is a nucleic acid encoding the multispecific antibody according to the invention.

Another aspect of the invention is a vector comprising a nucleic acid according to the invention, wherein the vector is capable of expressing said nucleic acid in a host cell.

Another aspect of the invention is a host cell comprising a vector according to the invention.

Another aspect of the invention is a pharmaceutical composition comprising a multispecific antibody according to the invention in combination with at least one pharmaceutically acceptable carrier.

Another aspect of the invention is an immunoconjugate comprising the multispecific antibody according to the invention coupled to a cytotoxic agent.

Another aspect of the invention is the use of a multispecific antibody according to the invention for the manufacture of a pharmaceutical composition.

Another aspect of the invention is the multispecific antibody according to the invention for use as a medicament.

Another aspect of the invention is a pharmaceutical composition comprising a multispecific antibody according to the invention in combination with at least one pharmaceutically acceptable carrier for use as a medicament.

Another aspect of the invention is the use of a multispecific antibody according to the invention for the manufacture of a medicament.

Another aspect of the invention is a method of treatment of a patient suffering from a disease by administering a multispecific antibody according to the invention to the patient in the need of such treatment.

Another aspect of the invention is a method of generating a multispecific antibody comprising a first light chain and a first heavy chain derived from a first antibody which specifically binds to a first antigen; and a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain;
- the method comprising the selection of the antibody which is has the higher aggregation onset temperature (Tagg) ( and/or thermal stability)) out of both antibodies as the the second antibody and introduce the domain replacement
- the method further comprising the introduction of the following amino acid substitutions only in the the constant domain CH1 of the heavy chain and the constant domain CL of the light chain of the first antibody which is has the lower aggregation onset temperature (Tagg) ( and/or thermal stability)) :
   - in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D, and wherein in the constant domain CL of the second heavy chain the amino acid at position 124 (numbering according to Kabat) is substituted by an amino acid selected from E and D; or

According to the invention the formation of undesired side products during the production of the multispecific antibodies can be reduced/ and the thermal stability (e.g. aggregation onset temperatures) can be increase due to the introduction of oppositely charged amino acids at specific positions in the CH1 and CL domains. Thereby, the purity and stability of the desired multispecific antibody can be increased.

### DESCRIPTION OF THE FIGURES

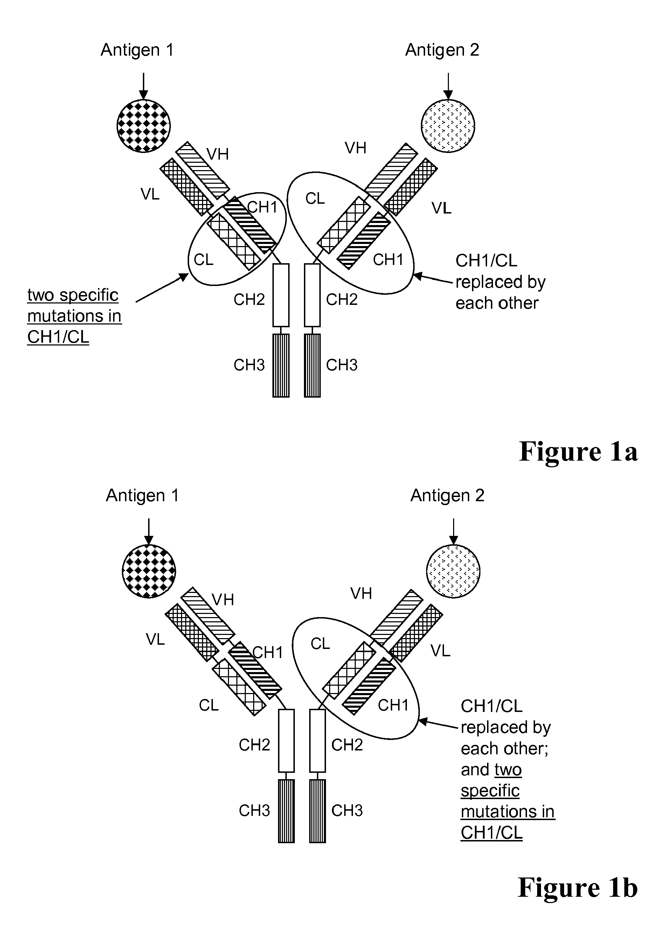

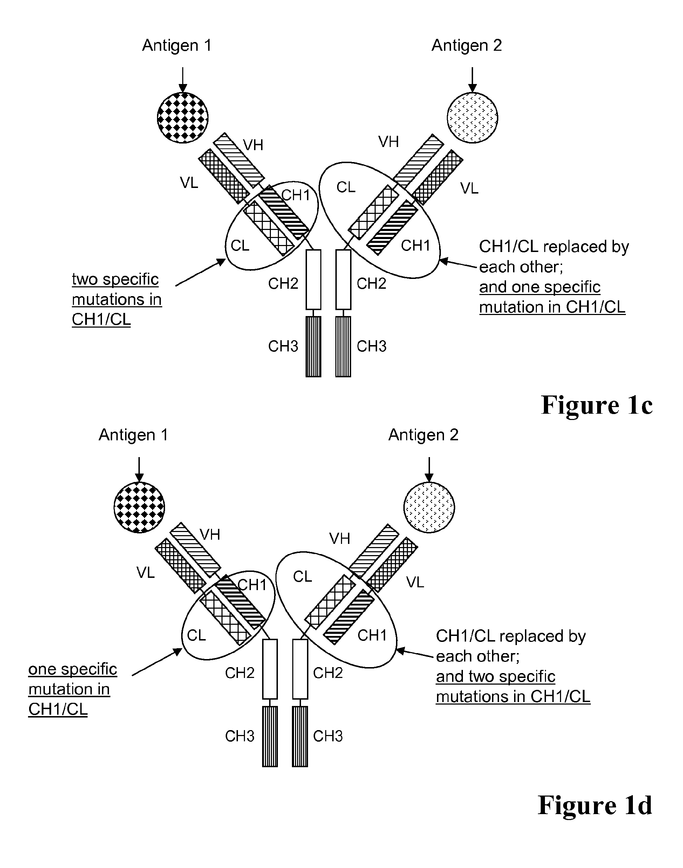

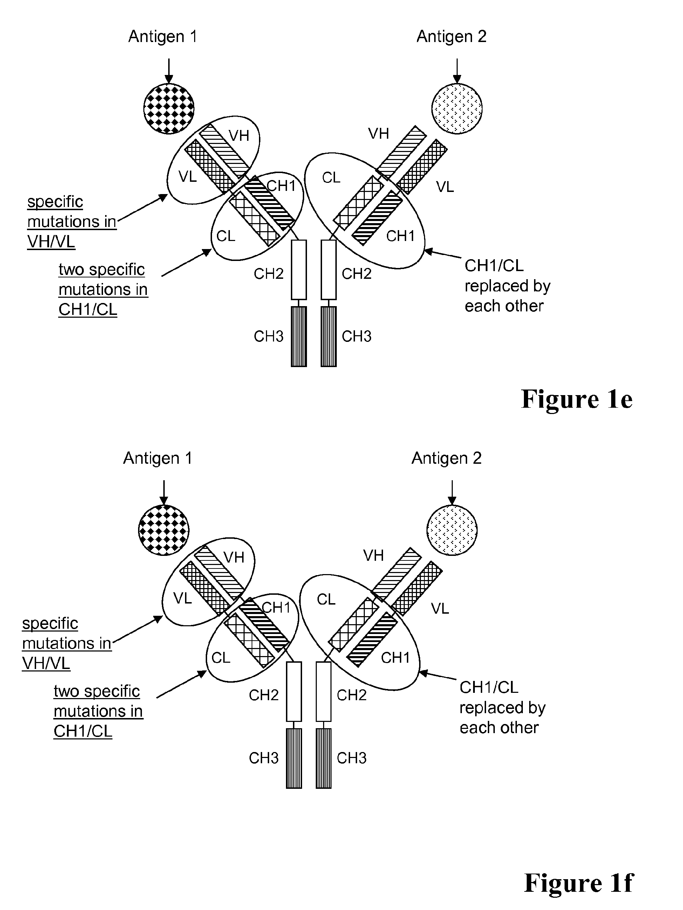

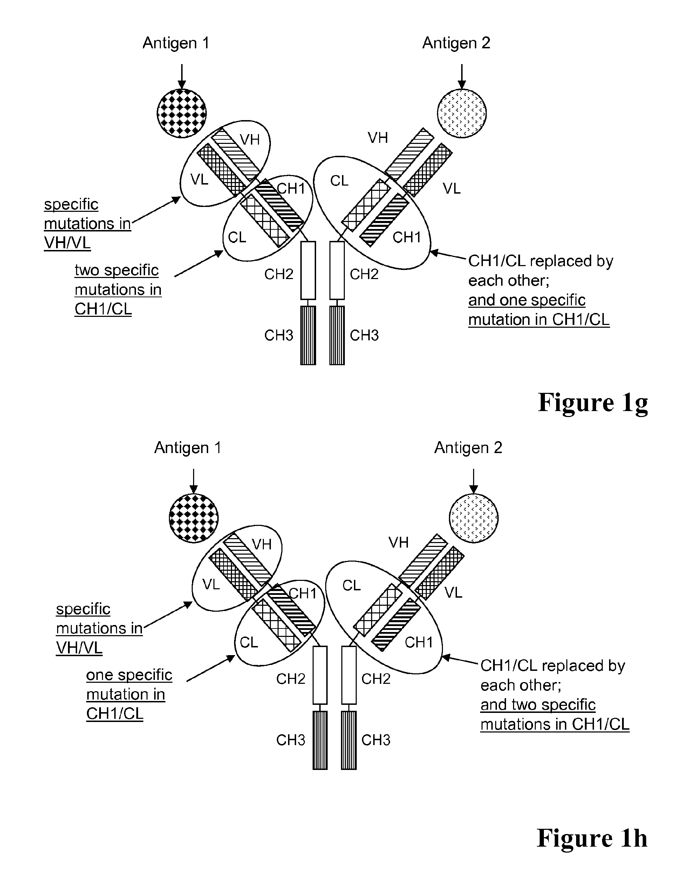

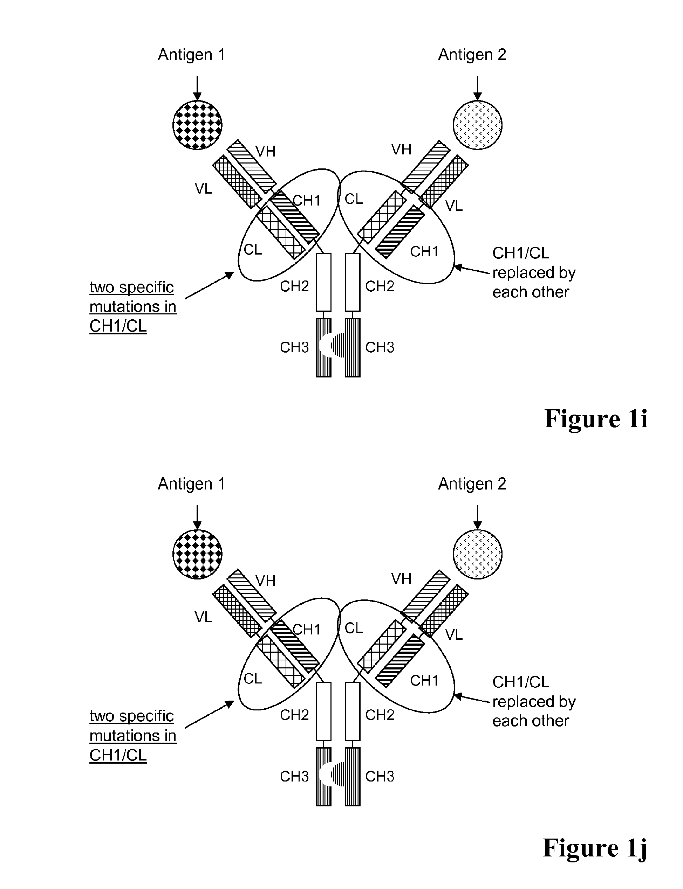

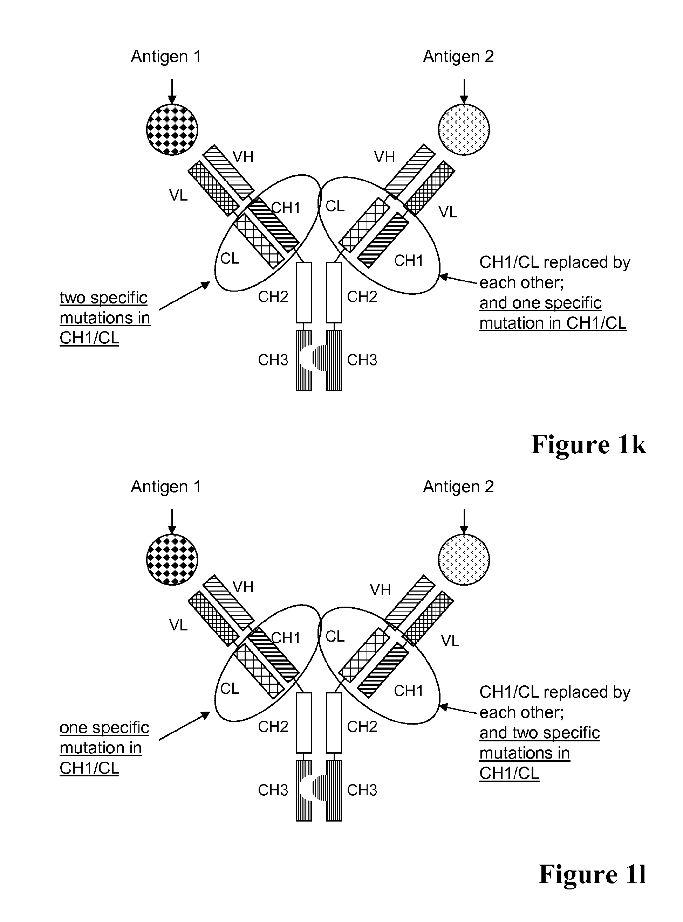

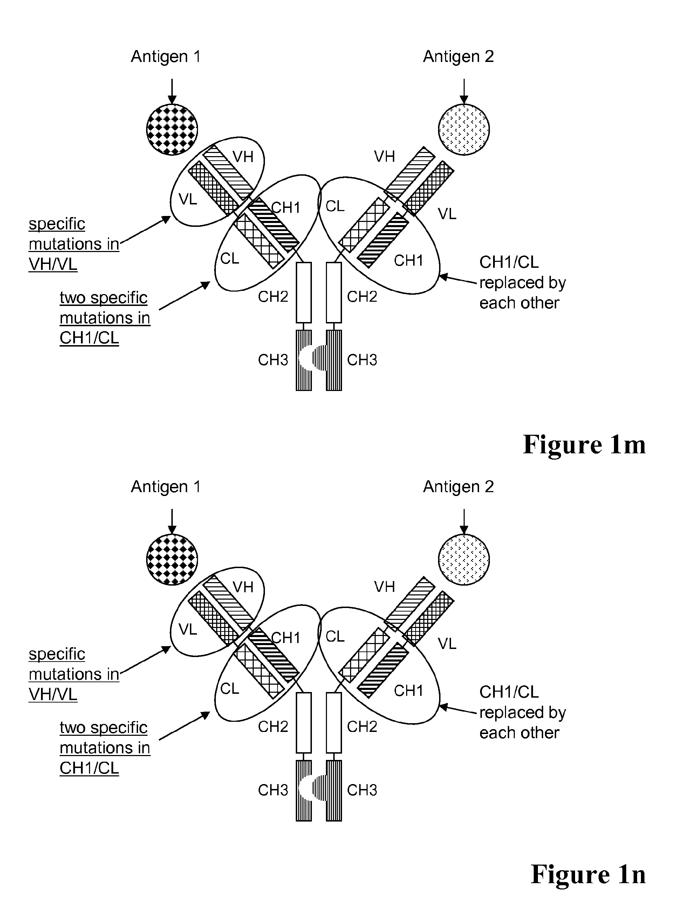

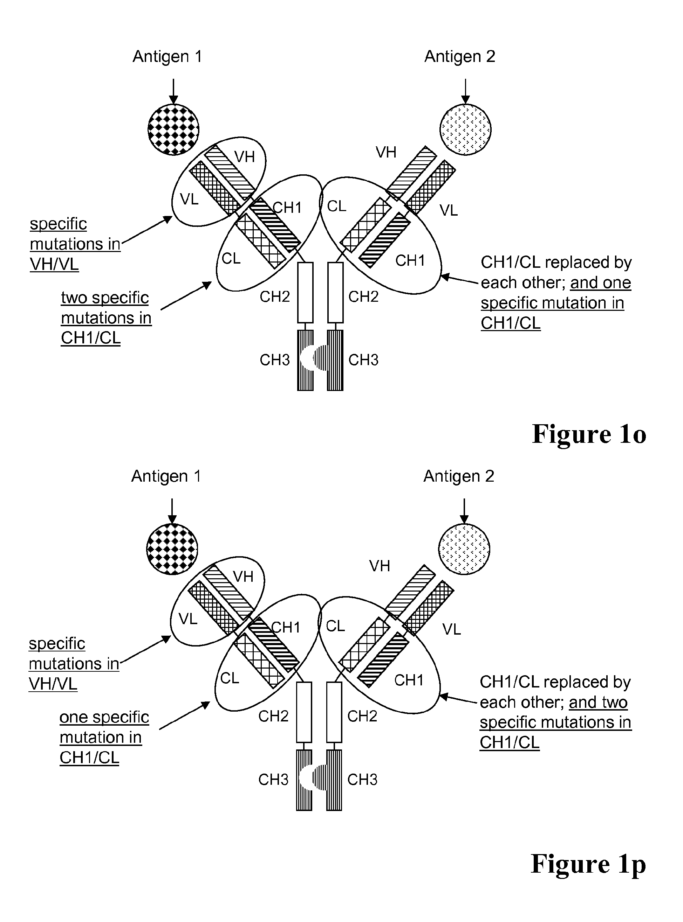

**Figure 1****:** Some examples of multispecific antibodies according to the invention with CL-CH1 domain exchange (CrossMAb^{CH1-CL}) in one antibody binding arm and specific amino acid substitutions in one CH1/CL domain interface:
   **Figure 1a****:** CL-CH1 domain exchange in one antibody binding arm and specific mutations in the CH1/CL domain interface of the other ("uncrossed") antibody binding arm.
   **Figure 1b****:** CL-CH1 domain exchange in one antibody binding arm and specific mutations in the CH1/CL domain interface of the same ("crossed") antibody binding arm.
   **Figure 1c****:** CL-CH1 domain exchange in one antibody binding arm with specific mutation in the same ("crossed") antibody binding arm and additional specific mutations in the CH1/CL domain interface of the other ("uncrossed") antibody binding arm.
   **Figure 1d****:** CL-CH1 domain exchange in one antibody binding arm and specific mutations in the CH1/CL domain interface of the same ("crossed") antibody binding arm with additional specific mutation in the CH1/CL domain interface of the other ("uncrossed") antibody binding arm.
   **Figure 1e****:** CL-CH1 domain exchange in one antibody binding arm and specific mutations in the CH1/CL domain interface of the other ("uncrossed") antibody binding arm and additional specific mutations in the VH/VL interface of both antibody binding arms.
   **Figure If:** CL-CH1 domain exchange in one antibody binding arm and specific mutations in the CH1/CL domain interface of the same ("crossed") antibody binding arm and additional specific mutations in the VH/VL interface of both antibody binding arms.
   **Figure 1g****:** CL-CH1 domain exchange in one antibody binding arm with specific mutation in the same ("crossed") antibody binding arm and additional specific mutations in the CH1/CL domain interface of the other ("uncrossed") antibody binding arm and further specific mutations in the VH/VL interface of both antibody binding arms.
   **Figure 1h****:** CL-CH1 domain exchange in one antibody binding arm with specific mutations in the CH1/CL domain interface of the same ("crossed") antibody binding arm with additional specific mutation in the CH1/CL domain interface of the other ("uncrossed") antibody binding arm and further specific mutations in the VH/VL interface of both antibody binding arms.
   **Figure 1i****:** CL-CH1 domain exchange in one antibody binding arm and specific mutations in the CH1/CL domain interface of the other ("uncrossed") antibody binding arm, and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1j****:** CL-CH1 domain exchange in one antibody binding arm and specific mutations in the CH1/CL domain interface of the same ("crossed") antibody binding arm, and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1k****:** CL-CH1 domain exchange in one antibody binding arm and specific mutations in the CH1/CL domain interface of the both antibody binding arms (double mutation on the "uncrossed" binding arm), and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1l****:** CL-CH1 domain exchange in one antibody binding arm and specific mutations in the CH1/CL domain interface of the both antibody binding arms (double mutation on the "crossed" binding arm), and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1m****:** CL-CH1 domain exchange in one antibody binding arm and specific mutations in the CH1/CL domain interface of the other ("uncrossed") antibody binding arm and additional specific mutations in the VH/VL interface of both antibody binding arms, and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1n**: CL-CH1 domain exchange in one antibody binding arm and specific mutations in the CH1/CL domain interface of the same ("crossed") antibody binding arm and additional specific mutations in the VH/VL interface of both antibody binding arms, and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1o****:** CL-CH1 domain exchange in one antibody binding arm and specific mutations in the CH1/CL domain interface of the both antibody binding arms (double mutation on the "uncrossed" binding arm) and additional specific mutations in the VH/VL interface of both antibody binding arms, and modifications of the CH3/CH3 domain interface to enforce heavy chain heterodimerization (like e.g. knob-into-hole technology or alternative heterodimerization technologies like e.g. substitution of charged amino acids with their respective opposite charge).
   **Figure 1p****:** CL-CH1 domain exchange in one antibody binding arm and specific mutations in the CH1/CL domain interface of the both antibody binding arms (double mutation on the "crossed" binding arm) and additional specific mutations in the VH/VL interface of both antibody binding arms, and modifications of the CH3/CH3 domain interface to enforce heavy chain heterodimerization (like e.g. knob-into-hole technology or alternative heterodimerization technologies like e.g. substitution of charged amino acids with their respective opposite charge).

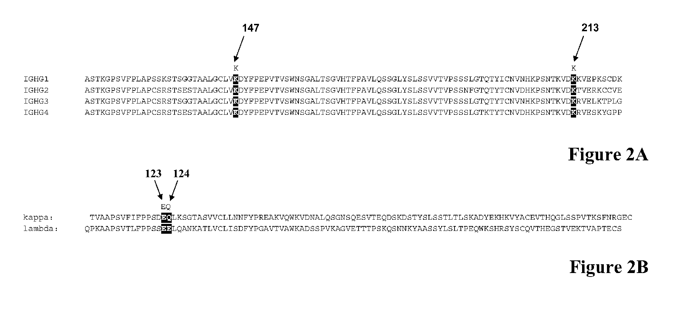

**Figure 2****:** Positions for amino acid substitutions in CH1 and CL domains
   **Figure 2A****:** wild type (wt) amino acid sequences in CH1 domain (four IgG isotypes are shown) with highlighted amino acid positions 147 and 213 (according to Kabat numbering).
   **Figure 2B****:** wild type (wt) amino acid sequences in the CL domain of kappa and lambda isotype with underlined and highlighted amino acid positions 123 and 124 (according to Kabat EU index numbering).

### DETAILED DESCRIPTION OF THE INVENTION

### I) DEFINITIONS

The terms "a", "an" and "the" generally include plural referents, unless the context clearly indicates otherwise.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

"Multispecific antibodies" bind two or more different epitopes (for example, two, three, four, or more different epitopes). The epitopes may be on the same or different antigens. An example of a multispecific antibody is a "bispecific antibody" which binds two different epitopes.

When an antibody possesses more than one specificity, the recognized epitopes may be associated with a single antigen or with more than one antigen.

The term "valent" as used herein denotes the presence of a specified number of binding sites in an antibody molecule. A natural antibody for example has two binding sites and is bivalent. As such, the term "trivalent" denotes the presence of three binding sites in an antibody molecule.

"Antibody specificity" refers to selective recognition of a particular epitope of an antigen by the antibody. Natural antibodies, for example, are monospecific. The term "monospecific antibody" as used herein denotes an antibody that has one or more binding sites each of which bind to the same epitope of the same antigen.

An epitope is a region of an antigen that is bound by an antibody. The term "epitope" includes any polypeptide determinant capable of specific binding to an antibody. In certain embodiments, epitope determinants include chemically active surface groupings of molecules such as amino acids, glycan side chains, phosphoryl, or sulfonyl, and, in certain embodiments, may have specific three dimensional structural characteristics, and/or specific charge characteristics. In certain embodiments, an antibody is said to specifically bind an antigen when it preferentially recognizes its target antigen in a complex mixture of proteins and/or macromolecules.

As used herein, the terms "binding" and "specific binding" refer to the binding of the antibody to an epitope of the antigen in an *in vitro* assay, preferably in a plasmon resonance assay (BIAcore, GE-Healthcare Uppsala, Sweden) with purified wild-type antigen.

The affinity of the binding of an antibody to an antigen is defined by the terms kₐ (rate constant for the association of the antibody from the antibody/antigen complex), k_{D} (dissociation constant), and K_{D} (k_{D}/ka). In one embodiment binding or that/which specifically binds to means a binding affinity (K_{D}) of 10⁻⁸ mol/l or less, in one embodiment 10⁻⁸ M to 10⁻¹³ mol/l. Thus, an multispecific antibody according to the invention specifically binds to each antigen for which it is specific with a binding affinity (K_{D}) of 10⁻⁸ mol/l or less, e.g. with a binding affinity (K_{D}) of 10⁻⁸ to 10⁻¹³ mol/l. in one embodiment with a binding affinity (K_{D}) of 10⁻⁹ to 10⁻¹³ mol/l.

Binding of the antibody to the FcγRIII can be investigated by a BIAcore assay (GE-Healthcare Uppsala, Sweden). The affinity of the binding is defined by the terms ka (rate constant for the association of the antibody from the antibody/antigen complex), k_{D} (dissociation constant), and K_{D} (k_{D}/ka).

The terms "monoclonal antibody" or "monoclonal antibody composition" as used herein refer to a preparation of antibody molecules of a single amino acid composition.

The term "chimeric" antibody refers to an antibody in which a portion of the heavy and/or light chain is derived from a particular source or species, while the remainder of the heavy and/or light chain is derived from a different source or species.

The term "humanized antibody" refers to antibodies in which the framework or the CDRs have been modified to comprise the CDR of an immunoglobulin of different specificity as compared to that of the parent immunoglobulin. In one preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Other forms of humanized antibodies encompassed by the present invention are those in which the constant region has been additionally modified or changed from that of the original antibody to generate the properties according to the invention, especially in regard to C1q binding and/or Fc receptor (FcR) binding.

The term "human antibody", as used herein, is intended to include antibodies having variable and constant regions derived from human germ line immunoglobulin sequences. Human antibodies are well-known in the state of the art (van Dijk, M.A., and van de Winkel, J.G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon antigen challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 33-40). Human antibodies can also be produced in phage display libraries (Hoogenboom, H.R., and Winter, G., J. Mol. Biol. 227 (1992) 381-388; Marks, J.D., et al., J. Mol. Biol. 222 (1991) 581-597). The techniques of Cole et al. and Boerner et al. are also available for the preparation of human monoclonal antibodies (Cole, et al., Monoclonal Antibodies and Cancer Therapy, Alan R. Liss, p. 77 (1985); and Boerner, P., et al., J. Immunol. 147 (1991) 86-95). As already mentioned for chimeric and humanized antibodies according to the invention the term "human antibody" as used herein also comprises such antibodies which are modified in the constant region to generate the properties according to the invention, especially in regard to C1q binding and/or FcR binding, e.g. by "class switching" i.e. change or mutation of Fc parts (e.g. from IgG1 to IgG4 and/or IgG1/IgG4 mutation).

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

The terms "binding site" or "antigen-binding site" as used herein denotes the region(s) of an antibody molecule to which a ligand (e.g. the antigen or antigen fragment of it) actually binds and which is derived from an antibody. The antigen-binding site includes antibody heavy chain variable domains (VH) and/or antibody light chain variable domains (VL), or pairs of VH/VL.

The antigen-binding sites that specifically bind to the desired antigen can be derived a) from known antibodies specifically binding to the antigen or b) from new antibodies or antibody fragments obtained by de novo immunization methods using inter alia either the antigen protein or nucleic acid or fragments thereof or by phage display.

An antigen-binding site of an antibody according to the invention can contain six complementarity determining regions (CDRs) which contribute in varying degrees to the affinity of the binding site for antigen. There are three heavy chain variable domain CDRs (CDRH1, CDRH2 and CDRH3) and three light chain variable domain CDRs (CDRL1, CDRL2 and CDRL3). The extent of CDR and framework regions (FRs) is determined by comparison to a compiled database of amino acid sequences in which those regions have been defined according to variability among the sequences. Also included within the scope of the invention are functional antigen binding sites comprised of fewer CDRs (i.e., where binding specificity is determined by three, four or five CDRs). For example, less than a complete set of 6 CDRs may be sufficient for binding. In some cases, a VH or a VL domain will be sufficient.

The "light chains" of antibodies (immunoglobulins) from any vertebrate species can be assigned to one of two distinct types, called kappa (κ) and lambda (λ), based on the amino acid sequences of their constant domains. A wild type light chain typically contains two immunoglobulin domains, usually one variable domain (VL) that is important for binding to an antigen and a constant domain (CL).

Several different types of "heavy chains" exist that define the class or isotype of an antibody. A wild type heavy chain contains a series of immunoglobulin domains, usually with one variable domain (VH) that is important for binding antigen and several constant domains (CH1, CH2, CH3, etc.).

The term "Fc domain" is used herein to define a C-terminal region of an immunoglobulin heavy chain that contains at least a portion of the constant region. For example in natural antibodies, the Fc domain is composed of two identical protein fragments, derived from the second and third constant domains of the antibody's two heavy chains in IgG, IgA and IgD isotypes; IgM and IgE Fc domains contain three heavy chain constant domains (CH domains 2-4) in each polypeptide chain. "Devoid of the Fc domain" as used herein means that the bispecific antibodies of the invention do not comprise a CH2, CH3 and CH4 domain; i.e. the constant heavy chain consists solely of one or more CH1 domains.

The "variable domains" or "variable region" as used herein denotes each of the pair of light and heavy chains which is involved directly in binding the antibody to the antigen. The variable domain of a light chain is abbreviated as "VL" and the variable domain of a light chain is abbreviated as "VH". The variable domains of human light chains and heavy chains have the same general structure. Each variable domain comprises four framework (FR) regions, the sequences of which are widely conserved. The FR are connected by three "hypervariable regions" (or "complementarity determining regions", CDRs). CDRs on each chain are separated by such framework amino acids. Therefore, the light and heavy chains of an antibody comprise from N- to C-terminal direction the domains FR1, CDR1, FR2, CDR2, FR3, CDR3, and FR4. The FR adopt a β-sheet conformation and the CDRs may form loops connecting the β-sheet structure. The CDRs in each chain are held in their three-dimensional structure by the FR and form together with the CDRs from the other chain an "antigen binding site". Especially, CDR3 of the heavy chain is the region which contributes most to antigen binding. CDR and FR regions are determined according to the standard definition of Kabat, et al., Sequences of Proteins of Immunological Interest, 5th ed., Public Health Service, National Institutes of Health, Bethesda, MD (1991).

The term "constant domains" or "constant region" as used within the current application denotes the sum of the domains of an antibody other than the variable region. The constant region is not directly involved in binding of an antigen, but exhibits various effector functions.

Depending on the amino acid sequence of the constant region of their heavy chains, antibodies are divided in the classes: IgA, IgD, IgE, IgG and IgM, and several of these may are further divided into subclasses, such as IgG1, IgG2, IgG3, and IgG4, IgA1 and IgA2. The heavy chain constant regions that correspond to the different classes of antibodies are called α, δ, ε, γ and µ, respectively, respectively. The light chain constant regions (CL) which can be found in all five antibody classes are called κ (kappa) and λ (lambda). The "constant domains" as used herein are from human origin, which is from a constant heavy chain region of a human antibody of the subclass IgG1, IgG2, IgG3, or IgG4 and/or a constant light chain kappa or lambda region. Such constant domains and regions are well known in the state of the art and e.g. described by Kabat, et al., Sequences of Proteins of Immunological Interest, 5th ed., Public Health Service, National Institutes of Health, Bethesda, MD (1991).

The term "tertiary structure" as used herein refers to the geometric shape of the antibody according to the invention. The tertiary structure comprises a polypeptide chain backbone comprising the antibody domains, while amino acid side chains interact and bond in a number of ways.

The term "amino acid" as used herein denotes an organic molecule possessing an amino moiety located at α-position to a carboxylic group. Examples of amino acids include: arginine, glycine, ornithine, lysine, histidine, glutamic acid, asparagic acid, isoleucine, leucine, alanine, phenylalanine, tyrosine, tryptophane, methionine, serine, proline. The amino acid employed is optionally in each case the L-form. The term "positively charged" or "negatively charged" amino acid refers to the amino acid side-chain charge at pH 7.4. Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

**Table - Amino acids with specific properties**

| **Amino Acid** | **3-Letter** | **1-Letter** | **Side-chain polarity** | **Side-chain charge (pH 7.4)** |
|---|---|---|---|---|
| Alanine | Ala | A | nonpolar | neutral |
| Arginine | Arg | R | basic polar | positive |
| Asparagine | Asn | N | polar | neutral |
| Aspartic acid | Asp | D | acidic polar | negative |
| Cysteine | Cys | C | nonpolar | neutral |
| Glutamic acid | Glu | E | acidic polar | negative |
| Glutamine | Gln | Q | polar | neutral |
| Glycine | Gly | G | nonpolar | neutral |
| Histidine | His | H | basic polar | positive (10%) |
| | | | | neutral (90%) |
| Isoleucine | Ile | I | nonpolar | neutral |
| Leucine | Leu | L | nonpolar | neutral |
| Lysine | Lys | K | basic polar | positive |
| Methionine | Met | M | nonpolar | neutral |
| Phenylalanine | Phe | F | nonpolar | neutral |
| Proline | Pro | P | nonpolar | neutral |
| Serine | Ser | S | polar | neutral |
| Threonine | Thr | T | polar | neutral |
| Tryptophan | Trp | W | nonpolar | neutral |
| Tyrosine | Tyr | Y | polar | neutral |
| Valine | Val | V | nonpolar | neutral |

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991), which is referred to herein as "numbering according to Kabat et al.". In particular, for variable domains and for the light chain constant domain CL of kappa and lambda isotype, the Kabat numbering system (see pages 647-660) of Kabat, et al., Sequences of Proteins of Immunological Interest, 5th ed., Public Health Service, National Institutes of Health, Bethesda, MD (1991) is used and is herein referred to as "numbering according to Kabat" and, nonwithstanding this, for the constant heavy chain domains (CH1, Hinge, CH2 and CH3) the Kabat EU index numbering system (see pages 661-723) is used and is herein referred to as "numbering according to EU index of Kabat".

Amino acid substitutions (or mutations) within the polypeptide chains of the multispecific antibody are prepared by introducing appropriate nucleotide changes into the antibody DNA, or by nucleotide synthesis. Such modifications can be performed, however, only in a very limited range, e.g. as described above. For example, the modifications do not alter the above mentioned antibody characteristics such as the IgG isotype and antigen binding, but may further improve the yield of the recombinant production, protein stability or facilitate the purification. In certain embodiments, antibody variants having one or more conservative amino acid substitutions are provided.

The antibody according to the invention is produced by recombinant means. Methods for recombinant production of antibodies are widely known in the state of the art and comprise protein expression in prokaryotic and eukaryotic cells with subsequent isolation of the antibody and usually purification to a pharmaceutically acceptable purity. For the expression of the antibodies as aforementioned in a host cell, nucleic acids encoding the respective (modified) light and heavy chains are inserted into expression vectors by standard methods. Expression is performed in appropriate prokaryotic or eukaryotic host cells, like CHO cells, NS0 cells, SP2/0 cells, HEK293 cells, COS cells, PER.C6 cells, yeast, or E. coli cells, and the antibody is recovered from the cells (supernatant or cells after lysis). General methods for recombinant production of antibodies are well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

"Polynucleotide" or "nucleic acid" as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. A sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR2 ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a vector are capable of directing the expression of nucleic acids to which they are operatively linked. When the expression vector is introduced into an appropriate host cell, it can be transcribed and translated into a polypeptide. When transforming host cells in methods according to the invention, "expression vectors" are used; thereby the term "vector" in connection with transformation of host cells as described herein means "expression vector". An "expression system" usually refers to a suitable host cell comprised of an expression vector that can function to yield a desired expression product.

As used herein, "expression" refers to the process by which a nucleic acid is transcribed into mRNA and/or to the process by which the transcribed mRNA (also referred to as transcript) is subsequently being translated into peptides, polypeptides, or proteins. The transcripts and the encoded polypeptides are collectively referred to as gene product. If the polynucleotide is derived from genomic DNA, expression in a eukaryotic cell may include splicing of the mRNA.

The term "transformation" as used herein refers to process of transfer of a vectors/nucleic acid into a host cell. If cells without formidable cell wall barriers are used as host cells, transfection is carried out e.g. by the calcium phosphate precipitation method as described by Graham and Van der Eh, Virology 52 (1978) 546ff. However, other methods for introducing DNA into cells such as by nuclear injection or by protoplast fusion may also be used. If prokaryotic cells or cells which contain substantial cell wall constructions are used, e.g. one method of transfection is calcium treatment using calcium chloride as described by Cohen, F.N, et al., PNAS 69 (1972) 7110 et seq.

The term "host cell" as used in the current application denotes any kind of cellular system which can be engineered to generate the antibodies according to the current invention.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included. Where distinct designations are intended, it will be clear from the context.

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; and Norderhaug, L., et al., J. Immunol. Methods 204 (1997) 77-87. A preferred transient expression system (HEK 293) is described by Schlaeger, E.-J., and Christensen, K., in Cytotechnology 30 (1999) 71-83 and by Schlaeger, E.-J., J. Immunol. Methods 194 (1996) 191-199.

Antibodies produced by host cells may undergo post-translational cleavage of one or more, particularly one or two, amino acids from the C-terminus of the heavy chain. Therefore an antibody produced by a host cell by expression of a specific nucleic acid molecule encoding a full-length heavy chain may include the full-length heavy chain, or it may include a cleaved variant of the full-length heavy chain (also referred to herein as a cleaved variant heavy chain). This may be the case where the final two C-terminal amino acids of the heavy chain are glycine (G446) and lysine (K447, numbering according to Kabat EU index).

Therefore, amino acid sequences of heavy chains including CH3 domains are denoted herein without C-terminal glycine-lysine dipeptide if not indicated otherwise.

Compositions of the invention, such as the pharmaceutical compositions described herein, comprise a population of antibodies of the invention. The population of antibodies may comprise antibodies having a full-length heavy chain and antibodies having a cleaved variant heavy chain. In one embodiment, the population of antibodies consists of a mixture of antibodies having a full-length heavy chain and antibodies having a cleaved variant heavy chain, wherein at least 50%, at least 60%, at least 70%, at least 80% or at least 90% of the antibodies have a cleaved variant heavy chain.

Purification of antibodies (recovering the antibodies from the host cell culture) is performed in order to eliminate cellular components or other contaminants, e.g. other cellular nucleic acids or proteins, by standard techniques, including alkaline/SDS treatment, CsCI banding, column chromatography, agarose gel electrophoresis, and others well known in the art. See Ausubel, F., et al., ed. Current Protocols in Molecular Biology, Greene Publishing and Wiley Interscience, New York (1987). Different methods are well established and widespread used for protein purification, such as affinity chromatography with microbial proteins (e.g. protein A or protein G affinity chromatography), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis) (Vijayalakshmi, M.A., Appl. Biochem. Biotech. 75 (1998) 93-102).

The term "pharmaceutical composition" refers to a preparation which is in such form as to permit the biological activity of an active ingredient contained therein to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. To administer an antibody according to the invention by certain routes of administration, it may be necessary to coat the antibody with, or co-administer the antibody with, a material to prevent its inactivation. For example, the antibody may be administered to a subject in an appropriate carrier, for example, liposomes, or a diluent. Pharmaceutically acceptable diluents include saline and aqueous buffer solutions.

A "pharmaceutically acceptable carrier" refers to an ingredient in a pharmaceutical formulation, other than an active ingredient, which is nontoxic to a subject. Pharmaceutically acceptable carriers includes any and all solvents, dispersion media, coatings, antibacterial and antifungal agents, isotonic and absorption delaying agents, and the like that are physiologically compatible. In one preferred embodiment, the carrier is suitable for intravenous, intramuscular, subcutaneous, parenteral, spinal or epidermal administration (e.g. by injection or infusion).

The pharmaceutical compositions according to the invention may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The composition must be sterile and fluid to the extent that the composition is deliverable by syringe. In addition to water, in one embodiment the carrier is an isotonic buffered saline solution.

Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition.

An "immunoconjugate" is an antibody conjugated to one or more heterologous molecule(s), including but not limited to a cytotoxic agent.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

The term "cancer" as used herein refers to proliferative diseases, such as lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, including refractory versions of any of the above cancers, or a combination of one or more of the above cancers.

"Human VEGF" as used herein refers to human vascular endothelial growth factor (VEGF/VEGF-A) which is described in e.g. Leung, D.W., et al., Science 246 (1989) 1306-9; Keck, P.J., et al., Science 246 (1989) 1309-12 and Connolly, D.T., et al., J. Biol. Chem. 264 (1989) 20017-24. VEGF is involved in the regulation of normal and abnormal angiogenesis and neovascularization associated with tumors and intraocular disorders (Ferrara, N., et al., Endocr. Rev. 18 (1997) 4-25; Berkman, R.A., et al., J. Clin. Invest. 91 (1993) 153-159; Brown, L.F., et al., Human Pathol. 26 (1995) 86-91; Brown, L.F., et al., Cancer Res. 53 (1993) 4727-4735; Mattern, J., et al., Brit. J. Cancer. 73 (1996) 931-934; and Dvorak, H., et al., Am. J. Pathol. 146 (1995) 1029-1039). VEGF is a homodimeric glycoprotein that has been isolated from several sources. VEGF shows highly specific mitogenic activity for endothelial cells.

Human "ANG-2" as used herein refers to human angiopoietin-2 (ANG-2) (alternatively abbreviated with ANGPT2 or ANG2) which is described in Maisonpierre, P.C., et al, Science 277 (1997) 55-60 and Cheung, A.H., et al., Genomics 48 (1998) 389-91. The angiopoietins-1 and -2 were discovered as ligands for the Ties, a family of tyrosine kinases that is selectively expressed within the vascular endothelium (Yancopoulos, G.D., et al., Nature 407 (2000) 242-48). There are now four definitive members of the angiopoietin family. Angiopoietin-3 and -4 (Ang-3 and Ang-4) may represent widely diverged counterparts of the same gene locus in mouse and man (Kim, I., et al., FEBS Let, 443 (1999) 353-56; Kim, I., et al., J Biol Chem 274 (1999) 26523-28).

Human TWEAK (UniProtKB 043508, TNF-related weak inducer of apoptosis) is a cell surface associated type II transmembrane protein. TWEAK is described in Chicheportiche, Y., et al., J. Biol. Chem. 272 (1997) 32401-32410; Marsters, S.A., et al., Curr. Biol. 8 (1998) 525-528; Lynch, C.N., et al., J. Biol. Chem. 274 (1999) 8455-8459. The active form of TWEAK is a soluble homotrimer. Human and murine TWEAK show 93 % sequence identity in receptor binding domain. The TWEAK receptor Fn14 (fibroblast growth factor inducible 14 kDa protein) is a 129 aa type I transmembrane protein consisting of one single cysteine rich domain in ligand binding domain. Signaling of TWEAK occurs via NF-KB pathway activation. TWEAK mRNA is expressed in a variety of tissues and found in most major organs like heart, brain, skeletal muscle, and pancreas, tissues related to the immune system like spleen, lymph nodes, and thymus. Fn14 mRNA has been detected in heart, brain, lung, placenta, vascular EC and smooth muscle cells. TWEAK-null and Fnl4-null knockout mice are viable, healthy and fertile and have more natural killer cells and display an enhanced innate inflammatory response. TWEAK is involved in apoptosis, proliferation, angiogenesis, ischemic penumbra, cerebral edema, multiple sclerosis.

Human IL-17 (also named IL17-A; CTLA-8, Swiss Prot Q16552, IL17) is a pro-inflammatory cytokine produced by a subset of memory T cells (called Th17) that has been implicated e.g. in the pathogenesis of multiple sclerosis. IL-17A plays a role in the induction of other inflammatory cytokines, chemokines and adhesion molecules. Treatment of animals with IL-17A neutralizing antibodies decreases disease incidence and severity in autoimmune encephalomyelitis (Komiyama, Y. et al., J. Immunol. 177 (2006) 566-573). IL-17A is over-expressed in the cerebrospinal fluid of MS patients (Hellings, P.W. et al., Am. J. Resp. Cell Mol. Biol. 28 (2003) 42-50; Matusevicius, D. et al., Multiple Sclerosis 5 (1999) 101-104; WO 2005/051422). In addition, IL-17A neutralizing antibodies reduce severity and incidence of mouse rheumatoid arthritis model of collagen induced arthritis, and high levels of IL-17A can be detected in the synovial fluid of inflamed joints from RA patients (Ziolkowska, M. et al., J. Immunol. 164 (2000) 2832-2838; Kotake, S., et al., J. Clin. Invest. 103 (1999) 1345-1352; Hellings, P.W. et al., Am. J. Resp. Cell Mol. Biol. 28 (2003) 42-50).

### II) DETAILED DESCRIPTION OF THE EMBODIMENTS OF THE INVENTION

Multispecific antibodies with a CL-CH1 replacement in one binding arm (CrossMab^{CH1-CL}) are described in detail in WO 2009/080253 and Schaefer, W. et al, PNAS, 108 (2011) 11187-1191 (which are incorporated as reference herein). With these antibodies, the byproduct formation caused by a mismatch of a light chain of a first antibody that specifically binds to a first antigen with the wrong heavy chain of a second antibody that specifically binds to a second antigen can be considerably reduced (when compared to approaches without such domain exchanges). However their preparation is not completely free of side products. The side product profile depends on the structure of the multispecific antibody with a CL-CH1 replacement/exchange in one binding arm. Furthermore sometimes the CL-CH1 replacement/exchange in one binding arm leads to slightly reduce the aggregation temperature (an important feature for production, purification and long term stability).

The invention provides an approach to further reduce the formation of unwanted side products and/or to increase the aggregation temperature by substituting particular amino acids in the CH1 and CL domains by oppositely charged amino acids, respectively.. Additionally substituting a particular amino acid in the CL domain of the multispecific antibodies might be also of further benefit. Additionally substituting particular amino acids in the VH and VL domains of the multispecific antibodies might be also of further benefit.

Therefore, the invention relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid; or
   ii) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

In accordance with the concept of the invention, the antibody according to the invention comprises only one of the modifications as indicated under i) and ii) above and below. Furthermore the accordance with the concept of the invention, the antibody according to the invention does not include an additional VH/VL exchange in the binding arm with domain replacement/exchange.

Hence, the multispecific antibody according to the invention comprises
a) a first light chain and a first heavy chain derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain of the second light chain and the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid;
      or
   ii) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid,
with the proviso that the multispecific antibody does not comprise both of the modifications mentioned under i) and ii) and the proviso that in the second light chain the variable domain VL is not replaced by the variable domain VH of the second heavy chain; and in the second heavy chain the variable domain VH is not replaced by the variable domain VL of the second light chain.

In one embodiment, the invention relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid.
      (with the proviso that in the second light chain the variable domain VL is not replaced by the variable domain VH of the second heavy chain; and in the second heavy chain the variable domain VH is not replaced by the variable domain VL of the second light chain).

In one embodiment, the invention relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

In one embodiment of the invention, the negatively charged amino acid is selected from E and D. In one embodiment of the invention, the negatively charged amino acid is E.

In one embodiment of the invention, the positively charged amino acid is selected from K, R and H. In one embodiment of the invention, the positively charged amino acid is selected from K and R. In one embodiment of the invention, the positively charged amino acid is K.

The invention also relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D; or
   ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D.

In one embodiment, the invention relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D.

In one embodiment, the invention relates to a multispecific antibody, comprising
a) a first light chain and a first heavy chain derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D.

In one embodiment of the invention, in the constant domain CL the amino acids at position 124 and 123 (numbering according to Kabat) are substituted independently from each other by an amino acid selected from K and R.

In one embodiment of the invention, in the constant domain CL the amino acids at position 124 and 123 (numbering according to Kabat) are substituted by K.

In one embodiment of the invention, in the constant domain CH1 the amino acids at position 147 and 213 (numbering according to EU index of Kabat) are substituted by E.

In one embodiment of the invention, in the constant domain CL the amino acids at position 124 and 123 (numbering according to Kabat) are substituted independently from each other by an amino acid selected from K and R, and in the constant domain CH1 the amino acids at position 147 and 213 (numbering according to EU index of Kabat) are substituted independently from each other by an amino acid selected from E or D.

In one embodiment of the invention, in the constant domain CL the amino acids at position 124 and 123 (numbering according to Kabat) are substituted by K, and in the constant domain CH1 the amino acids at position 147 and 213 (numbering according to EU index of Kabat) are substituted by E.

In one embodiment of the invention, in the constant domain CL of the first light chain the amino acids at position 124 and 123 (numbering according to Kabat) are substituted independently from each other by an amino acid selected from K and R.

In one embodiment of the invention, in the constant domain CL of the first light chain the amino acids at position 124 and 123 (numbering according to Kabat) are substituted by K.

In one embodiment of the invention, in the constant domain CH1 of the first heavy chain the amino acids at position 147 and 213 (numbering according to EU index of Kabat) are substituted by E.

In one embodiment of the invention, in the constant domain CL of the first light chain the amino acids at position 124 and 123 (numbering according to Kabat) are substituted independently from each other by an amino acid selected from K and R, and in the constant domain CH1 of the first heavy chain the amino acids at position 147 and 213 (numbering according to EU index of Kabat) are substituted independently from each other by an amino acid selected from E or D.

In one embodiment of the invention, in the constant domain CL of the first light chain the amino acids at position 124 and 123 (numbering according to Kabat) are substituted by K, and in the constant domain CH1 of the first heavy chain the amino acids at position 147 and 213 (numbering according to EU index of Kabat) are substituted by E.

In one embodiment of the invention, in the constant domain CL of the second heavy chain the amino acids at position 124 and 123 (numbering according to Kabat) are substituted independently from each other by an amino acid selected from K and R.

In one embodiment of the invention, in the constant domain CL of the second heavy chain the amino acids at position 124 and 123 (numbering according to Kabat) are substituted by K.

In one embodiment of the invention, in the constant domain CH1 of the second light chain the amino acids at position 147 and 213 (numbering according to EU index of Kabat) are substituted by E.

In one embodiment of the invention, in the constant domain CL of the second heavy chain the amino acids at position 124 and 123 (numbering according to Kabat) are substituted independently from each other by an amino acid selected from K and R, and in the constant domain CH1 of the second light chain the amino acids at position 147 and 213 (numbering according to EU index of Kabat) are substituted independently from each other by an amino acid selected from E or D.

In one embodiment of the invention, in the constant domain CL of the second heavy chain the amino acids at position 124 and 123 (numbering according to Kabat) are substituted by K, and in the constant domain CH1 of the second light chain the amino acids at position 147 and 213 (numbering according to EU index of Kabat) are substituted by E.

The "light chain of an antibody" as used herein is a polypeptide comprising in N-terminal to C-terminal direction an antibody light chain variable domain (VL), and an antibody light chain constant domain (CL), abbreviated as VL-CL. The "heavy chain of an antibody" as used herein is a polypeptide comprising in N-terminal to C-terminal direction an antibody heavy chain variable domain (VH) and an antibody constant heavy chain domain 1 (CH1). In one preferred embodiment of the invention a heavy chain of an antibody comprises in N-terminal to C-terminal direction an antibody heavy chain variable domain (VH), an antibody constant heavy chain domain 1 (CH1), an antibody heavy chain constant domain 2 (CH2) and an antibody heavy chain constant domain 3 (CH3), abbreviated as VH-CH1- CH2-CH3.

Thus, in a multispecific antibody according to the invention in said first light chain derived from said first antibody the sequential arrangement of the domains of the light chain (CL-VL) remains unaltered. In said first heavy chain derived from said first antibody the sequential arrangement of the domains of the heavy chain (CH1-CL or CH3-CH2-CH1-CL) remains unaltered. Because the Fab region derived from said first antibody does not include a domain crossover, this region is herein also referred to as "non-crossed Fab region" of the multispecific antibody according to the invention.

In one embodiment of the invention, the multispecific antibody comprises a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH and CH1 domains (i.e. VH-CH1 from N-terminal to C-terminal direction), wherein the first light chain and the first heavy chain are derived from a first antibody which specifically binds to a first antigen.

In one embodiment of the invention, the multispecific antibody comprises a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH, CH1, CH2 and CH3 domains (i.e. VH-CH1-CH2-CH3 from N-terminal to C-terminal direction), wherein the first light chain and the first heavy chain are derived from a first antibody which specifically binds to a first antigen.

In contrast, within said second light chain (which is herein also referred to as "modified second light chain" or "LC*") the original constant domain CL is replaced by the constant domain CH1 of the (original) second heavy chain. Therefore, the modified second light chain is built up of the original variable domain VL of the second light chain and the CH1 domain of the second heavy chain derived from said second antibody. In consequence, the sequential arrangement of the light chain domains of said modified second light chain is VL-CH1 (in N-terminal to C-terminal direction).

Additionally, within said second heavy chain (which is herein also referred to as "modified second heavy chain" or "HC*") the original constant domain CH1 is replaced by the constant domain CL of the (original) second light chain. Therefore, the modified second heavy chain comprises at least a sequential arrangement of the original variable domain VH of the second heavy chain and of the CL domain of the second light chain derived from said second antibody. In consequence, the sequential arrangement of the heavy chain domains of said modified second heavy chains is at least VH-CL (in N-terminal to C-terminal direction).

In summary, within said second heavy chain and said second light chain derived from said second antibody the constant domains CL and CH1 are replaced by each other. Because the Fab region derived from said second antibody includes a CH1-CL domain crossover, as described above, this region is herein also referred to as "crossed CH1-CL Fab region" of the multispecific antibody according to the invention. Thus, the multispecific antibody according to the invention includes at least one non-crossed Fab region and at least one crossed CH1-CL Fab region.

In one embodiment of the invention the modified heavy chain of a multispecific antibody according to the invention consists of a sequential arrangement (N-terminal to C-terminal direction) of the VH and CL domains of the second light chain (however particular amino acid substitutions in said VH and CL domains as described for the invention are possible). Thus, in this embodiment the multispecific antibody comprises at least two Fab fragments, including a first Fab fragment derived from said first antibody (non-crossed Fab fragment) and a second Fab fragment derived from said second antibody (crossed CH1-CL Fab fragment), wherein the multispecific antibody according to this embodiment does not comprise the respective Fc domains of said first and said second antibody (hence, the multispecific antibody is devoid of an Fc domain). In one embodiment, a multispecific antibody comprises two to five Fab fragments. In one embodiment of a multispecific antibody the Fab fragments are connected with each other via a peptide linker. The term "peptide linker" as used herein denotes a peptide with amino acid sequences, which is preferably of synthetic origin. In one embodiment a peptide linker is used to connect one of the Fab fragments to the C- or N-terminus of the other Fab fragment in order to form a multispecific antibody according to the invention. In one preferred embodiment said peptide linker is a peptide with an amino acid sequence with a length of at least 5 amino acids, in one embodiment with a length of 5 to 100, in a further embodiment of 10 to 50 amino acids. In one embodiment said peptide linker is (GxS)ₙ or (GxS)ₙGₘ with G = glycine, S = serine, and (x = 3, n= 3, 4, 5 or 6, and m= 0, 1, 2 or 3) or (x=4, n=2, 3, 4 or 5 and m= 0, 1, 2 or 3), in one embodiment x=4 and n=2 or 3, in a further embodiment x=4 and n=2. In one embodiment said peptide linker is (G₄S)₂. The peptide linker is used to connect the first and the second Fab fragment. In one embodiment the first Fab fragment is connected to the C- or N- terminus of the second Fab fragment. Multispecific antibodies of this format have been previously described in WO 2013/026835.

In one embodiment of the invention, the multispecific antibody comprises a first light chain and a first heavy chain derived from a first antibody which specifically binds to said first antigen and a third antigen. This embodiments includes the variable light chain domain and the variable heavy chain domain in a so called dual-acting Fab, as described previously (WO 2013/174873).

In one embodiment of the invention, the multispecific antibody is devoid of an Fc domain and comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH and CH1 domains (i.e. VH-CH1 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid; or
   ii) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

In one embodiment of the invention, the multispecific antibody is devoid of an Fc domain and comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH and CH1 domains (i.e. VH-CH1 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid.

In one embodiment of the invention, the multispecific antibody is devoid of an Fc domain and comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH and CH1 domains (i.E. VH-CH1 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

In one embodiment of the invention, the multispecific antibody comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH, CH1, CH2 and CH3 domains (i.e. VH-CH1-CH2-CH3 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid; or
   ii) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

In one embodiment of the invention, the multispecific antibody comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH, CH1, CH2 and CH3 domains (i.e. VH-CH1-CH2-CH3 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid.

In one embodiment of the invention, the multispecific antibody comprises
a) a first light chain comprising in N-terminal to C-terminal direction a sequential arrangement of the VL and CL domains (i.e. VL-CL from N-terminal to C-terminal direction) and a first heavy chain comprising in N-terminal to C-terminal direction a sequential arrangement of the VH, CH1, CH2 and CH3 domains (i.e. VH-CH1-CH2-CH3 from N-terminal to C-terminal direction), wherein the first light chain and the first heavy chain are derived from a first antibody which specifically binds to a first antigen; and
b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
   i) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid.

One embodiment of the invention relates to a multispecific antibody, wherein in addition to the above modifications, in the respective other CL domain (i.e. of the second heavy chain or the first light chain) the amino acid at position 124 (numbering according to Kabat) is substituted by a negatively charged amino acid. The introduced negative charge at position 124 supports pairing of the CL domain with the corresponding CH1 domain (both domains derived from either the first or the second antibody). This is due to the fact that the introduced negatively charged amino acid at position 124 (numbering according to Kabat) in the CL domain faces the positively charged amino acid K at position 124 on the corresponding CH1 domain in the tertiary structure of the multispecific antibody. As a result, the negative charge originally present in this environment caused by the glutamic acid (E) residue at position 123 (numbering according to Kabat) in the CL domain is enhanced by introducing a second negatively charged amino acid, thereby improving the pairing of CL and CH1. By the amino acid substitution according to this embodiment, the yield of the multispecific antibody can be further improved and the formation of side products can be further impaired.

One embodiment of the invention relates to a multispecific antibody,
i) wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid and wherein in the constant domain CL of the second heavy chain the amino acid at position 124 (numbering according to Kabat) is substituted by a negatively charged amino acid; or
ii) wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid and wherein in the constant domain CL of the first light chain the amino acid at position 124 (numbering according to Kabat) is substituted by a negatively charged amino acid.

One embodiment of the invention relates to a multispecific antibody
i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D and wherein in the constant domain CL of the second heavy chain the amino acid at position 124 (numbering according to Kabat) is substituted by an amino acid selected from E or D; or
ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D and wherein in the constant domain CL of the first light chain the amino acid at position 124 (numbering according to Kabat) is substituted by an amino acid selected from E or D.

One embodiment of the invention relates to a multispecific antibody, wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid and wherein in the constant domain CL of the second heavy chain the amino acid at position 124 (numbering according to Kabat) is substituted by a negatively charged amino acid.

One embodiment of the invention relates to a multispecific antibody, wherein in the constant domain CL of the first light chain the amino acids at position 124 and 123 (numbering according to Kabat) are substituted independently from each other by a positively charged amino acid; and wherein in the constant domain CH1 of the first heavy chain the amino acids at position 147 and 213 (numbering according to EU index of Kabat) are substituted independently from each other by a negatively charged amino acid and wherein in the constant domain CL of the second heavy chain the amino acid at position 124 (numbering according to Kabat) is substituted by an amino acid selected from E or D, in one preferred embodiment by E.

One embodiment of the invention relates to a multispecific antibody wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D and wherein in the constant domain CL of the second heavy chain the amino acid at position 124 (numbering according to Kabat) is substituted by an amino acid selected from E or D, in one preferred embodiment by E.

In one embodiment of the invention, in the constant domain CL of the first light chain the amino acids at position 124 and 123 (numbering according to Kabat) are substituted independently from each other by an amino acid selected from K and R; and in the constant domain CH1 of the first heavy chain the amino acids at position 147 and 213 (numbering according to EU index of Kabat) are substituted independently from each other by an amino acid selected from E or D; and in the constant domain CL of the second heavy chain the amino acid at position 124 (numbering according to Kabat) is substituted by an amino acid selected from E and D, in one preferred embodiment by E.

In one embodiment of the invention, in the constant domain CL of the first light chain the amino acids at position 124 and 123 (numbering according to Kabat) are substituted by K; and in the constant domain CH1 of the first heavy chain the amino acids at position 147 and 213 (numbering according to EU index of Kabat) are substituted by E; and in the constant domain CL of the second heavy chain the amino acid at position 124 (numbering according to Kabat) is substituted by an amino acid selected from E and D, in one preferred embodiment by E.

One embodiment of the invention relates to a multispecific antibody, wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid and wherein in the constant domain CL of the first light chain the amino acid at position 124 (numbering according to Kabat) is substituted by a negatively charged amino acid.

One embodiment of the invention relates to a multispecific antibody, wherein in the constant domain CL of the second heavy chain the amino acids at position 124 and 123 (numbering according to Kabat) are substituted independently from each other by a positively charged amino acid; and wherein in the constant domain CH1 of the second light chain the amino acids at position 147 and 213 (numbering according to EU index of Kabat) are substituted independently from each other by a negatively charged amino acid and wherein in the constant domain CL of the first light chain the amino acid at position 124 (numbering according to Kabat) is substituted by an amino acid selected from E or D, in one preferred embodiment by E.

One embodiment of the invention relates to a multispecific antibody, wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D and wherein in the constant domain CL of the first light chain the amino acid at position 124 (numbering according to Kabat) is substituted by an amino acid selected from E or D, in one preferred embodiment by E.

In one embodiment of the invention, in the constant domain CL of the second heavy chain the amino acids at position 124 and 123 (numbering according to Kabat) are substituted independently from each other by an amino acid selected from K and R; and in the constant domain CH1 of the second light chain the amino acids at position 147 and 213 (numbering according to EU index of Kabat) are substituted independently from each other by an amino acid selected from E or D; and in the constant domain CL of the first light chain the amino acid at position 124 (numbering according to Kabat) is substituted by an amino acid selected from E and D, in one preferred embodiment by E.

In one embodiment of the invention, in the constant domain CL of the second heavy chain the amino acids at position 124 and 123 (numbering according to Kabat) are substituted by K; and in the constant domain CH1 of the second light chain the amino acids at position 147 and 213 (numbering according to EU index of Kabat) are substituted by E; and in the constant domain CL of the first light chain the amino acid at position 124 (numbering according to Kabat) is substituted by an amino acid selected from E and D, in one preferred embodiment by E.

One embodiment of the invention relates to a multispecific antibody, wherein in the variable domain VL of the first light chain the amino acid at position 38 (numbering according to Kabat) is substituted by an amino acid selected from K, R and H; and wherein in the variable domain VH of the first heavy chain the amino acid at position 39 (numbering according to Kabat) is substituted by an amino acid selected from E and D; and wherein in the variable domain VL of the second heavy chain the amino acid at position 38 (numbering according to Kabat) is substituted by an amino acid selected from E and D; and wherein in the variable domain VH of the second light chain the amino acid at position 39 (numbering according to Kabat) is substituted by an amino acid selected from K, R and H.

In one embodiment of the invention, in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention
- in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D; or
- in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E; or
- in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E; or
- in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E; and
in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E; and in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E; and in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention
- in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by an amino acid selected from E and D; or
- in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted by an amino acid selected from E and D; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by an amino acid selected from E and D; or
- in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted by an amino acid selected from E and D; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by E; or
- in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted E; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted independently from each other by an amino acid selected from K and R; and wherein in the constant domain CH1 of the first heavy chain the amino acids at position 147 and 213 (numbering according to EU index of Kabat) are substituted independently from each other by an amino acid selected from E or D and in the constant domain CL of the second heavy chain the amino acid at position 124 (numbering according to Kabat) is substituted by E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted independently from each other by an amino acid selected from K and R; and wherein in the constant domain CH1 of the second light chain the amino acids at position 147 and 213 (numbering according to EU index of Kabat) are substituted independently from each other by an amino acid selected from E or D and in the constant domain CL of the first light chain the amino acid at position 124 (numbering according to Kabat) is substituted E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by an amino acid selected from E and D; or
- in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by an amino acid selected from E and D; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by an amino acid selected from E and D; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by E; or
- in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by E; and
in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by E; and in the variable domain VL of the first light chain the amino acid at position 38 (numbering according to Kabat) is substituted by an amino acid selected from K, R and H; in the variable domain VH of the first heavy chain the amino acid at position 39 (numbering according to Kabat) is substituted by an amino acid selected from E and D; in the variable domain VL of the second heavy chain the amino acid at position 38 (numbering according to Kabat) is substituted by an amino acid selected from E and D; and in the variable domain VH of the second light chain the amino acid at position 39 (numbering according to Kabat) is substituted by an amino acid selected from K, R and H.

In one embodiment of the invention
- in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by E; or
- in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by E; and
in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention in the constant domain CL of the first light chain the amino acids at position 124 and 123 (numbering according to Kabat) are substituted by K; and wherein in the constant domain CH1 of the first heavy chain the amino acids at position 147 and 213 (numbering according to EU index of Kabat) are substituted by E and in the constant domain CL of the second heavy chain the amino acid at position 124 (numbering according to Kabat) is substituted by E; and in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In one embodiment of the invention in the constant domain CL of the second heavy chain the amino acids at position 124 and 123 (numbering according to Kabat) are substituted by K; and wherein in the constant domain CH1 of the second light chain the amino acids at position 147 and 213 (numbering according to EU index of Kabat) are substituted by E, and in the constant domain CL of the first light chain the amino acid at position 124 (numbering according to Kabat) is substituted by E; and in the variable domain VL of the first light chain the amino acid at position 38 (numbering according to Kabat) is substituted by K; in the variable domain VH of the first heavy chain the amino acid at position 39 (numbering according to Kabat) is substituted by E; in the variable domain VL of the second heavy chain the amino acid at position 38 (numbering according to Kabat) is substituted by E; and in the variable domain VH of the second light chain the amino acid at position 39 (numbering according to Kabat) is substituted by K.

In case the multispecific antibody according to the invention comprises the an Fc region and/or [a first heavy chain including the domains VH-CH1-CH2-CH3 and a modified second heavy chain including the domains VL-CL-CH2-CH3], an additional aspect of the invention is to provide an approach in order to improve the ratio of the desired multispecific antibody compared to undesired side products (that may for example be formed by mispairing of the first heavy chain with another first heavy chain, or mispairing of the second heavy chain with another second heavy chain). According to the invention this can be additionally supported by amino acid substitutions in the CH3 domains of the first heavy chain and the second heavy chain, respectively. By these approaches, which are to be described in further detail in the following paragraphs, heterodimerisation of the first heavy chain and the modified second heavy chain is improved.

Thus, one embodiment of the invention is a multispecific antibody according to the invention, which comprises a first heavy chain including a CH3 domain derived from said first antibody and a second heavy chain including a CH3 domain derived from said second antibody, wherein both CH3 domains are engineered in a complementary manner by respective amino acid substitutions, in order to support heterodimerisation of the first heavy chain and the modified second heavy chain.

Several approaches for CH3-modifications in order to support heterodimerization have been described, for example in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, WO 2013/096291, which are herein included by reference. Typically, in the approaches known in the art, the CH3 domain of the first heavy chain and the CH3 domain of the second heavy chain are both engineered in a complementary manner so that the heavy chain comprising one engineered CH3 domain can no longer homodimerize with another heavy chain of the same structure (e.g. a CH3-engineered first heavy chain can no longer homodimerize with another CH3-engineered first heavy chain; and a CH3-engineered second heavy chain can no longer homodimerize with another CH3-engineered second heavy chain). Thereby the heavy chain comprising one engineered CH3 domain is forced to heterodimerize with another heavy chain comprising the CH3 domain, which is engineered in a complementary manner. For this embodiment of the invention, the CH3 domain of the first heavy chain and the CH3 domain of the second heavy chain are engineered in a complementary manner by amino acid substitutions, such that the first heavy chain and the second heavy chain are forced to heterodimerize, whereas the first heavy chain and the second heavy chain can no longer homodimerize (e.g. for sterical reasons).

The different approaches for supporting heavy chain heterodimerization known in the art, that were cited and included above, are contemplated as different alternatives used in a multispecific antibody according to the invention, which comprises a "non-crossed Fab region" derived from a first antibody, which specifically binds to a first antigen, and a "crossed Fab region" derived from a second antibody, which specifically binds to a second antigen, in combination with the particular amino acid substitutions described above for the invention.

In one embodiment of the multispecific antibody according to the invention, which comprises a first heavy chain including a CH3 domain derived from said first antibody and a second heavy chain including a CH3 domain derived from said second antibody, the CH3 domains of the first and second heavy chain are engineered by the so-called "knob-into-hole" technology, which is described in detail providing several examples in e.g. WO 96/027011, Ridgway, J.B., et al., Protein Eng. 9 (1996) 617-621; Merchant, A.M., et al., Nat. Biotechnol. 16 (1998) 677-681; and WO 98/ 050431, which are herein included by reference. In the "knob-into-hole" technology, within the interface formed between the CH3 domain of the first heavy chain and the CH3 domain of the second heavy chain in the tertiary structure of the antibody, particular amino acids on each CH3 domain are engineered to produce a protuberance ("knob") in the CH3 domain of one heavy chain and a cavity ("hole") in the CH3 domain of the other heavy chain, respectively. In the tertiary structure of the multispecific antibody the introduced protuberance in the CH3 domain of the one heavy chain is positionable in the introduced cavity in the CH3 domain of the other heavy chain. Each of the heavy chains can comprise the "knob" in its CH3 domain while the other heavy chain comprises the "hole" in its CH3 domain.

Thus, one embodiment relates to a multispecific antibody according to the invention, wherein in the tertiary structure of the antibody the CH3 domain of the first heavy chain and the CH3 domain of the second heavy chain form an interface that is located between the respective antibody CH3 domains, wherein the respective amino acid sequences of the CH3 domain of the first heavy chain and the CH3 domain of the second heavy chain each comprise a set of amino acids that is located within said interface in the tertiary structure of the antibody,
- wherein from the set of amino acids that is located in the interface in the CH3 domain of one heavy chain at least one amino acid residue is substituted by an amino acid residue having a larger side chain volume than the original amino acid residue, thereby generating a protuberance within the interface, wherein the protuberance is located in the CH3 domain of the one heavy chain, and wherein the protuberance is positionable in a cavity located in the CH3 domain of the other heavy chain within the interface; and
- wherein from the set of amino acids that is located in the interface in the CH3 domain of the other heavy chain at least one amino acid residue is substituted by an amino acid residue having a smaller side chain volume than the original amino acid residue, thereby generating a cavity within the interface, wherein the cavity is located in the CH3 domain of the other heavy chain, and wherein in the cavity the protuberance within the interface located in the CH3 domain of the one heavy chain is positionable. The multispecific antibody according to this embodiment is herein also referred to as "CH3(KiH)-engineered multispecific antibody" (wherein the abbreviation "KiH" stands for the "knob-into-hole technology").

According to this embodiment of the invention relating to the CH3(KiH)-engineered multispecific antibody, the characteristics of the CH3(KiH)-engineered multispecific antibody, as well as the various embodiments of said CH3(KiH)-engineered multispecific antibody, which are described in further detail below, can be combined with either of the embodiments of said multispecific antibody as described above.

In one embodiment of said CH3(KiH)-engineered multispecific antibody according to the invention said amino acid residue having a larger side chain volume than the original amino acid residue is selected from R, F, Y and W.

In one embodiment of said CH3(KiH)-engineered multispecific antibody according to the invention said amino acid residue having a smaller side chain volume than the original amino acid residue is selected from A, S, T and V.

In one embodiment of said CH3(KiH)-engineered multispecific antibody according to the invention said amino acid residue having a larger side chain volume than the original amino acid residue is selected from R, F, Y and W; and said amino acid residue having a smaller side chain volume than the original amino acid residue is selected from A, S, T and V.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W, the amino acid R at position 409 (numbering according to EU index of Kabat) is substituted by D, and the amino acid K at position 370 (numbering according to EU index of Kabat) is substituted by E; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V, the amino acid D at position 399 (numbering according to EU index of Kabat) is substituted by K, and the amino acid E at position 357 (numbering according to EU index of Kabat) is substituted by K.

In addition to the engineering of the CH3 domains of the first and second heavy chain by the "knob-into-hole" technology, the introduction of a disulfide bridge further stabilizes the heterodimers (Atwell, S., et al., J. Mol. Biol. 270 (1997) 26-35; Merchant, A.M., et al., Nature Biotech. 16 (1998) 677-681). Thereby the additional introduction of a disulfide bridge further increases the yield of the multispecific antibody according to the invention.

Therefore, in one embodiment of said CH3(KiH)-engineered multispecific antibody according to the invention, from the set of amino acids that is located in the interface in the CH3 domain of the one heavy chain a first amino acid is substituted by cysteine; and from the set of amino acids that is located in the interface in the CH3 domain of the other heavy chain a second amino acid is substituted by cysteine, wherein the second amino acid is facing the first amino acid within the interface; such that a disulfide bridge between the CH3 domain of the one heavy chain and the CH3 domain of the other heavy chain can be formed via the introduced cysteine residues.

In one embodiment of said CH3(KiH)-engineered multispecific antibody according to the invention said amino acid residue having a larger side chain volume than the original amino acid residue is selected from R, F, Y and W; and from the set of amino acids that is located in the interface in the CH3 domain of the one heavy chain a first amino acid is substituted by cysteine; and from the set of amino acids that is located in the interface in the CH3 domain of the other heavy chain a second amino acid is substituted by cysteine, wherein the second amino acid is facing the first amino acid within the interface; such that a disulfide bridge between the CH3 domain of the one heavy chain and the CH3 domain of the other heavy chain can be formed via the introduced cysteine residues.

In one embodiment of said CH3(KiH)-engineered multispecific antibody according to the invention said amino acid residue having a smaller side chain volume than the original amino acid residue is selected from A, S, T and V; and from the set of amino acids that is located in the interface in the CH3 domain of the one heavy chain a first amino acid is substituted by cysteine; and from the set of amino acids that is located in the interface in the CH3 domain of the other heavy chain a second amino acid is substituted by cysteine, wherein the second amino acid is facing the first amino acid within the interface; such that a disulfide bridge between the CH3 domain of the one heavy chain and the CH3 domain of the other heavy chain can be formed via the introduced cysteine residues.

In one embodiment of said CH3(KiH)-engineered multispecific antibody according to the invention said amino acid residue having a larger side chain volume than the original amino acid residue is selected from R, F, Y and W; said amino acid residue having a smaller side chain volume than the original amino acid residue is selected from A, S, T and V; and from the set of amino acids that is located in the interface in the CH3 domain of the one heavy chain a first amino acid is substituted by cysteine; and from the set of amino acids that is located in the interface in the CH3 domain of the other heavy chain a second amino acid is substituted by cysteine, wherein the second amino acid is facing the first amino acid within the interface; such that a disulfide bridge between the CH3 domain of the one heavy chain and the CH3 domain of the other heavy chain can be formed via the introduced cysteine residues.

In one embodiment of said CH3(KiH)-engineered multispecific antibody according to the invention said amino acid residue having a larger side chain volume than the original amino acid residue is selected from R, F, Y and W; and in the CH3 domain of the one heavy chain (the heavy chain comprising the "knob") either the amino acid E at position 356 (numbering according to EU index of Kabat) or the amino acid S at position 354 (numbering according to EU index of Kabat) is substituted by C and in the CH3 domain of the other heavy chain (the heavy chain comprising the "hole") the amino acid Y at position 349 (numbering according to EU index of Kabat) is substituted by C.

In one embodiment of said CH3(KiH)-engineered multispecific antibody according to the invention said amino acid residue having a smaller side chain volume than the original amino acid residue is selected from A, S, T and V; and in the CH3 domain of the one heavy chain (the heavy chain comprising the "knob") either the amino acid E at position 356 (numbering according to EU index of Kabat) or the amino acid S at position 354 (numbering according to EU index of Kabat) is substituted by C and in the CH3 domain of the other heavy chain (the heavy chain comprising the "hole") the amino acid Y at position 349 (numbering according to EU index of Kabat) is substituted by C..

In one embodiment of said CH3(KiH)-engineered multispecific antibody according to the invention said amino acid residue having a larger side chain volume than the original amino acid residue is selected from R, F, Y and W; and said amino acid residue having a smaller side chain volume than the original amino acid residue is selected from A, S, T and V; and in the CH3 domain of the one heavy chain (the heavy chain comprising the "knob") either the amino acid E at position 356 (numbering according to EU index of Kabat) or the amino acid S at position 354 (numbering according to EU index of Kabat) is substituted by C and in the CH3 domain of the other heavy chain (the heavy chain comprising the "hole") the amino acid Y at position 349 (numbering according to EU index of Kabat) is substituted by C.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W and either the amino acid E at position 356 (numbering according to EU index of Kabat) or the amino acid S at position 354 (numbering according to EU index of Kabat) is substituted by C; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A, the amino acid Y at position 407 (numbering according to EU index of Kabat) is substituted by V and the amino acid Y at position 349 (numbering according to EU index of Kabat) is substituted byC.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W and the amino acid Y at position 349 (numbering according to EU index of Kabat) is substituted by C; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A, the amino acid Y at position 407 (numbering according to EU index of Kabat) is substituted by V and either the amino acid E at position 356 (numbering according to EU index of Kabat) or the amino acid S at position 354 (numbering according to EU index of Kabat) is substituted by C.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W, the amino acid R at position 409 (numbering according to EU index of Kabat) is substituted by D, the amino acid K at position 370 (numbering according to EU index of Kabat) is substituted by E and either the amino acid E at position 356 (numbering according to EU index of Kabat) or the amino acid S at position 354 (numbering according to EU index of Kabat) is substituted by C; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V, the amino acid D at position 399 (numbering according to EU index of Kabat) is substituted by K, the amino acid E at position 357 (numbering according to EU index of Kabat) is substituted by K and the amino acid Y at position 349 (numbering according to EU index of Kabat) is substituted by C.

In one embodiment of said CH3(KiH)-engineered multispecific antibody according to the invention, in the CH3 domain of the one heavy chain (the heavy chain comprising the "knob") the amino acid T at position 366 (numbering according to EU index of Kabat) is substituted by W, the amino acid R at position 409 (numbering according to EU index of Kabat) is substituted by D, the amino acid K at position 370 (numbering according to EU index of Kabat) is substituted by E and the amino acid Y at position 349 (numbering according to EU index of Kabat) is substituted by C; and in the CH3 domain of the other heavy chain (the heavy chain comprising the "hole") the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V, the amino acid D at position 399 (numbering according to EU index of Kabat) is substituted by K, the amino acid E at position 357 (numbering according to EU index of Kabat) is substituted by K and either the amino acid E at position 356 (numbering according to EU index of Kabat) or the amino acid S at position 354 (numbering according to EU index of Kabat) is substituted by C.

Apart from the "knob-into-hole technology" other techniques for modifying the CH3 domains of the heavy chains of a multispecific antibody to enforce heterodimerization are known in the art. These technologies, especially the ones described in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954 and WO 2013/096291 are contemplated herein as alternatives to the "knob-into-hole technology" in combination with a multispecific antibody according to the invention.

In one embodiment of a multispecific antibody according to the invention the approach described in EP 1870459 is used to support heterodimerization of the first heavy chain and the second heavy chain of the multispecific antibody. This approach is based on the introduction of charged amino acids with opposite charges at specific amino acid positions in the CH3/CH3-domain-interface between both, the first and the second heavy chain.

Accordingly, this embodiment relates to a multispecific antibody according to the invention, wherein in the tertiary structure of the antibody the CH3 domain of the first heavy chain and the CH3 domain of the second heavy chain form an interface that is located between the respective antibody CH3 domains, wherein the respective amino acid sequences of the CH3 domain of the first heavy chain and the CH3 domain of the second heavy chain each comprise a set of amino acids that is located within said interface in the tertiary structure of the antibody, wherein from the set of amino acids that is located in the interface in the CH3 domain of one heavy chain a first amino acid is substituted by a positively charged amino acid and from the set of amino acids that is located in the interface in the CH3 domain of the other heavy chain a second amino acid is substituted by a negatively charged amino acid. The multispecific antibody according to this embodiment is herein also referred to as "CH3(+/-)-engineered multispecific antibody" (wherein the abbreviation "+/-" stands for the oppositely charged amino acids that were introduced in the respective CH3 domains).

In one embodiment of said CH3(+/-)-engineered multispecific antibody according to the invention the positively charged amino acid is selected from K, R and H; and the negatively charged amino acid is selected from E or D.

In one embodiment of said CH3(+/-)-engineered multispecific antibody according to the invention the positively charged amino acid is selected from K and R; and the negatively charged amino acid is selected from E or D.

In one embodiment of said CH3(+/-)-engineered multispecific antibody according to the invention the positively charged amino acid is K; and the negatively charged amino acid is E.

In one embodiment of said CH3(+/-)-engineered multispecific antibody according to the invention in the CH3 domain of one heavy chain the amino acid R at position 409 (numbering according to EU index of Kabat) is substituted by D and the amino acid K at position 370 (numbering according to EU index of Kabat) is substituted by E; and in the CH3 domain of the other heavy chain the amino acid D at position 399 (numbering according to EU index of Kabat) is substituted by K and the amino acid E at position 357 (numbering according to EU index of Kabat) is substituted by K.

In one embodiment of a multispecific antibody according to the invention the approach described in WO2013/157953 is used to support heterodimerization of the first heavy chain and the second heavy chain of the multispecific antibody. In one embodiment of said CH3-engineered multispecific antibody according to the invention, in the CH3 domain of one heavy chain the amino acid T at position 366 (numbering according to EU index of Kabat) is substituted by K; and in the CH3 domain of the other heavy chain the amino acid L at position 351 (numbering according to EU index of Kabat) is substituted by D. In another embodiment of said CH3-engineered multispecific antibody according to the invention, in the CH3 domain of one heavy chain the amino acid T at position 366 (numbering according to EU index of Kabat) is substituted by K and the amino acid L at position 351 (numbering according to EU index of Kabat) is substituted by K; and in the CH3 domain of the other heavy chain the amino acid L at position 351 (numbering according to EU index of Kabat) is substituted by D.

In another embodiment of said CH3-engineered multispecific antibody according to the invention, in the CH3 domain of one heavy chain the amino acid T at position 366 (numbering according to EU index of Kabat) is substituted by K and the amino acid L at position 351 (numbering according to EU index of Kabat) is substituted by K; and in the CH3 domain of the other heavy chain the amino acid L at position 351 (numbering according to EU index of Kabat) is substituted by D. Additionally at least one of the following substitutions is comprised in the CH3 domain of the other heavy chain: the amino acid Y at position 349 (numbering according to EU index of Kabat) is substituted by E, the amino acid Y at position 349 (numbering according to EU index of Kabat) is substituted by D and the amino acid L at position 368 (numbering according to EU index of Kabat) is substituted by E. In one embodiment the amino acid L at position 368 (numbering according to EU index of Kabat) is substituted by E.

In one embodiment of a multispecific antibody according to the invention the approach described in WO2012/058768 is used to support heterodimerization of the first heavy chain and the second heavy chain of the multispecific antibody. In one embodiment of said CH3-engineered multispecific antibody according to the invention, in the CH3 domain of one heavy chain the amino acid L at position 351 (numbering according to EU index of Kabat) is substituted by Y and the amino acid Y at position 407 (numbering according to EU index of Kabat) is substituted by A; and in the CH3 domain of the other heavy chain the amino acid T at position 366 (numbering according to EU index of Kabat) is substituted by A and the amino acid K at position 409 (numbering according to EU index of Kabat) is substituted by F. In another embodiment, in addition to the aforementioned substitutions, in the CH3 domain of the other heavy chain at least one of the amino acids at positions 411 (originally T), 399 (originally D), 400 (originally S), 405 (originally F), 390 (originally N) and 392 (originally K) is substituted. Preferred substitutions are:
- substituting the amino acid T at position 411 (numbering according to EU index of Kabat) by an amino acid selected from N, R, Q, K, D, E and W;
- substituting the amino acid D at position 399 (numbering according to EU index of Kabat) by an amino acid selected from R, W, Y, and K;
- substituting the amino acid S at position 400 (numbering according to EU index of Kabat) by an amino acid selected from E, D, R and K;
- substituting the amino acid F at position 405 (numbering according to EU index of Kabat) by an amino acid selected from I, M, T, S, V and W;
- substituting the amino acid N at position 390 (numbering according to EU index of Kabat) by an amino acid selected from R, K and D; and
- substituting the amino acid K at position 392 (numbering according to EU index of Kabat) by an amino acid selected from V, M, R, L, F and E.

In another embodiment of said CH3-engineered multispecific antibody according to the invention (engineered according to WO2012/058768), in the CH3 domain of one heavy chain the amino acid L at position 351 (numbering according to EU index of Kabat) is substituted by Y and the amino acid Y at position 407 (numbering according to EU index of Kabat) is substituted by A; and in the CH3 domain of the other heavy chain the amino acid T at position 366 (numbering according to EU index of Kabat) is substituted by V and the amino acid K at position 409 (numbering according to EU index of Kabat) is substituted by F. In another embodiment of said CH3-engineered multispecific antibody according to the invention, in the CH3 domain of one heavy chain the amino acid Y at position 407 (numbering according to EU index of Kabat) is substituted by A; and in the CH3 domain of the other heavy chain the amino acid T at position 366 (numbering according to EU index of Kabat) is substituted by A and the amino acid K at position 409 (numbering according to EU index of Kabat) is substituted by F. In said last aforementioned embodiment, in the CH3 domain of said other heavy chain the amino acid K at position 392 (numbering according to EU index of Kabat) is substituted by E, the amino acid T at position 411 (numbering according to EU index of Kabat) is substituted by E, the amino acid D at position 399 (numbering according to EU index of Kabat) is substituted by R and the amino acid S at position 400 (numbering according to EU index of Kabat) is substituted by R.

In one embodiment of a multispecific antibody according to the invention the approach described in WO 2011/143545 is used to support heterodimerization of the first heavy chain and the second heavy chain of the multispecific antibody. In one embodiment of said CH3-engineered multispecific antibody according to the invention, amino acid modifications in the CH3 domains of both heavy chains are introduced at positions 368 and/or 409.

In one embodiment of a multispecific antibody according to the invention the approach described in WO 2011/090762 is used to support heterodimerization of the first heavy chain and the second heavy chain of the multispecific antibody. WO 2011/090762 relates to amino acid modifications according to the "knob-into-hole" technology. In one embodiment of said CH3(KiH)-engineered multispecific antibody according to the invention, in the CH3 domain of one heavy chain the amino acid T at position 366 (numbering according to EU index of Kabat) is substituted by W; and in the CH3 domain of the other heavy chain the amino acid Y at position 407 (numbering according to EU index of Kabat) is substituted by A. In another embodiment of said CH3(KiH)-engineered multispecific antibody according to the invention, in the CH3 domain of one heavy chain the amino acid T at position 366 (numbering according to EU index of Kabat) is substituted by Y; and in the CH3 domain of the other heavy chain the amino acid Y at position 407 (numbering according to EU index of Kabat) is substituted by T.

In one embodiment of a multispecific antibody according to the invention, which is of IgG2 isotype, the approach described in WO 2011/090762 is used to support heterodimerization of the first heavy chain and the second heavy chain of the multispecific antibody.

In one embodiment of a multispecific antibody according to the invention, the approach described in WO 2009/089004 is used to support heterodimerization of the first heavy chain and the second heavy chain of the multispecific antibody. In one embodiment of said CH3-engineered multispecific antibody according to the invention, in the CH3 domain of one heavy chain the amino acid K or N at position 392 (numbering according to EU index of Kabat) is substituted by a negatively charged amino acid (in one preferred embodiment by E or D, in one preferred embodiment by D); and in the CH3 domain of the other heavy chain the amino acid D at position 399 the amino acid E or D at position 356 or the amino acid E at position 357 (numberings according to EU index of Kabat) is substituted by a positively charged amino acid (in one preferred embodiment K or R, in one preferred embodiment by K, in one preferred embodiment the amino acids at positions 399 or 356 are substituted by K). In one further embodiment, in addition to the aforementioned substitutions, in the CH3 domain of the one heavy chain the amino acid K or R at position 409 (numbering according to EU index of Kabat) is substituted by a negatively charged amino acid (in one preferred embodiment by E or D, in one preferred embodiment by D). In one even further embodiment, in addition to or alternatively to the aforementioned substitutions, in the CH3 domain of the one heavy chain the amino acid K at position 439 and/or the amino acid K at position 370 (numbering according to EU index of Kabat) is substituted independently from each other by a negatively charged amino acid (in one preferred embodiment by E or D, in one preferred embodiment by D).

In one embodiment of a multispecific antibody according to the invention, the approach described in WO 2007/147901 is used to support heterodimerization of the first heavy chain and the second heavy chain of the multispecific antibody. In one embodiment of said CH3-engineered multispecific antibody according to the invention, in the CH3 domain of one heavy chain the amino acid K at position 253 (numbering according to EU index of Kabat) is substituted by E, the amino acid D at position 282 (numbering according to EU index of Kabat) is substituted by K and the amino acid K at position 322 (numbering according to EU index of Kabat) is substituted by D; and in the CH3 domain of the other heavy chain the amino acid D at position 239 (numbering according to EU index of Kabat) is substituted by K, the amino acid E at position 240 (numbering according to EU index of Kabat) is substituted by K and the amino acid K at position 292 (numbering according to EU index of Kabat) is substituted by D.

In one embodiment of a multispecific antibody according to the invention, the approach described in WO 2007/110205 is used to support heterodimerization of the first heavy chain and the second heavy chain of the multispecific antibody.

In one embodiment of the invention the multispecific antibody is a bispecific antibody or a trispecific antibody. In one preferred embodiment of the invention the multispecific antibody is a bispecific antibody.

In one embodiment of the invention, the antibody is a bivalent or trivalent antibody. In one embodiment of the invention, the antibody is a bivalent antibody.

In one embodiment of the invention, the multispecific antibody comprises immunoglobulin constant regions of one or more immunoglobulin classes. Immunoglobulin classes include IgG, IgM, IgA, IgD, and IgE isotypes and, in the case of IgG and IgA, their subtypes.

In one embodiment of the invention, the multispecific antibody has a constant domain structure of an IgG type antibody. In one further embodiment of the invention, the multispecific antibody is characterized in that said multispecific antibody is of human IgG1 subclass, or of human IgG1 subclass with the mutations L234A and L235A. In one further embodiment of the invention, the multispecific antibody is characterized in that said multispecific antibody is of human IgG2 subclass. In one further embodiment of the invention, the multispecific antibody is characterized in that said multispecific antibody is of human IgG3 subclass. In one further embodiment of the invention, the multispecific antibody is characterized in that said multispecific antibody is of human IgG4 subclass or, of human IgG4 subclass with the additional mutation S228P. In one further embodiment of the invention, the multispecific antibody is characterized in that said multispecific antibody is of human IgG1 or human IgG4 subclass. In one further embodiment of the invention, the multispecific antibody is characterized in that said multispecific antibody is of human IgG1 subclass with the mutations L234A and L235A (numbering according to EU index of Kabat). In one further embodiment of the invention, the multispecific antibody is characterized in that said multispecific antibody is of human IgG1 subclass with the mutations L234A, L235A and P329G (numbering according to EU index of Kabat). In one further embodiment of the invention, the multispecific antibody is characterized in that said multispecific antibody is of human IgG4 subclass with the mutations S228P and L235E (numbering according to EU index of Kabat). In one further embodiment of the invention, the multispecific antibody is characterized in that said multispecific antibody is of human IgG4 subclass with the mutations S228P, L235E and P329G (numbering according to EU index of Kabat).

In one embodiment, an antibody comprising a heavy chain including a CH3 domain as specified herein, comprises an additional C-terminal glycine-lysine dipeptide (G446 and K447, numbering according to EU index of Kabat). In one embodiment, an antibody comprising a heavy chain including a CH3 domain, as specified herein, comprises an additional C-terminal glycine residue (G446, numbering according to EU index of Kabat).

The multispecific antibody is prepared by recombinant methods. Thus, the invention also relates to a method for the preparation of a multispecific antibody according to the invention, comprising the steps of
- transforming a host cell with vectors comprising nucleic acids encoding
   a) the first light chain as defined for a multispecific antibody according to the invention derived from a first antibody which specifically binds to a first antigen;
   b) the first heavy chain as defined for a multispecific antibody according to the invention derived from a first antibody which specifically binds to a first antigen;
   c) the second light chain as defined for a multispecific antibody according to the invention derived from a second antibody which specifically binds to a second antigen; and
   d) the second heavy chain as defined for a multispecific antibody according to the invention derived from a second antibody which specifically binds to a second antigen,
- culturing said host cell under conditions that allow synthesis of said multispecific antibody; and
- recovering said multispecific antibody from said host cell culture.

In one embodiment of a method according to the invention, the host cell is transformed with vectors comprising nucleic acids encoding
a) the first light chain derived from a first antibody which specifically binds to a first antigen, wherein in the constant domain CL of the first light chain the amino acids at position 124 and 123 (numbering according to Kabat) are substituted independently from each other by an amino acid selected from K, R and H (in one preferred embodiment by K or R, in one preferred embodiment by K);
b) the first heavy chain derived from a first antibody which specifically binds to a first antigen, wherein in the constant domain CH1 of the first heavy chain the amino acids at position 147 and 213 (numbering according to EU index of Kabat) are substituted independently from each other by an amino acid selected from E or D (in one preferred embodiment by E);
c) the second light chain derived from a second antibody which specifically binds to a second antigen, wherein in the second light chain the constant domain CL is replaced by the constant domain CH1 of the second heavy chain; and
d) the second heavy chain derived from a second antibody which specifically binds to a second antigen, wherein in the second heavy chain the constant domain CH1 is replaced by the constant domain CL of the second light chain.

In one embodiment of a method according to the invention, the host cell is transformed with vectors comprising nucleic acids encoding
a) the first light chain derived from a first antibody which specifically binds to a first antigen;
b) the first heavy chain derived from a first antibody which specifically binds to a first antigen,;
c) the second light chain derived from a second antibody which specifically binds to a second antigen, wherein in the second light chain the constant domain CL is replaced by the constant domain CH1 of the second heavy chain,
   and wherein in the constant domain CL of the second heavy chain the amino acids at position 124 and 123 (numbering according to Kabat) are substituted independently from each other by an amino acid selected from K, R and H (in one preferred embodiment by K or R, in one preferred embodiment by K); and
d) the second heavy chain derived from a second antibody which specifically binds to a second antigen, wherein in the second heavy chain the constant domain CH1 is replaced by the constant domain CL of the second light chain,
   and wherein in the constant domain CH1 of the second light chain the amino acids at position 147 and 213 (numbering according to EU index of Kabat) are substituted independently from each other by an amino acid selected from E or D (in one preferred embodiment by E).

Another object of the invention is a multispecific antibody produced by a method according to the invention.

Another object of the invention is a host cell comprising
a) a vector comprising nucleic acids encoding the first light chain as defined in one of claims 1 to 9 derived from a first antibody which specifically binds to a first antigen;
b) a vector comprising nucleic acids encoding the first heavy chain as defined in one of claims 1 to 9 derived from a first antibody which specifically binds to a first antigen;
c) a vector comprising nucleic acids encoding the second light chain as defined in one of claims 1 to 9 derived from a second antibody which specifically binds to a second antigen; and
d) a vector comprising nucleic acids encoding the second heavy chain as defined in one of claims 1 to 9 derived from a second antibody which specifically binds to a second antigen.

Another object of the invention is a nucleic acid encoding the multispecific antibody according to the invention.

In one embodiment the nucleic acid encodes a first light chain as defined for a multispecific antibody according to the invention. In one embodiment the nucleic acid encodes a first light chain as defined for a multispecific antibody according to the invention, wherein in the constant domain CL of the first light chain the amino acids at position 124 and 123 (numbering according to Kabat) are substituted independently from each other by an amino acid selected from K, R and H (in one embodiment selected from K and R, in one embodiment the amino acid is K). In one embodiment the nucleic acid encodes a first light chain as defined for a multispecific antibody according to the invention, wherein in the constant domain CL of the first light chain the amino acid at position 124 (numbering according to Kabat) is substituted by an amino acid selected from E and D (in one embodiment the amino acid is E).

In one embodiment the nucleic acid encodes a first heavy chain as defined for a multispecific antibody according to the invention. In one embodiment the nucleic acid encodes a first heavy chain as defined for a multispecific antibody according to the invention, wherein in the constant domain CH1 of the first heavy chain the amino acids at position 147 and 213 (numbering according to EU index of Kabat) are substituted independently from each other by an amino acid selected from E or D (in one embodiment the amino acid is E).

In one embodiment the nucleic acid encodes a modified second light chain as defined for a multispecific antibody according to the invention. In one embodiment the nucleic acid encodes a modified second light chain as defined for a multispecific antibody according to the invention, wherein in the constant domain CH1 of the second light chain the amino acids at position 147 and 213 (numbering according to EU index of Kabat) are substituted independently from each other by an amino acid selected from E or D (in one embodiment the amino acid is E). In one further embodiment, the nucleic acid encodes a modified second light chain as defined for a multispecific antibody according to the invention, wherein in the variable domain VH the amino acid at position 39 (numbering according to Kabat) is substituted by an amino acid selected from E and D (in one preferred embodiment by E).

In one embodiment the nucleic acid encodes a modified second heavy chain as defined for a multispecific antibody according to the invention. In one embodiment the nucleic acid encodes a modified second heavy chain as defined for a multispecific antibody according to the invention, wherein in the constant domain CL of the second heavy chain the amino acids at position 124 and 123 (numbering according to Kabat) are substituted independently from each other by an amino acid selected from K, R and H (in one embodiment selected from K and R, in one embodiment the amino acid is K). In one further embodiment, the nucleic acid encodes a modified second heavy chain as defined for a multispecific antibody according to the invention, wherein in the constant domain CL the amino acid at position 124 (numbering according to Kabat) is substituted by an amino acid selected from E and D (in one embodiment the amino acid is E). In one further embodiment, the nucleic acid encodes a modified second heavy chain as defined for a multispecific antibody according to the invention, wherein in the variable domain VL of the second heavy chain the amino acid at position 38 (numbering according to Kabat) is substituted by an amino acid selected from K, R and H (in one preferred embodiment by K or R, in one preferred embodiment by K). In one further embodiment, the nucleic acid encodes a modified second heavy chain as defined for a multispecific antibody according to the invention, wherein in the constant domain CL the amino acid at position 124 (numbering according to Kabat) is substituted by an amino acid selected from E and D; and wherein in the variable domain VL of the second heavy chain the amino acid at position 38 (numbering according to Kabat) is substituted by an amino acid selected from E and D (in one preferred embodiment by E).

In one embodiment, the nucleic acid according to the invention is an isolated nucleic acid.

Another object of the invention is an expression vector comprising a nucleic acid according to the invention, wherein the vector is capable of expressing said nucleic acid in a host cell.

Another object of the invention is a host cell comprising a nucleic acid according to the invention. Another object of the invention is a host cell comprising an expression vector according to the invention. In one embodiment the host cell is a HEK293 cells or a CHO cell.

Another object of the invention is a method of producing an antibody comprising culturing said host cell of the invention so that the antibody is produced. In one embodiment of said method the method further comprises recovering the antibody from the host cell.

Another object of the invention is a pharmaceutical composition comprising a multispecific antibody according to the invention. One aspect of the invention is a pharmaceutical composition comprising a multispecific antibody according to the invention in combination with at least one pharmaceutically acceptable carrier.

In one embodiment, a composition (in one preferred embodiment a pharmaceutical composition) comprising a population of antibodies of the invention comprises an antibody comprising a heavy chain including a CH3 domain, as specified herein, with an additional C-terminal glycine-lysine dipeptide (G446 and K447, numbering according to EU index of Kabat). In one embodiment, a composition comprising a population of antibodies of the invention comprises an antibody comprising a heavy chain including a CH3 domain, as specified herein, with an additional C-terminal glycine residue (G446, numbering according to EU index of Kabat).

In one embodiment, such a composition comprises a population of antibodies comprised of antibodies comprising a heavy chain including a CH3 domain, as specified herein; antibodies comprising a heavy chain including a CH3 domain, as specified herein, with an additional C-terminal glycine residue (G446, numbering according to EU index of Kabat); and antibodies comprising a heavy chain including a CH3 domain, as specified herein, with an additional C-terminal glycine-lysine dipeptide (G446 and K447, numbering according to EU index of Kabat).

Another object of the invention is an immunoconjugate comprising the multispecific antibody according to the invention coupled to a cytotoxic agent.

Another object of the invention is the use of a multispecific antibody according to the invention for the manufacture of a pharmaceutical composition. Another object of the invention is a method for the manufacture of a pharmaceutical composition comprising a multispecific antibody according to the invention, including formulating the multispecific antibody according to the invention in combination with at least one pharmaceutically acceptable carrier.

Another object of the invention is the multispecific antibody according to the invention for use as a medicament. Another object of the invention is the multispecific antibody according to the invention for use in the treatment of cancer. Another object of the invention is the multispecific antibody according to the invention for use in the treatment of inflammatory diseases, autoimmune diseases, rheumatoid arthritis, psioratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.

Another object of the invention is a pharmaceutical composition comprising a multispecific antibody according to the invention in combination with at least one pharmaceutically acceptable carrier for use as a medicament. Another object of the invention is a pharmaceutical composition comprising a multispecific antibody according to the invention in combination with at least one pharmaceutically acceptable carrier for use in the treatment of cancer. Another object of the invention is a pharmaceutical composition comprising a multispecific antibody according to the invention in combination with at least one pharmaceutically acceptable carrier for use in the treatment of inflammatory diseases, autoimmune diseases, rheumatoid arthritis, psioratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.

Another object of the invention is an immunoconjugate comprising the multispecific antibody according to the invention coupled to a cytotoxic agent for use as a medicament. Another object of the invention is an immunoconjugate comprising the multispecific antibody according to the invention coupled to a cytotoxic agent for use in the treatment of cancer. Another object of the invention is an immunoconjugate comprising the multispecific antibody according to the invention coupled to a cytotoxic agent for use in the treatment of inflammatory diseases, autoimmune diseases, rheumatoid arthritis, psioratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.

Another object of the invention is the use of a multispecific antibody according to the invention for the manufacture of a medicament. Another object of the invention is the use of a multispecific antibody according to the invention for the manufacture of a medicament for the treatment of cancer. Another object of the invention is the use of a multispecific antibody according to the invention for the manufacture of a medicament for the treatment of inflammatory diseases, autoimmune diseases, rheumatoid arthritis, psioratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.

Another object of the invention is a method of treatment of a patient suffering from a disease by administering a multispecific antibody according to the invention to the patient in the need of such treatment. Another object of the invention is a method of treatment of a patient suffering from cancer by administering a multispecific antibody according to the invention to the patient in the need of such treatment. Another object of the invention is a method of treatment of a patient suffering from at least one of the following diseases including inflammatory diseases, autoimmune diseases, rheumatoid arthritis, psioratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and vascular injury; by administering a multispecific antibody according to the invention to the patient in the need of such treatment.

### III) SPECIFIC EMBODIMENTS OF THE INVENTION

In the following specific embodiments of the invention are listed.
1. A multispecific antibody, comprising
   a) a first light chain and a first heavy chain derived from a first antibody which specifically binds to a first antigen; and
   b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D; or
      ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D.
   (with the proviso that the multispecific antibody does not comprise both of the modifications mentioned under i) and ii) and the proviso that in the second light chain the variable domain VL is not replaced by the variable domain VH of the second heavy chain; and in the second heavy chain the variable domain VH is not replaced by the variable domain VL of the second light chain)
2. The multispecific antibody according to embodiment 1, wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K), and wherein in the constant domain CH1 of the first heavy chain the amino acids at position 147 and 213 (numbering according to EU index of Kabat) are substituted independently from each other by an amino acid selected from E or D (in one preferred embodiment by E).
3. The multispecific antibody according to embodiment 1, wherein in the constant domain CL of the second heavy chain the amino acids at position 124 and 123 (numbering according to Kabat) are substituted independently from each other by an amino acid selected from K, R and H (in one preferred embodiment independently from each other by K or R; in one further preferred embodiment by K), and wherein in the constant domain CH1 of the second light chain the amino acids at position 147 and 213 (numbering according to EU index of Kabat) are substituted independently from each other by an amino acid selected from E or D (in one preferred embodiment by E).
4. The multispecific antibody according to embodiment 1, wherein
   i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D, and in the constant domain CL of the second heavy chain the amino acid at position 124 (numbering according to Kabat) is substituted by an amino acid selected from E and D (in one preferred embodiment by E); or
   ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D, and in the constant domain CL of the first light chain the amino acid at position 124 (numbering according to Kabat) is substituted by an amino acid selected from E and D (in one preferred embodiment by E).
5. The multispecific antibody according to embodiment 2, wherein in the constant domain CL of the second heavy chain the amino acid at position 124 (numbering according to Kabat) is substituted by an amino acid selected from E and D (in one preferred embodiment by E).
6. The multispecific antibody according to embodiment 3, wherein in the constant domain CL of the first light chain the amino acid at position 124 (numbering according to Kabat) is substituted by an amino acid selected from E and D (in one preferred embodiment by E).
7. The multispecific antibody according to any one of the preceding embodiments,
   iii) wherein in the variable domain VL of the first light chain the amino acid at position 38 (numbering according to Kabat) is substituted by an amino acid selected from K, R and H (in one preferred embodiment independently from each other by K or R; in one further preferred embodiment by K); and
      wherein in the variable domain VH of the first heavy chain the amino acid at position 39 (numbering according to Kabat) is substituted by an amino acid selected from E and D (in one preferred embodiment by E); and
   iv) wherein in the variable domain VL of the second heavy chain the amino acid at position 38 (numbering according to Kabat) is substituted by an amino acid selected from E and D (in one preferred embodiment E); and wherein in the variable domain VH of the second light chain the amino acid at position 39 (numbering according to Kabat) is substituted by an amino acid selected from K, R and H (in one preferred embodiment by K or R, in one embodiment by K).
8. The multispecific antibody according to any one of the preceding embodiments, wherein the constant domain CL of the first light chain and the constant domain CL of the second heavy chain are of kappa isotype.
9. The multispecific antibody according to any one embodiments 1 to 7, wherein the constant domain CL of the first light chain is of kappa isotype and the constant domain CL of the second heavy chain is of lambda isotype.
10. The multispecific antibody according to any one embodiments 1 to 7, wherein the constant domain CL of the first light chain is of lambda isotype and the constant domain CL of the second heavy chain is of kappa isotype.
11. The multispecific antibody according to any one embodiments 1 to 7, wherein the constant domain CL of the first light chain and the constant domain CL of the second heavy chain are of lambda isotype.
12. The multispecific antibody according to any one of embodiments 1 to 9, wherein the multispecific antibody comprises a constant domain CL of kappa isotype in the first light chain, wherein in the constant domain CL of the first light chain the amino acid E at position 123 (numbering according to Kabat) is substituted by an amino acid selected from K, R and H (in one preferred embodiment by K or R; in one further preferred embodiment by K) and the amino acid E at position 124 (numbering according to Kabat) is substituted by an amino acid selected from K, R and H (in one preferred embodiment by K or R; in one further preferred embodiment by K).
13. The multispecific antibody according to any one of embodiments 1 to 8 and 10, wherein the multispecific antibody comprises a constant domain CL of kappa isotype in the second heavy chain, wherein in the constant domain CL of the second heavy chain the amino acid E at position 123 (numbering according to Kabat) is substituted by an amino acid selected from K, R and H (in one preferred embodiment by K or R; in one further preferred embodiment by K) and the amino acid E at position 124 (numbering according to Kabat) is substituted by an amino acid selected from K, R and H (in one preferred embodiment by K or R; in one further preferred embodiment by K).
14. The multispecific antibody according to any one of embodiments 1 to 7, 10 and 11, wherein the multispecific antibody comprises a constant domain CL of lambda isotype in the first light chain, wherein in the constant domain CL of the first light chain the amino acid E at position 123 (numbering according to Kabat) is substituted by an amino acid selected from K, R and H (in one preferred embodiment independently from each other by K or R; in one further preferred embodiment by K) and the amino acid Q at position 124 (numbering according to Kabat) is substituted by an amino acid selected from K, R and H (in one preferred embodiment independently from each other by K or R; in one further preferred embodiment by K).
15. The multispecific antibody according to any one of embodiments 1 to 7, 9 and 11, wherein the multispecific antibody comprises a constant domain CL of lambda isotype in the second heavy chain, wherein in the constant domain CL of the second heavy chain the amino acid E at position 123 (numbering according to Kabat) is substituted by an amino acid selected from K, R and H (in one preferred embodiment independently from each other by K or R; in one further preferred embodiment by K) and the amino acid Q at position 124 (numbering according to Kabat) is substituted by an amino acid selected from K, R and H (in one preferred embodiment independently from each other by K or R; in one further preferred embodiment by K).
16. The multispecific antibody according to any one of the preceding embodiments, wherein in the tertiary structure of the antibody the CH3 domain of the first heavy chain and the CH3 domain of the second heavy chain form an interface that is located between the respective antibody CH3 domains, wherein the respective amino acid sequences of the CH3 domain of the first heavy chain and the CH3 domain of the second heavy chain each comprise a set of amino acids that is located within said interface in the tertiary structure of the antibody,
   - wherein from the set of amino acids that is located in the interface in the CH3 domain of one heavy chain at least one amino acid residue is substituted by an amino acid residue having a larger side chain volume than the original amino acid residue, thereby generating a protuberance within the interface, wherein the protuberance is located in the CH3 domain of the one heavy chain, and wherein the protuberance is positionable in a cavity located in the CH3 domain of the other heavy chain within the interface; and
   - wherein from the set of amino acids that is located in the interface in the CH3 domain of the other heavy chain at least one amino acid residue is substituted by an amino acid residue having a smaller side chain volume than the original amino acid residue, thereby generating a cavity within the interface, wherein the cavity is located in the CH3 domain of the other heavy chain, and wherein in the cavity the protuberance within the interface located in the CH3 domain of the one heavy chain is positionable.
17. The multispecific antibody according to embodiment 16, wherein said amino acid residue having a larger side chain volume than the original amino acid residue is selected from R, F, Y and W; and/or wherein said amino acid residue having a smaller side chain volume than the original amino acid residue is selected from A, S, T and V.
18. The multispecific antibody according to any one of embodiments 16 or 17, wherein in the CH3 domain of the one heavy chain the amino acid T at position 366 (numbering according to EU index of Kabat) is substituted by W; and in the CH3 domain of the other heavy chain the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V.
19. The multispecific antibody according to any one of embodiments 16 to 18, wherein in the CH3 domain of the one heavy chain the amino acid T at position 366 (numbering according to EU index of Kabat) is substituted by W, the amino acid R at position 409 (numbering according to EU index of Kabat) is substituted by D, and the amino acid K at position 370 (numbering according to EU index of Kabat) is substituted by E; and in the CH3 domain of the other heavy chain the amino acid T at position 366 (numbering according to EU index of Kabat) is substituted by S, the amino acid L at position 368 (numbering according to EU index of Kabat) is substituted by A and the amino acid Y at position 407 (numbering according to EU index of Kabat) is substituted by V, the amino acid D at position 399 (numbering according to EU index of Kabat) is substituted by K, and the amino acid E at position 357 (numbering according to EU index of Kabat) is substituted by K.
20. The multispecific antibody according to any one of embodiments 16 to 19, wherein from the set of amino acids that is located in the interface in the CH3 domain of the one heavy chain a first amino acid is substituted by cysteine; and from the set of amino acids that is located in the interface in the CH3 domain of the other heavy chain a second amino acid is substituted by cysteine, wherein the second amino acid is facing the first amino acid within the interface; such that a disulfide bridge between the CH3 domain of the one heavy chain and the CH3 domain of the other heavy chain can be formed via the introduced cysteine residues.
21. The multispecific antibody according to embodiment 20, wherein in the CH3 domain of the one heavy chain either the amino acid E at position 356 (numbering according to EU index of Kabat) or the amino acid S at position 354 (numbering according to EU index of Kabat) is substituted by C and in the CH3 domain of the other heavy chain the amino acid Y at position 349 (numbering according to EU index of Kabat) is substituted by C.
22. The multispecific antibody according to embodiment 20, wherein in the CH3 domain of the one heavy chain the amino acid Y at position 349 (numbering according to EU index of Kabat) is substituted by C; and in the CH3 domain of the other heavy chain the amino acid S at position 354 (numbering according to EU index of Kabat) is substituted by C.
23. The multispecific antibody according to any one of the preceding embodiments, wherein the antibody is bispecific, trispecific or tetraspecific.
24. The multispecific antibody according to any one of the preceding embodiments, wherein the antibody is bispecific or trispecific.
25. The multispecific antibody according to any one of the preceding embodiments, wherein the antibody is bispecific.
26. The multispecific antibody according to any one of the preceding embodiments, wherein the antibody is bivalent or trivalent.
27. The multispecific antibody according to any one of the preceding embodiments, wherein the antibody is bivalent.
28. The multispecific antibody according to any one of the preceding embodiments, wherein the antibody has a constant domain structure of an IgG type antibody.
29. The multispecific antibody according to any one of the preceding embodiments, wherein the antibody is of human IgG1 or human IgG4 subclass.
30. The multispecific antibody according to any one of the preceding embodiments, wherein the antibody is of human IgG1 subclass with the mutations L234A and L235A (numbering according to EU index of Kabat).
31. The multispecific antibody according to any one of the preceding embodiments, wherein the antibody is of human IgG4 subclass with the mutations S228P and L235E (numbering according to EU index of Kabat).
32. The multispecific antibody according to embodiment 30 or 31, wherein the antibody further comprises a P329G mutation (numbering according to EU index of Kabat).
33. The multispecific antibody according to any one of embodiments 1 to 15, 23 to 27, wherein the antibody is devoid of Fc domains.
34. The multispecific antibody according to any one of the preceding embodiments that specifically binds to human Angiopoietin-2 and human VEGF, wherein
   a) the antibody comprises a variable heavy chain domain (VH) according to SEQ ID NO: 26 (<VH Ang2>) and a variable light chain domain (VL) according to SEQ ID NO: 27 (<VL Ang2>); and
   b) the antibody comprises a variable heavy chain domain (VH) according to SEQ ID NO: 24 (<VH VEGF>) and a variable light chain domain (VL) according to SEQ ID NO: 25 (<VL VEGF>).
35. The multispecific antibody according to any one of the preceding embodiments that specifically binds to human Angiopoietin-2 and human VEGF, wherein
   a) the first antibody comprises a variable heavy chain domain (VH) according to SEQ ID NO: 26 (<VH Ang2>) and a variable light chain domain (VL) according to SEQ ID NO: 27 (<VL Ang2>); and
   b) the second antibody comprises a variable heavy chain domain (VH) according to SEQ ID NO: 24 (<VH VEGF>) and a variable light chain domain (VL) according to SEQ ID NO: 25 (<VL VEGF>).
36. A bispecific antibody specifically binding to human Angiopoietin-2 and human VEGF, comprising
   a) a first light chain and a first heavy chain derived from a first antibody which specifically binds to human Angiopoietin-2, which in one preferred embodiment comprises a variable heavy chain domain according to SEQ ID NO: 26 and a variable light chain domain according to SEQ ID NO: 27; and
   b) a second light chain and a second heavy chain derived from a second antibody which specifically binds to human VEGF, which in one preferred embodiment comprises a variable heavy chain domain according to SEQ ID NO: 24 and a variable light chain domain according to SEQ ID NO: 25, wherein in the second light chain the constant domain CL is replaced by the constant domain CH1 of the second heavy chain; and
      in the second heavy chain the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E); or
      ii) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
37. A bispecific antibody specifically binding to human Angiopoietin-2 and human VEGF, comprising
   a) a first light chain and a first heavy chain derived from a first antibody which specifically binds to human Angiopoietin-2, which in one preferred embodiment comprises a variable heavy chain domain according to SEQ ID NO: 26 and a variable light chain domain according to SEQ ID NO: 27; and
   b) a second light chain and a second heavy chain derived from a second antibody which specifically binds to human VEGF, which in one preferred embodiment comprises a variable heavy chain domain according to SEQ ID NO: 24 and a variable light chain domain according to SEQ ID NO: 25, wherein in the second light chain the constant domain CL is replaced by the constant domain CH1 of the second heavy chain; and
      in the second heavy chain the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
38. A bispecific antibody specifically binding to human Angiopoietin-2 and human VEGF, comprising
   a) a first light chain and a first heavy chain derived from a first antibody which specifically binds to human Angiopoietin-2, which in one preferred embodiment comprises a variable heavy chain domain according to SEQ ID NO: 26 and a variable light chain domain according to SEQ ID NO: 27; and
   b) a second light chain and a second heavy chain derived from a second antibody which specifically binds to human VEGF, which in one preferred embodiment comprises a variable heavy chain domain according to SEQ ID NO: 24 and a variable light chain domain according to SEQ ID NO: 25, wherein in the second light chain the constant domain CL is replaced by the constant domain CH1 of the second heavy chain; and
      in the second heavy chain the constant domain CH1 is replaced by the constant domain CL of the second light chain; and
      i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E).
39. The bispecific antibody specifically binding to human Angiopoietin-2 and human VEGF according to embodiment 36,
   i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the second heavy chain the amino acid at position 124 (numbering according to Kabat) is substituted by an amino acid selected from E and D (in one preferred embodiment by E); or
   ii) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the first light chain the amino acid at position 124 (numbering according to Kabat) is substituted by an amino acid selected from E and D (in one preferred embodiment by E).
40. The bispecific antibody specifically binding to human Angiopoietin-2 and human VEGF according to embodiment 37,
   i) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the second heavy chain the amino acid at position 124 (numbering according to Kabat) is substituted by an amino acid selected from E and D (in one preferred embodiment by E).
41. The bispecific antibody specifically binding to human Angiopoietin-2 and human VEGF according to embodiment 38,
   ii) wherein in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and wherein in the constant domain CH1 of the first heavy chain the amino acids at position 147 and 213 (numbering according to EU index of Kabat) are substituted independently from each other by an amino acid selected from E or D (in one preferred embodiment by E), and wherein in the constant domain CL of the first light chain the amino acid at position 124 (numbering according to Kabat) is substituted by an amino acid selected from E and D (in one preferred embodiment by E).
42. The bispecific antibody specifically binding to human Angiopoietin-2 and human VEGF according to any one of embodiments 36 to 41,
   - wherein in the variable domain VL of the first light chain the amino acid at position 38 (numbering according to Kabat) is substituted by an amino acid selected from K, R and H (in one preferred embodiment independently from each other by K or R; in one further preferred embodiment by K); and wherein in the variable domain VH of the first heavy chain the amino acid at position 39 (numbering according to Kabat) is substituted by an amino acid selected from E and D (in one preferred embodiment by E); and
   - wherein in the variable domain VL of the second heavy chain the amino acid at position 38 (numbering according to Kabat) is substituted by an amino acid selected from E and D (in one preferred embodiment E); and wherein in the variable domain VH of the second light chain the amino acid at position 39 (numbering according to Kabat) is substituted by an amino acid selected from K, R and H (in one preferred embodiment by K or R, in one embodiment by K).
43. The antibody according to any one of the preceding embodiments, for use as a medicament.
44. The antibody according to any one of embodiments 1 to 42, for use in the treatment of cancer.
45. The antibody according to any one of embodiments 1 to 42, for use in the treatment of inflammatory diseases, autoimmune diseases, rheumatoid arthritis, psioratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.
46. Use of the antibody according to any one of embodiments 1 to 42 for the manufacture of a medicament, in one preferred embodiment for the treatment of cancer or for the treatment of inflammatory diseases, autoimmune diseases, rheumatoid arthritis, psioratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.
47. A method for the preparation of a multispecific antibody according to any one of embodiments 1 to 42, comprising the steps of
   - transforming a host cell with vectors comprising nucleic acids encoding
      a) the first light chain as defined in one of embodiments 1 to 42 derived from a first antibody which specifically binds to a first antigen;
      b) the first heavy chain as defined in one of embodiments 1 to 42 derived from a first antibody which specifically binds to a first antigen;
      c) the second light chain as defined in one of embodiments 1 to 42 derived from a second antibody which specifically binds to a second antigen; and
      d) the second heavy chain as defined in one of embodiments 1 to 42 derived from a second antibody which specifically binds to a second antigen,
   - culturing said host cell under conditions that allow synthesis of said multispecific antibody; and
   - recovering said multispecific antibody from said host cell culture.
48. A method for the reduction of side product formation during the preparation and/or for increasing the aggregation temperature of a multispecific antibody, in one preferred embodiment according to any one of the preceding embodiments, comprising the steps of
   - transforming a host cell with vectors comprising nucleic acids encoding
      a) a first light chain and a first heavy chain derived from a first antibody which specifically binds to a first antigen; and
      b) a second light chain and a second heavy chain derived from a second antibody which specifically binds to a second antigen, wherein in the second light chain the constant domain CL is replaced by the constant domain CH1 of the second heavy chain; and in the second heavy chain the constant domain CH1 is replaced by the constant domain CL of the second light chain;
         and wherein in order to reduce the formation of side product of the multispecific antibody:
         i) in the constant domain CL of the first light chain the amino acids at position 124 and 123 (numbering according to Kabat) are substituted independently from each other by an amino acid selected from K, R and H; and in the constant domain CH1 of the first heavy chain the amino acids at position 147 and 213 (numbering according to EU index of Kabat) are substituted independently from each other by an amino acid selected from E or D; or
         ii) in the constant domain CL of the second heavy chain the amino acids at position 124 and 123 (numbering according to Kabat) are substituted independently from each other by an amino acid selected from K, R and H; and in the constant domain CH1 of the second light chain the amino acids at position 147 and 213 (numbering according to EU index of Kabat) are substituted independently from each other by an amino acid selected from E or D;
   - culturing said host cell under conditions that allow synthesis of said multispecific antibody; and
   - recovering said multispecific antibody from said host cell culture.
49. The method according to embodiment 48, wherein in order to reduce the formation of side product of the multispecific antibody:
   i) in the constant domain CL of the first light chain the amino acids at position 124 and 123 (numbering according to Kabat) are substituted independently from each other by an amino acid selected from K, R and H; and in the constant domain CH1 of the first heavy chain the amino acids at position 147 and 213 (numbering according to EU index of Kabat) are substituted independently from each other by an amino acid selected from E or D.
50. The method according to embodiment 48, wherein in order to reduce the formation of side product of the multispecific antibody:
   i) in the constant domain CL of the second heavy chain the amino acids at position 124 and 123 (numbering according to Kabat) are substituted independently from each other by an amino acid selected from K, R and H; and in the constant domain CH1 of the second light chain the amino acids at position 147 and 213 (numbering according to EU index of Kabat) are substituted independently from each other by an amino acid selected from E or D.
51. The method according to embodiment 48, wherein in order to reduce the formation of side product of the multispecific antibody
   i) in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and in the constant domain CH1 of the first heavy chain the amino acids at position 147 and 213 (numbering according to EU index of Kabat) are substituted independently from each other by an amino acid selected from E or D (in one preferred embodiment by E), and in the constant domain CL of the second heavy chain the amino acid at position 124 (numbering according to Kabat) is substituted by an amino acid selected from E and D (in one preferred embodiment by E); or
   ii) in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and in the constant domain CH1 of the first heavy chain the amino acids at position 147 and 213 (numbering according to EU index of Kabat) are substituted independently from each other by an amino acid selected from E or D (in one preferred embodiment by E), and in the constant domain CL of the first light chain the amino acid at position 124 (numbering according to Kabat) is substituted by an amino acid selected from E and D (in one preferred embodiment by E).
52. The method according to embodiment 49, wherein in order to reduce the formation of side product and/or to increase the aggregation temperature of the multispecific antibody
   i) in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and in the constant domain CH1 of the first heavy chain the amino acids at position 147 and 213 (numbering according to EU index of Kabat) are substituted independently from each other by an amino acid selected from E or D (in one preferred embodiment by E), and in the constant domain CL of the second heavy chain the amino acid at position 124 (numbering according to Kabat) is substituted by an amino acid selected from E and D (in one preferred embodiment by E).
53. The method according to embodiment 50, wherein in order to reduce the formation of side product and/or to increase the aggregation temperature of the multispecific antibody
   i) in the constant domain CL of the first light chain the amino acids at position 124 and 123 (numbering according to Kabat) are substituted independently from each other by an amino acid selected from K, R and H (in one preferred embodiment independently from each other by K or R; in one further preferred embodiment by K); and in the constant domain CH1 of the first heavy chain the amino acids at position 147 and 213 (numbering according to EU index of Kabat) are substituted independently from each other by an amino acid selected from E or D (in one preferred embodiment by E), and in the constant domain CL of the first light chain the amino acid at position 124 (numbering according to Kabat) is substituted by an amino acid selected from E and D (in one preferred embodiment by E).
54. The method according to any one of embodiments 48 to 53, wherein in order to reduce the formation of side product and/or to increase the aggregation temperature of the multispecific antibody the following amino acid substitutions are included
   - in the variable domain VL of the first light chain the amino acid at position 38 (numbering according to Kabat) is substituted by an amino acid selected from K, R and H (in one preferred embodiment independently from each other by K or R; in one further preferred embodiment by K); and in the variable domain VH of the first heavy chain the amino acid at position 39 (numbering according to Kabat) is substituted by an amino acid selected from E and D (in one preferred embodiment by E); and
   - wherein in the variable domain VL of the second heavy chain the amino acid at position 38 (numbering according to Kabat) is substituted by an amino acid selected from E and D (in one preferred embodiment E); and wherein in the variable domain VH of the second light chain the amino acid at position 39 (numbering according to Kabat) is substituted by an amino acid selected from K, R and H (in one preferred embodiment by K or R, in one embodiment by K).
55. A multispecific antibody produced by a method according to embodiment 47.
56. A nucleic acid encoding the amino acid sequence of
   a) a first light chain as defined in one of embodiments 1 to 42,
   b) a second light chain as defined in one of embodiments 1 to 42,
   c) a first heavy chain as defined in one of embodiments 1 to 42; or
   d) a second heavy chain as defined in one of embodiments 1 to 42.
57. A vector comprising a nucleic acid according to embodiment 56, wherein the vector is capable of expressing said nucleic acid in a host cell.
58. An expression vector comprising a nucleic acid according to embodiment 56, wherein the vector is capable of expressing said nucleic acid in a host cell.
59. A host cell comprising a nucleic acid according to embodiment 56.
60. A host cell comprising a vector according to embodiment 57 or an expression vector according to embodiment 57.
61. A pharmaceutical composition comprising a multispecific antibody according to any one of embodiments 1 to 42 in combination with at least one pharmaceutically acceptable carrier.
62. The pharmaceutical composition according to embodiment 61, for use as a medicament.
63. The pharmaceutical composition according to embodiment 61, for use in the treatment of cancer.
64. The pharmaceutical composition according to embodiment 61, for use in the treatment of inflammatory diseases, autoimmune diseases, rheumatoid arthritis, psioratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.
65. An immunoconjugate comprising the antibody of one of embodiments 1 to 42 coupled to a cytotoxic agent.
66. The immunoconjugate according to embodiment 65 for use as a medicament.
67. The immunoconjugate according to embodiment 65 for use in the treatment of cancer, or in the treatment of inflammatory diseases, autoimmune diseases, rheumatoid arthritis, psioratic arthritis, muscle diseases (e.g. muscular dystrophy), multiple sclerosis, chronic kidney diseases, bone diseases (e.g. bone degeneration in multiple myeloma), systemic lupus erythematosus, lupus nephritis, and/or vascular injury.

**DESCRIPTION OF THE AMINO ACID SEQUENCES**

| | |
|---|---|
| SEQ ID NO: 1 | 7571 - kappa light chain (LC) <Ang-2> wild type (wt) |
| SEQ ID NO:2 | 7595 - heavy chain (HC) <Ang-2> wild type (wt) |
| SEQ ID NO:3 | 7594 - light chain (LC) <VEGF> with CL-CH1 domain exchange wild type (wt) |
| SEQ ID NO:4 | 7593 - heavy chain (HC) <VEGF> with CL-CH1 domain exchange wild type (wt) |
| SEQ ID NO:5 | 17914 - kappa light chain (LC) <Ang-2> with Q124K substitution |
| SEQ ID NO:6 | 17911 - heavy chain (HC) <Ang-2> with K147E substitution |
| SEQ ID NO:7 | 17912 - heavy chain (HC) <Ang-2> with K213E substitution |
| SEQ ID NO:8 | 17913 - kappa light chain (LC) <Ang-2> with E123K substitution |
| SEQ ID NO:9 | 17932 - light chain (LC) <VEGF> with CL-CH1 domain exchange and K147E substitution |
| SEQ ID NO: 10 | 17934 - heavy chain (HC) <VEGF> with CL-CH1 domain exchange and Q124K substitution |
| SEQ ID NO: 11 | 17933 - light chain (LC) <VEGF> with CL-CH1 domain exchange and K213E substitution |
| SEQ ID NO:12 | 17301 - kappa light chain (LC) <Ang-2> with E123R and Q124K substitutions |
| SEQ ID NO:13 | 14116 - heavy chain (HC) <Ang-2> with K147E and K213E substitutions |
| SEQ ID NO:14 | 17305 - heavy chain (HC) <Ang-2> with K147E and K213D substitutions |
| SEQ ID NO:15 | 17953 - kappa light chain (LC) <Ang-2> with Q38E, E123K and Q124K substitutions |
| SEQ ID NO:16 | 17954 - heavy chain (HC) <Ang-2> with Q39K, K147E and K213E substitutions |
| SEQ ID NO: 17 | 17938 - light chain (LC) <VEGF> with CL-CH1 domain exchange and Q38K substitution |
| SEQ ID NO:18 | 17936 - heavy chain (HC) <VEGF> with CL-CH1 domain exchange and Q39E substitution |
| SEQ ID NO:19 | 14098 - kappa light chain (LC) <Ang-2> with Q38K, E123K and Q124K substitutions |
| SEQ ID NO:20 | 14097 - heavy chain (HC) <Ang-2> with Q39E, K147E and K213E substitutions |
| SEQ ID NO:21 | 17937 - heavy chain (HC) <VEGF> with CL-CH1 domain exchange and with Q39E and Q124E substitutions |
| SEQ ID NO:22 | 17962 - kappa light chain (LC) <Ang-2> with Q124E substitution |
| SEQ ID NO:23 | 17935 - heavy chain (HC) <VEGF> with CL-CH1 domain exchange and Q124E substitution |
| SEQ ID NO:24 | variable heavy chain domain <VEGF> |
| SEQ ID NO:25 | variable light chain domain <VEGF> |
| SEQ ID NO:26 | variable heavy chain domain <Ang-2> |
| SEQ ID NO:27 | variable light chain domain <Ang-2> |
| SEQ ID NO:28 | 6413 - IL17 LC E123 (Wt)/ Q124 (Wt) |
| SEQ ID NO:29 | 11738 - IL17 HC K147 (Wt)/ K213(Wt) |
| SEQ ID NO:30 | 11755 - Tweak xLC K147(Wt)/ K213(Wt) |
| SEQ ID NO:31 | 11754 - Tweak xHC E123(Wt)/ Q124(Wt) |
| SEQ ID NO:32 | 17843 - IL17 LC Q124K |
| SEQ ID NO:33 | 17844 - IL17 HC K147E |
| SEQ ID NO:34 | 17845 - IL17 HC K213E |
| SEQ ID NO:35 | 17846 - IL17 LC E123K |
| SEQ ID NO:36 | 17847 - IL17 LC E123R / Q124K |
| SEQ ID NO:37 | 17848 - IL17 HC K147E / K213E |
| SEQ ID NO:38 | 17849 - IL17 HC K147E / K213D |
| SEQ ID NO:39 | 17850 - Tweak xHC Q124E |

### EXAMPLES

The following examples are provided to aid the understanding of the present invention, the true scope of which is set forth in the appended claims. It is understood that modifications can be made in the procedures set forth without departing from the spirit of the invention.

In the following section, if not stated otherwise, LC* denotes the modified light chain <CH1-VL> and HC* denotes the modified heavy chain <CH3-CH2-CL-VH>.

### Materials & general methods

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991). Amino acids of antibody chains are numbered and referred to according to numbering according to Kabat (Kabat, E.A., et al., Sequences of Proteins of Immunological Interest, 5th ed., Public Health Service, National Institutes of Health, Bethesda, MD (1991)).

### Recombinant DNA techniques

Standard methods were used to manipulate DNA as described in Sambrook, J. et al., Molecular Cloning: A laboratory manual; Cold Spring Harbor Laboratory Press, Cold Spring Harbor, New York, 1989. The molecular biological reagents were used according to the manufacturer's instructions.

### Gene synthesis

Desired gene segments were prepared from oligonucleotides made by chemical synthesis. The 600 - 1800 bp long gene segments, which were flanked by singular restriction endonuclease cleavage sites, were assembled by annealing and ligating oligonucleotides including PCR amplification and subsequently cloned via the indicated restriction sites e.g. KpnI/SacI or AscI/PacI into a pPCRScript (Stratagene) based pGA4 cloning vector. The DNA sequences of the subcloned gene fragments were confirmed by DNA sequencing. Gene synthesis fragments were ordered according to given specifications at Geneart (Regensburg, Germany).

### DNA sequence determination

DNA sequences were determined by double strand sequencing performed at MediGenomix GmbH (Martinsried, Germany) or Sequiserve GmbH (Vaterstetten, Germany).

### DNA and protein sequence analysis and sequence data management

The GCG's (Genetics Computer Group, Madison, Wisconsin) software package version 10.2 and Infomax's Vector NT1 Advance suite version 8.0 was used for sequence creation, mapping, analysis, annotation and illustration.

### Expression vectors

For the expression of the described antibodies, variants of expression plasmids for transient expression (e.g. in HEK293 EBNA or HEK293-F) cells based either on a cDNA organization with or without a CMV-Intron A promoter or on a genomic organization with a CMV promoter were applied.

Beside the antibody expression cassette the vectors contained:
- an origin of replication which allows replication of this plasmid in *E. coli,* and
- a ß-lactamase gene which confers ampicillin resistance in *E. coli.*

The transcription unit of the antibody gene was composed of the following elements:
- unique restriction site(s) at the 5' end
- the immediate early enhancer and promoter from the human cytomegalovirus,
- followed by the Intron A sequence in the case of the cDNA organization,
- a 5'-untranslated region of a human antibody gene,
- an immunoglobulin heavy chain signal sequence,
- the human antibody chain (wildtype or with domain exchange) either as cDNA or as genomic organization with the immunoglobulin exon-intron organization
- a 3' untranslated region with a polyadenylation signal sequence, and
- unique restriction site(s) at the 3' end.

The fusion genes comprising the antibody chains as described below were generated by PCR and/or gene synthesis and assembled by known recombinant methods and techniques by connection of the according nucleic acid segments e.g. using unique restriction sites in the respective vectors. The subcloned nucleic acid sequences were verified by DNA sequencing. For transient transfections larger quantities of the plasmids were prepared by plasmid preparation from transformed *E. coli* cultures (Nucleobond AX, Macherey-Nagel).

### Cell culture techniques

Standard cell culture techniques were used as described in Current Protocols in Cell Biology (2000), Bonifacino, J.S., Dasso, M., Harford, J.B., Lippincott-Schwartz, J. and Yamada, K.M. (eds.), John Wiley & Sons, Inc.

Multispecific antibodies were expressed by transient co-transfection of the respective expression plasmids in adherently growing HEK293-EBNA or in HEK29-F cells growing in suspension as described below.

### Transient transfections in HEK293-EBNA system

Multispecific antibodies were expressed by transient co-transfection of the respective expression plasmids (e.g. encoding the heavy and modified heavy chain, as well as the corresponding light and modified light chain) in adherently growing HEK293-EBNA cells (human embryonic kidney cell line 293 expressing Epstein-Barr-Virus nuclear antigen; American type culture collection deposit number ATCC # CRL-10852, Lot. 959 218) cultivated in DMEM (Dulbecco's modified Eagle's medium, Gibco®) supplemented with 10% Ultra Low IgG FCS (fetal calf serum, Gibco®), 2 mM L-Glutamine (Gibco®), and 250 µg/ml Geneticin (Gibco®). For transfection FuGENE™ 6 Transfection Reagent (Roche Molecular Biochemicals) was used in a ratio of FuGENE™ reagent (µl) to DNA (µg) of 4:1 (ranging from 3:1 to 6:1). Proteins were expressed from the respective plasmids using an equimolar ratio of (modified and wildtype) light chain and (modified and wildtype) heavy chain encoding plasmids. Cells were fed at day 3 with L-Glutamine ad 4 mM, Glucose [Sigma] and NAA [Gibco®]. Multispecific antibody containing cell culture supernatants were harvested from day 5 to 11 after transfection by centrifugation and stored at -20°C. General information regarding the recombinant expression of human immunoglobulins in e.g. HEK293 cells is given in: Meissner, P. et al., Biotechnol. Bioeng. 75 (2001) 197-203.

### Transient transfections in HEK293-F system

Multispecific antibodies were generated by transient transfection with the respective plasmids (e.g. encoding the heavy and modified heavy chain, as well as the corresponding light and modified light chain) using the HEK293-F system (Invitrogen) according to the manufacturer's instruction. Briefly, HEK293-F cells (Invitrogen) growing in suspension either in a shake flask or in a stirred fermenter in serum-free FreeStyle™ 293 expression medium (Invitrogen) were transfected with a mix of the four expression plasmids and 293fectin™ or fectin (Invitrogen). For 2 L shake flask (Corning) HEK293-F cells were seeded at a density of 1.0E*6 cells/mL in 600 mL and incubated at 120 rpm, 8% CO2. The day after the cells were transfected at a cell density of ca. 1.5E*6 cells/mL with ca. 42 mL mix of A) 20 mL Opti-MEM (Invitrogen) with 600 µg total plasmid DNA (1 µg/mL) encoding the heavy or modified heavy chain, respectively and the corresponding light or modified light chain in an equimolar ratio and B) 20 ml Opti-MEM + 1.2 mL 293 fectin or fectin (2 µl/mL). According to the glucose consumption glucose solution was added during the course of the fermentation. The supernatant containing the secreted antibody was harvested after 5-10 days and antibodies were either directly purified from the supernatant or the supernatant was frozen and stored.

### Protein determination

The protein concentration of purified antibodies and derivatives was determined by determining the optical density (OD) at 280 nm, using the molar extinction coefficient calculated on the basis of the amino acid sequence according to Pace, et al., Protein Science, 1995, 4, 2411-1423.

### Antibody concentration determination in supernatants

The concentration of antibodies and derivatives in cell culture supernatants was estimated by immunoprecipitation with Protein A Agarose-beads (Roche). 60 µL Protein A Agarose beads were washed three times in TBS-NP40 (50 mM Tris, pH 7.5, 150 mM NaCl, 1% Nonidet-P40). Subsequently, 1-15 mL cell culture supernatant were applied to the Protein A Agarose beads pre-equilibrated in TBS-NP40. After incubation for at 1 hour at room temperature the beads were washed on an Ultrafree-MC-filter column (Amicon) once with 0.5 mL TBS-NP40, twice with 0.5 mL 2x phosphate buffered saline (2xPBS, Roche) and briefly four times with 0.5 mL 100 mM Na-citrate pH 5,0. Bound antibody was eluted by addition of 35 µl NuPAGE® LDS Sample Buffer (Invitrogen). Half of the sample was combined with NuPAGE® Sample Reducing Agent or left unreduced, respectively, and heated for 10 min at 70°C. Consequently, 5-30 µl were applied to a 4-12% NuPAGE® Bis-Tris SDS-PAGE (Invitrogen) (with MOPS buffer for non-reduced SDS-PAGE and MES buffer with NuPAGE® Antioxidant running buffer additive (Invitrogen) for reduced SDS-PAGE) and stained with Coomassie Blue.

The concentration of antibodies and derivatives in cell culture supernatants was quantitatively measured by affinity HPLC chromatography. Briefly, cell culture supernatants containing antibodies and derivatives that bind to Protein A were applied to an Applied Biosystems Poros A/20 column in 200 mM KH2PO4, 100 mM sodium citrate, pH 7.4 and eluted from the matrix with 200 mM NaCl, 100 mM citric acid, pH 2,5 on an Agilent HPLC 1100 system. The eluted protein was quantified by UV absorbance and integration of peak areas. A purified standard IgG1 antibody served as a standard.

Alternatively, the concentration of antibodies and derivatives in cell culture supernatants was measured by Sandwich-IgG-ELISA. Briefly, StreptaWell High Bind Strepatavidin A-96 well microtiter plates (Roche) are coated with 100 µL/well biotinylated anti-human IgG capture molecule F(ab')2<h-Fcγ> BI (Dianova) at 0.1 µg/mL for 1 hour at room temperature or alternatively overnight at 4°C and subsequently washed three times with 200 µL/well PBS, 0.05% Tween (PBST, Sigma). 100 µL/well of a dilution series in PBS (Sigma) of the respective antibody containing cell culture supernatants was added to the wells and incubated for 1-2 hour on a microtiterplate shaker at room temperature. The wells were washed three times with 200 µL/well PBST and bound antibody was detected with 100 µl F(ab')2<hFcγ>POD (Dianova) at 0.1 µg/mL as the detection antibody for 1-2 hours on a microtiterplate shaker at room temperature. Unbound detection antibody was washed away three times with 200 µL/well PBST and the bound detection antibody was detected by addition of 100 µL ABTS/well. Determination of absorbance was performed on a Tecan Fluor Spectrometer at a measurement wavelength of 405 nm (reference wavelength 492 nm).

### Protein purification

Proteins were purified from filtered cell culture supernatants referring to standard protocols. In brief, antibodies were applied to a Protein A Sepharose column (GE healthcare) and washed with PBS. Elution of antibodies was achieved at pH 2.8 followed by immediate neutralization of the sample. Aggregated protein was separated from monomeric antibodies by size exclusion chromatography (Superdex 200, GE Healthcare) in PBS or in 20 mM Histidine, 150 mM NaCl pH 6.0. Monomeric antibody fractions were pooled, concentrated (if required) using e.g., a MILLIPORE Amicon Ultra (30 MWCO) centrifugal concentrator, frozen and stored at -20°C or -80°C. Part of the samples were provided for subsequent protein analytics and analytical characterization e.g. by SDS-PAGE, size exclusion chromatography (SEC) or mass spectrometry.

### SDS-PAGE

The NuPAGE® Pre-Cast gel system (Invitrogen) was used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer was used.

### CE-SDS

Purity and antibody integrity were analyzed by CE-SDS using microfluidic Labchip technology (PerkinElmer, USA). 5 µl of protein solution was prepared for CE-SDS analysis using the HT Protein Express Reagent Kit according manufacturer's instructions and analyzed on LabChip GXII system using a HT Protein Express Chip. Data were analyzed using LabChip GX Software.

### Analytical size exclusion chromatography

Size exclusion chromatography (SEC) for the determination of the aggregation and oligomeric state of antibodies was performed by HPLC chromatography. Briefly, Protein A purified antibodies were applied to a Tosoh TSKgel G3000SW column in 300 mM NaCl, 50 mM KH2PO4/K2HPO4, pH 7.5 on an Dionex Ultimate® system (Thermo Fischer Scientific) or to a Superdex 200 column (GE Healthcare) in 2 x PBS on a Dionex HPLC-System. The eluted protein was quantified by UV absorbance and integration of peak areas. BioRad Gel Filtration Standard 151-1901 served as a standard.

### Mass spectrometry

This section describes the characterization of the multispecific antibodies with CL-CH1 domain exchange (*CrossMAb^{Ch1-CL}*) with emphasis on their correct assembly. The expected primary structures were analyzed by electrospray ionization mass spectrometry (ESI-MS) of the deglycosylated intact CrossMAbs and in special cases of the deglycosylated/limited LysC digested CrossMabs.

The CrossMab^{Ch1-CL}s were deglycosylated with N-Glycosidase F in a phosphate or Tris buffer at 37°C for up to 17 h at a protein concentration of 1 mg/ml. The limited LysC (Roche) digestions were performed with 100 µg deglycosylated CrossMab^{CH1-CL}s in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

### Thermal stabilities (T_{agg} and Tₘ)

Samples were prepared at 1 mg/mL in 20 mM Histidine chloride, 140 mM NaCl, pH 6.0 and transferred in a 9 µL multi-cuvette array. The multi-cuvette array was heated from 35 °C to 90 °C at a constant rate of 0.1 °C/minute in an Optim1000 instrument (Avacta Analytical Inc.) . The instrument continuously records the intensity of scattered light of a 266 nm laser with a data point approximately every 0.5 °C. Light scattering intensities were plotted against the temperature. The aggregation onset temperature (Tagg) is defined as the

### Determination of binding and binding affinity of multispecific antibodies to the respective antigens using surface plasmon resonance (SPR) (BIACORE)

### VEGF binding was assessed according to the following procedure:

Binding of indicated antibodies to human VEGFA-121 was investigated by surface plasmon resonance using a BIACORE® T200 instrument (GE Healthcare). Around 10000 (RU) of anti His antibody (1 µg/ml anti His antibody; Order Code: 28995056; GE Healthcare Bio-Sciences AB, Sweden) were coupled on a Series S CM5 chip (GE Healthcare BR-1005-30) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was PBS-T (10 mM phosphate buffered saline including 0.05% Tween20) at pH 7.4. The flow cell was set to 25 °C - and the sample block set to 12 °C - and primed with running buffer twice prior to kinetic characterization.

VEFG-A-121-His was captured by injecting a 0.5 µg/ml solution for 30 sec at a flow of 5 µl/min. The association was measured by injection of the indicated antibodies in various concentrations in solution for 180 sec at a flow of 30 µl/min starting with 1000 nM in 1:3 serial dilutions. The dissociation phase was monitored for up to 600 sec and triggered by switching from the sample solution to running buffer. The surface was regenerated by 60 sec washing with a Glycine pH 1.5 solution at a flow rate of 30 µl/min. Bulk refractive index differences were corrected by subtracting the response obtained from a anti His antibody surface. Blank injections are also subtracted (= double referencing). For calculation of K_{D} and other kinetic parameters the Langmuir 1:1 model was used.

### Ang-2 binding was assessed according to the following procedure:

Binding of indicated antibodies to human Ang-2-RBD-Fc was investigated by surface plasmon resonance using a BIACORE® T200 instrument (GE Healthcare). Around 8000 (RU) of goat anti human F(ab')₂ (10 µg/ml anti human F(ab)'₂; Order Code: 28958325; GE Healthcare Bio-Sciences AB, Sweden) were coupled on a Series S CM5 chip (GE Healthcare BR-1005-30) at pH 5.0 by using an amine coupling kit supplied by the GE Healthcare. HBS-N (10 mM HEPES, 150 mM NaCl pH 7.4, GE Healthcare) was used as running buffer during the immobilization procedure. For the following kinetic characterization, sample and running buffer was PBS-T (10 mM phosphate buffered saline including 0.05% Tween20) at pH 7.4. The flow cell was set to 25 °C - and the sample block set to 12 °C - and primed with running buffer twice prior to kinetic characterization.

The bispecific antibody was captured by injecting a 5 nM solution for 25 sec at a flow of 5 µl/min. The association was measured by injection of human Ang2-RBD-Fc in various concentrations in solution for 120 sec at a flow of 30 µl/min starting with 100 nM in 1:3 serial dilutions. The dissociation phase was monitored for up to 180 sec and triggered by switching from the sample solution to running buffer. The surface was regenerated by 60 sec washing with a Glycine pH 2.1 solution at a flow rate of 30 µl/min. Bulk refractive index differences were corrected by subtracting the response obtained from a goat anti human F(ab')₂ surface. Blank injections are also subtracted (= double referencing). For calculation of apparent K_{D} the Langmuir 1:1 model was used.

### IL-17 binding was assessed according to the following procedure:

Around 6000 resonance units (RU) of the capturing system (15 µg/ml goat anti human F(ab')2; Order Code: 28958325; GE Healthcare Bio-Sciences AB, Schweden) were coupled on a CM5 chip at pH 5.0 by using an amine coupling kit supplied by the GE Healthcare. The sample and system buffer was PBS-T (10 mM phosphate buffered saline including 0.05% Tween 20) pH 7.4. The bispecific antibody was captured by injecting a 50 nM solution for 90 sec at a flow of 10 µl/min. Association was measured by injection human IL17 in various concentrations in solution for 3 min at a flow of 30 µl/min starting with 50 nM in 1:1 serial dilutions. The dissociation phase was monitored for up to 10 min and triggered by switching from the sample solution to running buffer. The surface was regenerated twice by 60 sec wash with a 10 mM glycine pH 2.1 solution at a flow rate of 30 µl/min. Bulk refractive index differences were corrected by subtracting the response obtained from a goat anti human F(ab')2 surface. Blank injections are also subtracted (= double referencing). For calculation of apparent K_{D} and other kinetic parameters, the Langmuir 1:1 model was used.

### TWEAK binding was assessed according to the following procedure:

Due to strong unspecific binding of the TWEAK analyte to the sensor surface, a reverse setup - using TWEAK as ligand - was chosen. Around 100 RU of TWEAK was immobilized on the C1 chip surface at pH 5.0 using an amine coupling kit supplied by the GE Healthcare. The sample and system buffer was PBS-T (10 mM phosphate buffered saline including 0.05% Tween 20) pH 7.4. Association was measured by injection the bispecific antibody in various concentrations in solution for 3 min at a flow of 30 µl/min starting with 50 nM in 1:1 serial dilutions. The dissociation phase was monitored for up to 10 min and triggered by switching from the sample solution to running buffer. The surface was regenerated three times by 30 sec wash with a 3 M MgCl2 solution at a flow rate of 30 µl/min. Bulk refractive index differences were corrected by subtracting the response obtained from a blank-coupled surface. Blank injections are also subtracted (= double referencing). For calculation of K_{D} and other kinetic parameters, the Langmuir 1:1 model was used.

### Example 1A

### Production and expression of bivalent, bispecific antibodies which bind to Angiopoietin-2 (ANG2) and Vascular endothelial growth factor (VEGF), with CL-CH1 domain exchange (CrossMAb^{CH1/CL}) in one binding arm and with one or two charged amino acid substitutions in the CH1/CL interface

In a first example bispecific antibodies which bind to human Angiopoietin-2 (ANG2) and human Vascular endothelial growth factor (VEGF) were generated as described in the general methods section by classical molecular biology techniques and expressed transiently in HEK293 cells as described above.

A general scheme of these respective bispecific antibodies is given in Figure 1. For comparative analyses the wild type (wt) CL-CH1 domain exchange antibodies without charged amino acid substitutions in the CH1/CL interface were prepared. The bispecific antibodies were expressed using expression plasmids containing the nucleic acids encoding the amino acid sequences as shown in **Table 1**.

**Table 1: Amino acid sequences of Ang2-VEGF CrossMAb^{CH1-CL} with CH1-CL domain exchanges on the VEGF Fab arm: wild type (wt) and different combinations of substitutions with charged amino acids**

| Bispecific antibody | Ang2_LC | Ang2_HC | VEGF_LC* | VEGF_HC* |
|---|---|---|---|---|
| | LC 1 | HC1 | xLC2 (short chain) | xHC2 (long chain) |
| Ang2VEGF-0454 | SEQ ID NO: 1 E123(Wt)/ Q124(Wt) | SEQ ID NO: 2 K147(Wt)/ K213(Wt) | SEQ ID NO: 3 K147(Wt)/ K213(Wt) | SEQ ID NO: 4 E123(Wt)/ Q124(Wt) |
| CrossMAb^{CH1-CL} with one additional charge pair in the CH1-CL interface | | | | |
| Ang2VEGF-0455 | SEQ ID NO: 5 Q124K | SEQ ID NO: 6 K147E | SEQ ID NO: 3 Wt | SEQ ID NO: 4 Wt |
| Ang2VEGF-0456 | SEQ ID NO: 5 Q124K | SEQ ID NO: 7 K213E | SEQ ID NO: 3 Wt | SEQ ID NO: 4 Wt |
| Ang2VEGF-0457 | SEQ ID NO: 8 E123K | SEQ ID NO: 7 K213E | SEQ ID NO: 3 Wt | SEQ ID NO: 4 Wt |
| Ang2VEGF-0458 | SEQ ID NO: 8 E123K | SEQ ID NO: 6 K147E | SEQ ID NO: 3 Wt | SEQ ID NO: 4 Wt |
| Ang2VEGF-0459 | SEQ ID NO: 1 Wt | SEQ ID NO: 2 Wt | SEQ ID NO: 9 K147E | SEQ ID NO: 10 Q124K |
| Ang2VEGF-0460 | SEQ ID NO: 1 Wt | SEQ ID NO: 2 Wt | SEQ ID NO: 11 K213E | SEQ ID NO: 10 Q124K |
| CrossMAbs^{CH1-CL} with two additional pair of charges in the CH1-CL interface (and optionally one additional charge (Q124E) in the CL) | | | | |
| Ang2VEGF-0461 | SEQ ID NO: 12 E123K/Q124R | SEQ ID NO: 13 K147E/ K213E | SEQ ID NO: 3 Wt | SEQ ID NO: 4 Wt |
| Ang2VEGF-0462 | SEQ ID NO: 12 E123K/Q124R | SEQ ID NO: 14 K147E/ K213D | SEQ ID NO: 3 Wt | SEQ ID NO: 4 Wt |
| Ang2VEGF-0463 | SEQ ID NO: 12 E123K/Q124R | SEQ ID NO: 14 K147E/ K213D | SEQ ID NO: 3 Wt | SEQ ID NO: 23 Q124E |
| CrossMAbs^{CH1-CL} with two additional pairs of charges in the CH1-CL interface and one additional charge pair in the VH-VL interfaces (and optionally one additional charge (Q124E) in the CL) | | | | |
| Ang2VEGF-0464 | SEQ ID NO: 15 Q38E/E123K/ Q124K | SEQ ID NO: 16 Q39K/K147E/ K213E | SEQ ID NO: 17 Q38K | SEQ ID NO: 18 Q39E |
| Ang2VEGF-0465 | SEQ ID NO: 19 Q38K/E123K/ Q124K | SEQ ID NO: 20 Q39E/K147E/ K213E | SEQ ID NO: 17 Q38K | SEQ ID NO: 18 Q39E |
| Ang2VEGF-0466 | SEQ ID NO: 19 Q38K/E123K/ Q124K | SEQ ID NO: 20 Q39E/K147E/ K213E | SEQ ID NO: 17 Q38K | SEQ ID NO: 21 Q39E/Q124E |
| CrossMAb^{CH1-CL} with one additional charge pair in the CH1-CL interface and one additional charge (Q124E) in the CL (or only two additional charges (Q124E) in the CLs) | | | | |
| Ang2VEGF-0474 | SEQ ID NO: 5 Q124K | SEQ ID NO: 7 K213E | SEQ ID NO: 3 wt | SEQ ID NO: 23 Q124E |
| Ang2VEGF-0475 | SEQ ID NO: 22 Q124E | SEQ ID NO: 2 wt | SEQ ID NO: 3 wt | SEQ ID NO: 23 Q124E |
| Ang2VEGF-0476 | SEQ ID NO: 22 Q124E | SEQ ID NO: 2 wt | SEQ ID NO: 11 K213E | SEQ ID NO: 10 Q124K |
| Ang2VEGF-0477 | SEQ ID NO: 5 Q124K | SEQ ID NO: 6 K147E | SEQ ID NO: 3 wt | SEQ ID NO: 23 Q124E |

For all constructs knob-into-hole heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349'C) (contained in the respective corresponding heavy chain (HC) sequences depicted above).

### Example 1B

### Protein A Purification of bivalent, bispecific antibodies which bind to ANG2 and VEGF, with CH1-CL domain exchange (CrossMAb^{CH1-CL}) in one binding arm and charge pair substitutions in the Fab arm and/or one additional charged amino acid substitutions in the CH1/CL interface.

The bispecific antibodies expressed above in example 1A were purified from the supernatant by a combination of Protein A affinity chromatography and size exclusion chromatography. All bispecific antibodies can be produced in good yields and are stable. The obtained products were characterized for identity by mass spectrometry and analytical properties such as purity by CE-SDS, monomer content and stability.

The expected primary structures were analyzed by electrospray ionization mass spectrometry (ESI-MS) of the deglycosylated intact CrossMAbs and deglycosylated/plasmin digested or alternatively deglycosylated/limited LysC digested CrossMAbs as described in the general methods section.

Results are shown in Table 2 for the purity after protein A chromatography and in Table 2b after Protein A and SEC chromatography. The aggregation onset temperature is given in Table 3, 4, 5 and 6.

**Table 2: Purity of different multispecific antibodies with charged amino acid substitutions in the CH1/CL interface after Protein A Purification**

| | Protein A Purification (Results) | | |
|---|---|---|---|
| THERAPS | Yield [mg] calculated for 1L supernatant | analytical SEC monomer[%] | CE-SDS monomer [%] |
| Ang2VEGF-0454-0002 (WT) | 67.5 | 82.36 | 83.59 |
| CrossMAb^{CH1-CL} with one additional charge pair in the CH1-CL interface | | | |
| Ang2VEGF-0455-0002 | 2.12 | 71.79 | 89.87 |
| Ang2VEGF-0456-0002 | 35.2 | 85.27 | 89.09 |
| Ang2VEGF-0457-0002 | 71.5 | 87.69 | 95.26 |
| Ang2VEGF-0458-0002 | 75 | 82.91 | 80.95 |
| Ang2VEGF-0459-0002 | 15.3 | 71.00 | 86.47 |
| Ang2VEGF-0460-0002 | 9.6 | 66.41 | 34.77 |
| CrossMAbs^{CH1-CL} with two additional pair of charges in the CH1-CL interface (and optionally one additional charge (Q124E) in the CL) | | | |
| Ang2VEGF-0461-0002 | 30.4 | 85.29 | 81.8 |
| Ang2VEGF-0462-0002 | 22.8 | 92.07 | 96.07 |
| Ang2VEGF-0463-0002 | 13.5 | 98.83 | 100 |
| CrossMAbs^{CH1-CL} with two additional pairs of charges in the CH1-CL interface and one additional charge pair in the VH-VL interfaces (and optionally one additional charge (Q124E) in the CL) | | | |
| Ang2VEGF-0464-0002 | 51.50 | 73.58 | 66.26 |
| Ang2VEGF-0465-0002 | 10.90 | 87.07 | 88.77 |
| Ang2VEGF-0466-0002 | 20.20 | 91.91 | 95.99 |
| CrossMAb^{CH1-CL} with one additional charge pair in the CH1-CL interface and one additional charge (Q124E) in the CL (or only two additional charges (Q124E) in the CLs) | | | |
| Ang2VEGF-0474 | 11.44 | 97.45 | n.a. |
| Ang2VEGF-0475 | 16.72 | 57.38 | 43.37 |
| Ang2VEGF-0476 | 23.32 | 83.49 | 85.46 |
| Ang2VEGF-0477 | 30.72 | 97.51 | >99 |

All CrossMAb^{CH1-CL} can be expressed and purified. The purity of the obtained material after Protein A chromatography is only a first rough estimation of the obtainable purities. Therefore an additional purification step by preparative SEC was used to better characterize how easy the bispecific antibodies could purified to obtain optimally a maximum of the monomeric form. Additional analytical methods to also better characterize the properties of the obtained bispecific antibodies (e.g. thermal stability (aggregation onset temperature), mass spectrometry and functional assessment) were only applied after protein A and SEC purification.

### Example 2

### SEC purification, analytical characterization and thermal stability of CrossMAbs^{CH1-CL} with one additional charge pair CrossMAbs^{CH1-CL} with one additional pair of charges in the CH1/CL interface.

The bispecific antibodies Ang2VEGF-0454 (control), -0455, -0456, -0457, - 0458, -0459 and -0460 were further purified by size exclusion chromatography. All bispecific antibodies can be produced in good yields and are stable. The obtained products were characterized for identity by mass spectrometry and analytical properties such as purity by CE-SDS, monomer content and thermal stability.

The expected primary structures were analyzed by electrospray ionization mass spectrometry (ESI-MS) of the deglycosylated intact CrossMAbs and deglycosylated/plasmin digested or alternatively deglycosylated/limited LysC digested CrossMAbs as described in the general methods section.

**Table 3: Purity and aggregation onset temperature of CrossMAbs^{CH1-CL} with one additional pair of charges. Increase of Aggregation temperature by charged amino acids substitutions in the CH1/CL interface.**

| | Protein A and preparative SEC Purification (Results) | | T_agg/°C | Identity by ESI-MS |
|---|---|---|---|---|
| Bispecific antibody | analytical SEC monomer [%] | CE-SDS monomer [%] | | |
| Ang2VEGF-0454 (WT) | 97.01 | 90.07 | 52.8 | Confirmed 92% CrossMab 3% HC Heterodimer 5% LC Heterodimer |
| CrossMAb^{CH1-CL} with one additional charge pair in the CH1-CL interface | | | | |
| Ang2VEGF-0455 | 97.31 | 98.1 | 58.5 | Confirmed 100% CrossMab |
| Ang2VEGF-0456 | >99 | 97.03 | 55.3 | Confirmed 98% CrossMab 2% HC Heterodimer |
| Ang2VEGF-0457 | >99 | 98.44 | 56.1 | Confirmed 99% CrossMab 1% HC Heterodimer |
| Ang2VEGF-0458 | 96.38 | 86.78 | 52.9 | Confirmed 97% CrossMab 3% HC Heterodimer |
| Ang2VEGF-0459 | 98.53 | 97.62 | 54.5 | Confirmed 100% CrossMab |
| Ang2VEGF-0460 | 83.08 | 76.52 | 49.5 | Confirmed 90% CrossMab 10% unknown Masses (102-103 kDa) |

The introduction of one additional pair of charged residues in the CrossMAb^{CH1-CL} shows:
- the purity with respect to analytical SEC of the obtained CrossMAbs increases dependent on the selected charge pair
- the purity with respect to CE-SDS of the obtained CrossMAbs increases dependent on the selected charge pair
- the thermal stability of the obtained CrossMAb increases dependent on the selected charge pair
- the thermal stability of the obtained CrossMAb increases dependent on the position of the selected charge pair (crossed versus non crossed Fab arm)

Based on these results one can conclude that the charge pair Q124K in the light chain and K147E in the heavy chain is the preferred charge pair. Additional charges should be introduced in the wt Fab arm (non crossed).

### Example 3

### SEC purification, analytical characterization and thermal stability of CrossMAbCH1-CL with two additional pairs of charges on the non crossed Fab arm.

The bispecific antibodies Ang2VEGF-0454 (control), -0461, -0462, and - 0463 were further purified by size exclusion chromatography. All bispecific antibodies can be produced in good yields and are stable. The obtained products were characterized for identity by mass spectrometry and analytical properties such as purity by CE-SDS, monomer content and thermal stability.

The expected primary structures were analyzed by electrospray ionization mass spectrometry (ESI-MS) of the deglycosylated intact CrossMAbs and deglycosylated/plasmin digested or alternatively deglycosylated/limited LysC digested CrossMAbs as described in the general methods section.

**Table 4: Purity and aggregation onset temperature of CrossMAbs^{CH1-CL} with two additional pairs of charges after Protein A and SEC chromatorgraphy. Increase of aggregation onset temperature by charged amino acids substitutions in the CH1/CL interface.**

| | Protein A and preparative SEC Purification (Results) | | T_agg/°C | Identity by ESI-MS |
|---|---|---|---|---|
| Bispecific antibody | analytical SEC monomer [%] | CE-SDS monomer [%] | | |
| Ang2VEGF-0454 (Ang2VEGF^{CH1- CL}WT control) | 97.01 | 90.,07 | 52.8 | Confirmed 92% CrossMab 3% HC Heterodimer 5% LC Heterodimer |
| CrossMAbs^{CH1-CL} with two additional pair of charges in the CH1-CL interface (and optionally one additional charge (Q124E) in the CL) | | | | |
| Ang2VEGF-0461 | >99 | 92.26 | 57.1 | Confirmed 99% CrossMab 1% ½CrossMab |
| Ang2VEGF-0462 | >99 | 98.33 | 56.4 | Confirmed 100% CrossMab |
| Ang2VEGF-0463 | >99 | 100 | 56.4 | Confirmed 100% CrossMab |

The introduction of two additional pairs of charged residues in the CrossMAb^{CH1-CL} shows:
- a clear overall increase of the aggregation temperature and thermal stability
- the purity with respect to analytical SEC of the obtained CrossMAbs does not increase any more after preparative SEC dependent on the selected charge pair, but clearly increased after Protein A chromatography (see Table 2)
- K213D seems to be slightly superior to K213E in CH1 of the uncrossed Fab arm with respect to CE-SDS monomer content.
- the purity with respect to CE-SDS of the obtained CrossMAbs increases dependent on the selected charge pair
- the purity with respect to CE-SDS of the obtained CrossMAbs clearly increases by the addition of a single charged amino acid in position Q124E in the crossed Fab arm
- The additional single charged amino acid Q124E in CL is beneficial for the overall purity (CE-SDS and SEC) and does not lead to an decrease in thermal stability.

Based on these results one can conclude that the charge pair Q124K/E123R in the light chain and K147E/K213E/D in the heavy chain is the preferred charge pair for the non crossed Fab arm. Addition of one single charged residue Q124E in CL in the crossed Fab arm slightly improves in addition the purity of the CrossMAb CH1-CL further (CE-SDS) and has no negative impact on the thermal stability.

### Example 4

### SEC purification, analytical characterization and thermal stability of CrossMAbCH1-CL with two additional charge pairs on the non crossed Fab arm and additional charges in the VH-VL interface.

The bispecific antibodies Ang2VEGF-0454 (control), -0464, -0465, and - 0466 were further purified by size exclusion chromatography. All bispecific antibodies can be produced in good yields and are stable. The obtained products were characterized for identity by mass spectrometry and analytical properties such as purity by CE-SDS, monomer content and thermal stability.

The expected primary structures were analyzed by electrospray ionization mass spectrometry (ESI-MS) of the deglycosylated intact CrossMAbs and deglycosylated/plasmin digested or alternatively deglycosylated/limited LysC digested CrossMAbs as described in the general methods section.

**Table 5: Purity and aggregation onset temperature of CrossMAbsCH1-CL with two additional pairs of charges and one additional charge pair in the VH-VL interface after Protein A and SEC chromatorgraphy..**

| | Protein A and SEC Purification (Results) | | T_agg/°C | Identity by ESI-MS |
|---|---|---|---|---|
| Bispecific antibody | analytical SEC monomer [%] | CE-SDS monomer [%] | | |
| Ang2VEGF-0454 (Ang2VEGF^{CH1- CL}WT control) | 97.01 | 90.,07 | 52.8 | Confirmed 92% CrossMab 3% HC Heterodimer 5% LC Heterodimer |
| CrossMAbs^{CH1-CL} with two additional pairs of charges in the CH1-CL interface and one additional charge pair in the VH-VL interfaces (and optionally one additional charge (Q124E) in the CL) | | | | |
| Ang2VEGF-0464 | 96.94 | 97.37 | 56.4 | Confirmed 100% CrossMab |
| Ang2VEGF-0465 | 98.03 | 98.22 | 54.8 | Confirmed 100% CrossMab |
| Ang2VEGF-0466 | 98.,61 | 98.94 | 55.6 | Confirmed 100% CrossMab |

The introduction of two additional pairs of charged residues in the CrossMAb^{CH1-CL} and one additional charge pair in Vh(Q38)-Vl(Q39) shows:
- the purity with respect to analytical SEC of the obtained CrossMAbs does not increase any more (compared to the CrossMAb^{CH1-CL}) after preparative SEC dependent on the selected charge pair, but clearly increased after Protein A chromatography (see Table 2)
- the purity with respect to CE-SDS of the obtained CrossMAbs increases dependent on the selected charge pairs
- The additional charged amino acids Q38E in CL and Q39K in Vh is beneficial for the overall purity (CE-SDS and SEC) and does not lead to an decrease in thermal stability. The orientation Q38E in VL and Q39K in Vh is preferred.

Based on these results one can conclude that the charge pair Q38E in VL and Q39K in Vh in the variable domains is the preferred charge pair for the non crossed Fab arm and Q38K in VL and Q39E in VH is the preferred charge pair for the crossed Fab arm. Addition of one single charged residue Q124E in CL in the crossed Fab arm improves the purity of the CrossMAb CH1-CL further (CE-SDS) and has no negative impact on the thermal stability.

### Example 5

### SEC purification, analytical characterization and thermal stability of CrossMAb^{CH1-CL} with one additional charge pair on the non crossed Fab arm or on the crossed Fab arm and one additional charge (Q124E) in the CL interface of the Fab arm without a charge pair modification.

The bispecific antibodies Ang2VEGF-0454 (control), -0464, -0465, and - 0466 were further purified by size exclusion chromatography. All bispecific antibodies can be produced in good yields and are stable. The obtained products were characterized for identity by mass spectrometry and analytical properties such as purity by CE-SDS, monomer content and thermal stability.

The expected primary structures were analyzed by electrospray ionization mass spectrometry (ESI-MS) of the deglycosylated intact CrossMAbs and deglycosylated/plasmin digested or alternatively deglycosylated/limited LysC digested CrossMAbs as described in the general methods section.

**Table 6: Purity and aggregation temperature of CrossMAbs^{CH1-CL} with one additional pair of charges and Q124E in CL of the other Fab arm after Protein A and SEC chromatorgraphy. The influence of aggregation onset temperatures by charged amino acids substitutions in the CH1/CL interface is depicted.**

| | Protein A and SEC Purification (Results) | | T_agg/°C | Identity by ESI-MS |
|---|---|---|---|---|
| Bispecific antibody | analytical SEC monomer [%] | CE-SDS monomer [%] | | |
| Ang2VEGF-0454 (Ang2VEGF^{CH1- CL}WT control) | 97.01 | 90.07 | 52.8 | confirmed |
| CrossMAb^{CH1-CL} with one additional charge pair in the CH1-CL interface and one additional charge (Q124E) in the CL (or only two additional charges (Q124E) in the CLs) | | | | |
| Ang2VEGF-0474 | >99 | 98,08 | 55.1 | Confirmed 97% CrossMab 1% knob HC Dimer 2% VEGF AK |
| Ang2VEGF-0475 | 89.87 | 83.72 | n.a. | Confirmed 69% Correct CrossMab 5% Ang2 AK 2% ¾ Ang2 AK 11% CrossMab with 2x LC Ang2 13% hole Heterodimer with 1 LC Ang2 and 1 LC VEGF |
| Ang2VEGF-0476 | >99 | 93.14 | 50.7 | Confirmed 87% CrossMab 7% HC Heterodimer 6% LC Heterodimer |
| Ang2VEGF-0477 | >99 | >99 | 56.6 | Confirmed 100% CrossMab |

Based on these results in Example 2 to 5 one can conclude that the charge pair Q124K (CL) and K147E (CH1) in the non crossed Fab arm and the single charge substitution Q124E in CL in the crossed Fab arm is the preferred charge pair modification of the CrossMAb^{CH1-CL}. Overall these four charge pair substitutions (CL: Q124K in combination with CH1: K147E or K213E/D) in combination with the single charge residue substitution Q124E in CL of the second Fab arm are most preferable charge substitutions for the CrossMAb^{CH1-CL} format. The charge pair substitutions were preferable in the non-crossed Fab arm, whereas the single charge substitution (Q124E in CL) is preferably placed in the crossed Fab arm. These 4 examples and substitutions all clearly show an beneficial increase with respect to purity and thermal stability compared to the parental CrossMAb^{CH1-CL} without charged amino acid substitutions.

### Example 6

### Antigen binding

Binding of the multispecific antibodies to their respective target antigens, i.e. ANG2 and VEGF, was assessed by Biacore^{®} as described in the general methods section.

As comparative example, a reference antibody specifically binding to Ang2 and VEGF comprising a CH1/CL domain exchange/replacement but lacking charged amino acid substitutions (Ang2VEGF-0454 antibody) was assessed in parallel.

Affinity measurements were conducted with the purified material from the protein A and SEC purification and the results are summarized in **Table 7**.

**Table 7: Affinity for VEGF of indicated antibodies and affinity for Ang2 of indicated antibodies**

| Bispecific antibody | VEGF 121 | | | Ang2 RBD | | |
|---|---|---|---|---|---|---|
| | ka (1/Ms) | kd (1/s) | KD* (nM) | ka (1/Ms) | kd (1/s) | App.KD* (nM) |
| Ang2VEGF-0454-0002 | 2.04E+04 | 8.65E-05 | 4 | 1.08E+07 | 1.82E-02 | 2 |
| Ang2VEGF-0455-0002 | 1.26E+04 | 9.01E-05 | 7 | 1.06E+07 | 2.27E-02 | 2 |
| Ang2VEGF-0456-0002 | 2.10E+04 | 8.40E-05 | 4 | 1.16E+07 | 2.52E-02 | 2 |
| Ang2VEGF-0459-0002 | 1.70E+04 | 7.19E-05 | 4 | 8.80E+06 | 1.99E-02 | 2 |
| Ang2VEGF-0460-0002 | 1.42E+04 | 8.18E-05 | 6 | 8.76E+06 | 1.63E-02 | 2 |
| Ang2VEGF-0461-0002 | 1.92E+04 | 7.67E-05 | 4 | 1.07E+07 | 1.63E-02 | 2 |
| Ang2VEGF-0462-0002 | 2.12E+04 | 7.52E-05 | 4 | 1.21E+07 | 2.52E-02 | 2 |
| Ang2VEGF-0463-0002 | 1.76E+04 | 7.44E-05 | 4 | 9.54E+06 | 2.81E-02 | 3 |
| Ang2VEGF-0464 | 1.66E+04 | 7.54E-05 | 5 | 8.81E+06 | 1.72E-02 | 2 |
| Ang2VEGF-0465-0002 | 1.60E+04 | 7.96E-05 | 5 | 1.17E+07 | 3.25E-02 | 3 |
| Ang2VEGF-0466-0002 | 1.86E+04 | 8.01E-05 | 4 | 1.06E+07 | 3.10E-02 | 3 |
| Ang2VEGF-0474 | 2.13E+04 | 5.41E-05 | 3 | 2.04E+06 | 9.70E-03 | 5 |
| Ang2VEGF-0475 | 1.05E+04 | 7.26E-05 | 7 | 1.38E+06 | 5.05E-03 | 4 |
| Ang2VEGF-0476 | 1.85E+04 | 6.94E-05 | 4 | 1.32E+06 | 1.14E-02 | 9 |
| Ang2VEGF-0477 | 1.95E+04 | 6.11E-05 | 3 | 2.19E+06 | 8.23E-03 | 4 |

The tested antibodies specifically bind to both targets, Ang2 and VEGF, and exhibit an antigen affinity in the nanomolar range. All used charged amino acid substitutions deliver CrossMAbs^{CH1-CL} capable of binding to Ang2 and VEGF with similar affinities to each target and do not show any influence on the target binding in comparison to the Ang2VEGF-0454 CrossMAb (without charged amino acid substitutions).

### Example 7

### Production and expression of bivalent, bispecific antibodies which bind to TWEAK and IL-17, with CL-CH1 domain exchange (CrossMAb^{CH1/CL}) in one binding arm and with one or two charged amino acid substitutions in the CH1/CL interface

In a further example bispecific antibodies which bind to human TWEAK and human IL-17 are generated as described in the general methods section by classical molecular biology techniques and expressed transiently in HEK293 cells as described above.

A general scheme of these respective bispecific antibodies is given in Figure 1. For comparative analyses the wild type (wt) CL-CH1 domain exchange antibodies without charged amino acid substitutions in the CH1/CL interface are prepared. The bispecific antibodies are expressed using expression plasmids containing the nucleic acids encoding the amino acid sequences as shown in **Table 8**.

**Table 8: Amino acid sequences of TWEAK-IL17 CrossMAb^{CH1-CL} with CH1-CL domain exchanges on the TWEAK Fab arm: wild type (wt) and different combinations of substitutions with charged amino acids**

| **Bispecific antibody** | **IL17_LC** | **IL17_HC** | **Tweak_LC*** | **Tweak_HC*** |
|---|---|---|---|---|
| | LC 1 | HC1 | xLC2 (short chain) | xHC2 (long chain) |
| TweakIL17 -0049 | SEQ ID NO: 28 E123(Wt)/ Q124(Wt) | SEQ ID NO: 29 K147(Wt)/ K213(Wt) | SEQ ID NO: 30 K147(Wt)/ K213(Wt) | SEQ ID NO: 31 E123(Wt)/ Q124(Wt) |
| TweakIL17 -0129 | SEQ ID NO: 32 Q124K | SEQ ID NO: 33 K147E | SEQ ID NO: 30 Wt | SEQ ID NO: 31 Wt |
| TweakIL17 -0130 | SEQ ID NO: 35 E123K | SEQ ID NO: 34 K213E | SEQ ID NO: 30 Wt | SEQ ID NO: 31 Wt |
| TweakIL17 -0131 | SEQ ID NO: 35 E123K | SEQ ID NO: 34 K147E | SEQ ID NO: 30 Wt | SEQ ID NO: 39 Q124E |
| TweakIL17 -0132 | SEQ ID NO: 36 E123R/Q124K | SEQ ID NO: 37 K147E/K213E | SEQ ID NO: 30 Wt | SEQ ID NO: 31 Wt |
| TweakIL17 -0133 | SEQ ID NO: 36 E123R/Q124K | SEQ ID NO: 38 K147E/K213D | SEQ ID NO: 30 Wt | SEQ ID NO: 39 Q124E |
| TweakIL17 -0134 | SEQ ID NO: 32 Q124K | SEQ ID NO: 33 K147E | SEQ ID NO: 30 wt | SEQ ID NO: 39 Q124E |

For all constructs knob-into-hole heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349'C) (contained in the respective corresponding heavy chain (HC) sequences depicted above).

These antibodies can be characterizes as described above for their characteristic properties using the respective methods and antigens. (see e.g. methods and Example 1B to 6).

## Claims

1. A multispecific antibody, comprising
a) a first light chain and a first heavy chain derived from a first antibody which specifically binds to a first antigen; and
b) a second light chain and a second heavy chain derived from a second antibody which specifically binds to a second antigen, wherein
in the second light chain the constant domain CL is replaced by the constant domain CH1 of the second heavy chain; and
in the second heavy chain the constant domain CH1 is replaced by the constant domain CL of the second light chain; and
i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D; or
ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D.

2. The multispecific antibody according to claim 1, wherein
i) wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D, and
in the constant domain CL of the second heavy chain the amino acid at position 124 (numbering according to Kabat) is substituted by an amino acid selected from E and D; or
ii) wherein in the constant domain CL of the second heavy chain the amino acids at position 124 and 123 (numbering according to Kabat) are substituted independently from each other by an amino acid selected from K, R and H; and wherein in the constant domain CH1 of the second light chain the amino acids at position 147 and 213 (numbering according to EU index of Kabat) are substituted independently from each other by an amino acid selected from E or D, and
in the constant domain CL of the first light chain the amino acid at position 124 (numbering according to Kabat) is substituted by an amino acid selected from E and D.

3. The multispecific antibody according to any one of the preceding claims, wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D.

4. The multispecific antibody according to any one of the preceding claims, wherein in the constant domain CL of the first light chain the amino acids at position 124 and 123 (numbering according to Kabat) are substituted independently from each other by an amino acid selected from K, R and H; and wherein in the constant domain CH1 of the first heavy chain the amino acids at position 147 and 213 (numbering according to EU index of Kabat) are substituted independently from each other by an amino acid selected from E or D, and
in the constant domain CL of the second heavy chain the amino acid at position 124 (numbering according to Kabat) is substituted by an amino acid selected from E and D.

5. The multispecific antibody according to any one of the preceding claims,
i) wherein in the variable domain VL of the first light chain the amino acid at position 38 (numbering according to Kabat) is substituted by an amino acid selected from K, R and H; and wherein in the variable domain VH of the first heavy chain the amino acid at position 39 (numbering according to Kabat) is substituted by an amino acid selected from E and D; and
ii) wherein in the variable domain VL of the second heavy chain the amino acid at position 38 (numbering according to Kabat) is substituted by an amino acid selected from E and D (in one preferred embodiment E); and wherein in the variable domain VH of the second light chain the amino acid at position 39 (numbering according to Kabat) is substituted by an amino acid selected from K, R and H (in one preferred embodiment by K or R, in one embodiment by K).

6. The multispecific antibody according to any one of the preceding claims,
wherein
in the tertiary structure of the antibody the CH3 domain of the first heavy chain and the CH3 domain of the second heavy chain form an interface that is located between the respective antibody CH3 domains,
wherein the respective amino acid sequences of the CH3 domain of the first heavy chain and the CH3 domain of the second heavy chain each comprise a set of amino acids that is located within said interface in the tertiary structure of the antibody,
wherein from the set of amino acids that is located in the interface in the CH3 domain of one heavy chain at least one amino acid residue is substituted by an amino acid residue having a larger side chain volume than the original amino acid residue,
thereby generating a protuberance within the interface, wherein the protuberance is located in the CH3 domain of the one heavy chain, and wherein the protuberance is positionable in a cavity located in the CH3 domain of the other heavy chain within the interface; and
wherein from the set of amino acids that is located in the interface in the CH3 domain of the other heavy chain at least one amino acid residue is substituted by an amino acid residue having a smaller side chain volume than the original amino acid residue,
thereby generating a cavity within the interface, wherein the cavity is located in the CH3 domain of the other heavy chain, and wherein in the cavity the protuberance within the interface located in the CH3 domain of the one heavy chain is positionable.

7. The multispecific antibody according to claim 6, wherein
said amino acid residue having a larger side chain volume than the original amino acid residue is selected from R, F, Y and W; and
wherein said amino acid residue having a smaller side chain volume than the original amino acid residue is selected from A, S, T and V.

8. The multispecific antibody according to claim 6 or 7, wherein
from the set of amino acids that is located in the interface in the CH3 domain of the one heavy chain a first amino acid is substituted by cysteine; and
from the set of amino acids that is located in the interface in the CH3 domain of the other heavy chain a second amino acid is substituted by cysteine, wherein the second amino acid is facing the first amino acid within the interface;
such that a disulfide bridge between the CH3 domain of the one heavy chain and the CH3 domain of the other heavy chain can be formed via the introduced cysteine residues.

9. The multispecific antibody according to any one of the preceding claims that specifically binds to human Angiopoietin-2 and human VEGF, wherein
a) the antibody comprises a variable heavy chain domain (VH) according to SEQ ID NO: 26 (<VH Ang2>) and a variable light chain domain (VL) according to SEQ ID NO: 27 (<VL Ang2>); and
b) the antibody comprises a variable heavy chain domain (VH) according to SEQ ID NO: 24 (<VH VEGF>) and a variable light chain domain (VL) according to SEQ ID NO: 25 (<VL VEGF>).

10. A method for the preparation of a multispecific antibody according to any one of claims 1 to 9, comprising the steps of
- transforming a host cell with vectors comprising nucleic acids encoding
a) the first light chain as defined in one of claims 1 to 9 derived from a first antibody which specifically binds to a first antigen;
b) the first heavy chain as defined in one of claims 1 to 9 derived from a first antibody which specifically binds to a first antigen;
c) the second light chain as defined in one of claims 1 to 9 derived from a second antibody which specifically binds to a second antigen; and
d) the second heavy chain as defined in one of claims 1 to 9 derived from a second antibody which specifically binds to a second antigen,
- culturing said host cell under conditions that allow synthesis of said multispecific antibody; and
- recovering said multispecific antibody from said host cell culture.

11. A nucleic acid encoding the amino acid sequence of
a) a first light chain as defined in one of claims 1 to 9,
b) a second light chain as defined in one of claims 1 to 9,
c) a first heavy chain as defined in one of claims 1 to 9; or
d) a second heavy chain as defined in one of claims 1 to 9.

12. A vector comprising a nucleic acid according to claim 11, wherein the vector is capable of expressing said nucleic acid in a host cell.

13. A host cell comprising
a) a vector comprising nucleic acids encoding the first light chain as defined in one of claims 1 to 9 derived from a first antibody which specifically binds to a first antigen;
b) a vector comprising nucleic acids encoding the first heavy chain as defined in one of claims 1 to 9 derived from a first antibody which specifically binds to a first antigen;
c) a vector comprising nucleic acids encoding the second light chain as defined in one of claims 1 to 9 derived from a second antibody which specifically binds to a second antigen; and
d) a vector comprising nucleic acids encoding the second heavy chain as defined in one of claims 1 to 9 derived from a second antibody which specifically binds to a second antigen.

14. A pharmaceutical composition comprising a multispecific antibody according to any one of claims 1 to 9 in combination with at least one pharmaceutically acceptable carrier.

15. A multispecific antibody according to according to any one of claims 1 to 9 for use as a medicament.


SEQUENCE LISTING

<110>  F. Hoffmann-La Roche AG

<120>  MULTISPECIFIC ANTIBODIES

<130>  33105 EP

<160>  39

<170>  PatentIn version 3.5

<210>  1
<211>  215
<212>  PRT
<213>  Artificial

<220>
<223>  7571 - kappa light chain (LC) <Ang-2> wild type (wt)

<400>  1

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu

                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 2
<211> 459
<212> PRT
<213> Artificial

<220>
<223> 7595 - heavy chain (HC) <Ang-2> wild type (wt)

<400> 2

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Lys Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Lys Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe

405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro Gly Lys
    450                 455

<210>  3
<211>  212
<212>  PRT
<213>  Artificial

<220>
<223>  7594 - light chain (LC) <VEGF> with CL-CH1 domain exchange wild
       type (wt)

<400>  3

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys Ser Ser Ala Ser Thr
            100                 105                 110

Lys Gly Pro Ser Val Phe Pro Leu Ala Pro Ser Ser Lys Ser Thr Ser
        115                 120                 125

Gly Gly Thr Ala Ala Leu Gly Cys Leu Val Lys Asp Tyr Phe Pro Glu
    130                 135                 140

Pro Val Thr Val Ser Trp Asn Ser Gly Ala Leu Thr Ser Gly Val His

145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Lys Lys Val Glu
        195                 200                 205

Pro Lys Ser Cys
    210

<210>  4
<211>  457
<212>  PRT
<213>  Artificial

<220>
<223>  7593 - heavy chain (HC) <VEGF> with CL-CH1 domain exchange wild
       type (wt)

<400>  4

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Val Ala Ala
        115                 120                 125

Pro Ser Val Phe Ile Phe Pro Pro Ser Asp Glu Gln Leu Lys Ser Gly

130                 135                 140

Thr Ala Ser Val Val Cys Leu Leu Asn Asn Phe Tyr Pro Arg Glu Ala
145                 150                 155                 160

Lys Val Gln Trp Lys Val Asp Asn Ala Leu Gln Ser Gly Asn Ser Gln
                165                 170                 175

Glu Ser Val Thr Glu Gln Asp Ser Lys Asp Ser Thr Tyr Ser Leu Ser
            180                 185                 190

Ser Thr Leu Thr Leu Ser Lys Ala Asp Tyr Glu Lys His Lys Val Tyr
        195                 200                 205

Ala Cys Glu Val Thr His Gln Gly Leu Ser Ser Pro Val Thr Lys Ser
    210                 215                 220

Phe Asn Arg Gly Glu Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Cys Thr Leu Pro Pro Ser Arg Asp Glu Leu
        355                 360                 365

Thr Lys Asn Gln Val Ser Leu Ser Cys Ala Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Val Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455

<210>  5
<211>  215
<212>  PRT
<213>  Artificial

<220>
<223>  17914 - kappa light chain (LC) <Ang-2> with Q124K substitution

<400>  5

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Glu Lys Leu Lys Ser
        115                 120                 125

Gly Thr Ala Ser Val Val Cys Leu Leu Asn Asn Phe Tyr Pro Arg Glu

130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  6
<211>  459
<212>  PRT
<213>  Artificial

<220>
<223>  17911 - heavy chain (HC) <Ang-2> with K147E substitution

<400>  6

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Glu Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Lys Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe

370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro Gly Lys
    450                 455

<210>  7
<211>  459
<212>  PRT
<213>  Artificial

<220>
<223>  17912 - heavy chain (HC) <Ang-2> with K213E substitution

<400>  7

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Lys Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Glu Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe

370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro Gly Lys
    450                 455

<210>  8
<211>  215
<212>  PRT
<213>  Artificial

<220>
<223>  17913 - kappa light chain (LC) <Ang-2> with E123K substitution

<400>  8

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Lys Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  9
<211>  212
<212>  PRT
<213>  Artificial

<220>
<223>  17932 - light chain (LC) <VEGF> with CL-CH1 domain exchange and
       K147E substitution

<400>  9

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys Ser Ser Ala Ser Thr
            100                 105                 110

Lys Gly Pro Ser Val Phe Pro Leu Ala Pro Ser Ser Lys Ser Thr Ser
        115                 120                 125

Gly Gly Thr Ala Ala Leu Gly Cys Leu Val Glu Asp Tyr Phe Pro Glu
    130                 135                 140

Pro Val Thr Val Ser Trp Asn Ser Gly Ala Leu Thr Ser Gly Val His
145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Lys Lys Val Glu
        195                 200                 205

Pro Lys Ser Cys
    210

<210>  10
<211>  457
<212>  PRT
<213>  Artificial

<220>
<223>  17934 - heavy chain (HC) <VEGF> with CL-CH1 domain exchange and
       Q124K substitution

<400>  10

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Val Ala Ala
        115                 120                 125

Pro Ser Val Phe Ile Phe Pro Pro Ser Asp Glu Lys Leu Lys Ser Gly
    130                 135                 140

Thr Ala Ser Val Val Cys Leu Leu Asn Asn Phe Tyr Pro Arg Glu Ala
145                 150                 155                 160

Lys Val Gln Trp Lys Val Asp Asn Ala Leu Gln Ser Gly Asn Ser Gln
                165                 170                 175

Glu Ser Val Thr Glu Gln Asp Ser Lys Asp Ser Thr Tyr Ser Leu Ser
            180                 185                 190

Ser Thr Leu Thr Leu Ser Lys Ala Asp Tyr Glu Lys His Lys Val Tyr
        195                 200                 205

Ala Cys Glu Val Thr His Gln Gly Leu Ser Ser Pro Val Thr Lys Ser
    210                 215                 220

Phe Asn Arg Gly Glu Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln

340                 345                 350

Pro Arg Glu Pro Gln Val Cys Thr Leu Pro Pro Ser Arg Asp Glu Leu
        355                 360                 365

Thr Lys Asn Gln Val Ser Leu Ser Cys Ala Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Val Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455

<210>  11
<211>  212
<212>  PRT
<213>  Artificial

<220>
<223>  17933 - light chain (LC) <VEGF> with CL-CH1 domain exchange and
       K213E substitution

<400>  11

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp

85                  90                  95

Thr Phe Gly Gln Gly Thr Lys Val Glu Ile Lys Ser Ser Ala Ser Thr
            100                 105                 110

Lys Gly Pro Ser Val Phe Pro Leu Ala Pro Ser Ser Lys Ser Thr Ser
        115                 120                 125

Gly Gly Thr Ala Ala Leu Gly Cys Leu Val Lys Asp Tyr Phe Pro Glu
    130                 135                 140

Pro Val Thr Val Ser Trp Asn Ser Gly Ala Leu Thr Ser Gly Val His
145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Glu Lys Val Glu
        195                 200                 205

Pro Lys Ser Cys
    210

<210>  12
<211>  215
<212>  PRT
<213>  Artificial

<220>
<223>  17301 - kappa light chain (LC) <Ang-2> with E123R and Q124K
       substitutions

<400>  12

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly

65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Lys Arg Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  13
<211>  459
<212>  PRT
<213>  Artificial

<220>
<223>  14116 - heavy chain (HC) <Ang-2> with K147E and K213E
       substitutions

<400>  13

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe

50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Glu Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Glu Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro Gly Lys
    450                 455

<210>  14
<211>  459
<212>  PRT
<213>  Artificial

<220>
<223>  17305 - heavy chain (HC) <Ang-2> with K147E and K213D
       substitutions

<400>  14

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Glu Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Asp Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro Gly Lys
    450                 455

<210>  15
<211>  215
<212>  PRT
<213>  Artificial

<220>
<223>  17953 - kappa light chain (LC) <Ang-2> with Q38E, E123K and Q124[?]
       substitutions

<400>  15

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Glu Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Lys Lys Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  16
<211>  459
<212>  PRT
<213>  Artificial

<220>
<223>  17954 - heavy chain (HC) <Ang-2> with Q39K, K147E and K213E
       substitutions

<400>  16

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Lys Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Glu Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Glu Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro Gly Lys
    450                 455

<210>  17
<211>  212
<212>  PRT
<213>  Artificial

<220>
<223>  17938 - light chain (LC) <VEGF> with CL-CH1 domain exchange and
       Q38K substitution

<400>  17

Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Lys Lys Pro Gly Lys Ala Pro Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

Thr Phe Gly Gln Gly Thr Lys Val Glu Ile Lys Ser Ser Ala Ser Thr
            100                 105                 110

Lys Gly Pro Ser Val Phe Pro Leu Ala Pro Ser Ser Lys Ser Thr Ser
        115                 120                 125

Gly Gly Thr Ala Ala Leu Gly Cys Leu Val Lys Asp Tyr Phe Pro Glu
    130                 135                 140

Pro Val Thr Val Ser Trp Asn Ser Gly Ala Leu Thr Ser Gly Val His
145                 150                 155                 160

Thr Phe Pro Ala Val Leu Gln Ser Ser Gly Leu Tyr Ser Leu Ser Ser
                165                 170                 175

Val Val Thr Val Pro Ser Ser Ser Leu Gly Thr Gln Thr Tyr Ile Cys
            180                 185                 190

Asn Val Asn His Lys Pro Ser Asn Thr Lys Val Asp Lys Lys Val Glu
        195                 200                 205

Pro Lys Ser Cys
    210

<210>  18
<211>  457
<212>  PRT
<213>  Artificial

<220>
<223>  17936 - heavy chain (HC) <VEGF> with CL-CH1 domain exchange and
       Q39E substitution

<400>  18

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Glu Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Val Ala Ala
        115                 120                 125

Pro Ser Val Phe Ile Phe Pro Pro Ser Asp Glu Gln Leu Lys Ser Gly
    130                 135                 140

Thr Ala Ser Val Val Cys Leu Leu Asn Asn Phe Tyr Pro Arg Glu Ala
145                 150                 155                 160

Lys Val Gln Trp Lys Val Asp Asn Ala Leu Gln Ser Gly Asn Ser Gln
                165                 170                 175

Glu Ser Val Thr Glu Gln Asp Ser Lys Asp Ser Thr Tyr Ser Leu Ser
            180                 185                 190

Ser Thr Leu Thr Leu Ser Lys Ala Asp Tyr Glu Lys His Lys Val Tyr
        195                 200                 205

Ala Cys Glu Val Thr His Gln Gly Leu Ser Ser Pro Val Thr Lys Ser
    210                 215                 220

Phe Asn Arg Gly Glu Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val

260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Cys Thr Leu Pro Pro Ser Arg Asp Glu Leu
        355                 360                 365

Thr Lys Asn Gln Val Ser Leu Ser Cys Ala Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Val Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455

<210>  19
<211>  215
<212>  PRT
<213>  Artificial

<220>
<223>  14098 - kappa light chain (LC) <Ang-2> with Q38K, E123K and Q124K
       substitutions

<400>  19

Gln Pro Gly Leu Thr Gln Pro Pro Ser Val Ser Val Ala Pro Gly Gln

1               5                   10                  15

Thr Ala Arg Ile Thr Cys Gly Gly Asn Asn Ile Gly Ser Lys Ser Val
            20                  25                  30

His Trp Tyr Gln Lys Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Lys Lys Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  20
<211>  459
<212>  PRT
<213>  Artificial

<220>
<223>  14097 - heavy chain (HC) <Ang-2> with Q39E, K147E and K213E
       substitutions

<400>  20

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Glu Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser Ala Ser Thr Lys Gly Pro Ser Val Phe Pro Leu Ala Pro Ser Ser
    130                 135                 140

Lys Ser Thr Ser Gly Gly Thr Ala Ala Leu Gly Cys Leu Val Glu Asp
145                 150                 155                 160

Tyr Phe Pro Glu Pro Val Thr Val Ser Trp Asn Ser Gly Ala Leu Thr
                165                 170                 175

Ser Gly Val His Thr Phe Pro Ala Val Leu Gln Ser Ser Gly Leu Tyr
            180                 185                 190

Ser Leu Ser Ser Val Val Thr Val Pro Ser Ser Ser Leu Gly Thr Gln
        195                 200                 205

Thr Tyr Ile Cys Asn Val Asn His Lys Pro Ser Asn Thr Lys Val Asp
    210                 215                 220

Glu Lys Val Glu Pro Lys Ser Cys Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Cys Arg Asp
        355                 360                 365

Glu Leu Thr Lys Asn Gln Val Ser Leu Trp Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro Gly Lys
    450                 455

<210>  21
<211>  457
<212>  PRT
<213>  Artificial

<220>
<223>  17937 - heavy chain (HC) <VEGF> with CL-CH1 domain exchange and

with Q39E and Q124E substitutions

<400> 21

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Glu Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Val Ala Ala
        115                 120                 125

Pro Ser Val Phe Ile Phe Pro Pro Ser Asp Glu Glu Leu Lys Ser Gly
    130                 135                 140

Thr Ala Ser Val Val Cys Leu Leu Asn Asn Phe Tyr Pro Arg Glu Ala
145                 150                 155                 160

Lys Val Gln Trp Lys Val Asp Asn Ala Leu Gln Ser Gly Asn Ser Gln
                165                 170                 175

Glu Ser Val Thr Glu Gln Asp Ser Lys Asp Ser Thr Tyr Ser Leu Ser
            180                 185                 190

Ser Thr Leu Thr Leu Ser Lys Ala Asp Tyr Glu Lys His Lys Val Tyr
        195                 200                 205

Ala Cys Glu Val Thr His Gln Gly Leu Ser Ser Pro Val Thr Lys Ser
    210                 215                 220

Phe Asn Arg Gly Glu Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Cys Thr Leu Pro Pro Ser Arg Asp Glu Leu
        355                 360                 365

Thr Lys Asn Gln Val Ser Leu Ser Cys Ala Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Val Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455

<210>  22
<211>  215
<212>  PRT
<213>  Artificial

<220>

<223>  17962 - kappa light chain (LC) <Ang-2> with Q124E substitution

<400>  22

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu Arg Thr Val Ala
            100                 105                 110

Ala Pro Ser Val Phe Ile Phe Pro Pro Ser Asp Glu Glu Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  23
<211>  457
<212>  PRT

<213>  Artificial

<220>
<223>  17935 - heavy chain (HC) <VEGF> with CL-CH1 domain exchange and
      Q124E substitution

<400>  23

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Phe Ser Leu Asp Thr Ser Lys Ser Thr Ala Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Leu Val Thr Val Ser Ser Ala Ser Val Ala Ala
        115                 120                 125

Pro Ser Val Phe Ile Phe Pro Pro Ser Asp Glu Glu Leu Lys Ser Gly
    130                 135                 140

Thr Ala Ser Val Val Cys Leu Leu Asn Asn Phe Tyr Pro Arg Glu Ala
145                 150                 155                 160

Lys Val Gln Trp Lys Val Asp Asn Ala Leu Gln Ser Gly Asn Ser Gln
                165                 170                 175

Glu Ser Val Thr Glu Gln Asp Ser Lys Asp Ser Thr Tyr Ser Leu Ser
            180                 185                 190

Ser Thr Leu Thr Leu Ser Lys Ala Asp Tyr Glu Lys His Lys Val Tyr
        195                 200                 205

Ala Cys Glu Val Thr His Gln Gly Leu Ser Ser Pro Val Thr Lys Ser
    210                 215                 220

Phe Asn Arg Gly Glu Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Cys Thr Leu Pro Pro Ser Arg Asp Glu Leu
        355                 360                 365

Thr Lys Asn Gln Val Ser Leu Ser Cys Ala Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Val Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455

<210>  24
<211>  123

<212>  PRT
<213>  Artificial

<220>
<223>  variable heavy chain domain  <VEGF>

<400>  24


<210>  25
<211>  107
<212>  PRT
<213>  Artificial

<220>
<223>  variable light chain domain  <VEGF>

<400>  25

<210>  26
<211>  129
<212>  PRT
<213>  Artificial

<220>
<223>  variable heavy chain domain <Ang-2>

<400>  26

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Ser Pro Asn Pro Tyr Tyr Tyr Asp Ser Ser Gly Tyr Tyr Tyr
            100                 105                 110

Pro Gly Ala Phe Asp Ile Trp Gly Gln Gly Thr Met Val Thr Val Ser
        115                 120                 125

Ser

<210>  27
<211>  108

<212>  PRT
<213>  Artificial

<220>
<223>  variable light chain domain <Ang-2>

<400>  27

Gln Pro Gly Leu Thr Gln Pro Pro Ser Val Ser Val Ala Pro Gly Gln
1               5                   10                  15

Thr Ala Arg Ile Thr Cys Gly Gly Asn Asn Ile Gly Ser Lys Ser Val
            20                  25                  30

His Trp Tyr Gln Gln Lys Pro Gly Gln Ala Pro Val Leu Val Val Tyr
        35                  40                  45

Asp Asp Ser Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Ser Ser Ser Asp His
                85                  90                  95

Tyr Val Phe Gly Thr Gly Thr Lys Val Thr Val Leu
            100                 105


<210>  28
<211>  219
<212>  PRT
<213>  Artificial

<220>
<223>  6413 - IL17 LC E123 (Wt)/ Q124 (Wt)

<400>  28

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asp Thr Tyr Phe His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile

65                  70                  75                  80

Ser Arg Val Glu Ala Glu Asp Val Gly Val Tyr Tyr Cys Ser Gln Thr
                85                  90                  95

Thr His Ala Pro Phe Thr Phe Gly Gln Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  29
<211>  448
<212>  PRT
<213>  Artificial

<220>
<223>  11738 - IL17 HC K147 (Wt)/ K213(Wt)

<400>  29

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Leu Asp Ser Tyr
            20                  25                  30

Gly Val His Trp Val Arg Gln Ala Thr Gly Lys Gly Leu Glu Trp Val
        35                  40                  45

Ser Val Ile Trp Ser Asp Gly Thr Thr Thr Tyr Asn Ser Ala Leu Lys
    50                  55                  60

Ser Arg Phe Thr Ile Ser Arg Glu Asn Ala Lys Asn Ser Leu Tyr Leu
65                  70                  75                  80

Gln Met Asn Ser Leu Arg Ala Gly Asp Thr Ala Val Tyr Tyr Cys Ala
                85                  90                  95

Arg Asp Thr His Tyr Arg Leu Tyr Tyr Tyr Ala Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Cys Ser Arg Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Lys Thr Tyr Thr Cys Asn Val Asp His
        195                 200                 205

Lys Pro Ser Asn Thr Lys Val Asp Lys Arg Val Glu Ser Lys Tyr Gly
    210                 215                 220

Pro Pro Cys Pro Pro Cys Pro Ala Pro Glu Phe Glu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser Gln Glu Asp Pro
            260                 265                 270

Glu Val Gln Phe Asn Trp Tyr Val Asp Gly Val Glu Val His Asn Ala
        275                 280                 285

Lys Thr Lys Pro Arg Glu Glu Gln Phe Asn Ser Thr Tyr Arg Val Val
    290                 295                 300

Ser Val Leu Thr Val Leu His Gln Asp Trp Leu Asn Gly Lys Glu Tyr

305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Cys Thr Leu
            340                 345                 350

Pro Pro Ser Gln Glu Glu Met Thr Lys Asn Gln Val Ser Leu Ser Cys
        355                 360                 365

Ala Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Val Ser Arg Leu Thr Val Asp Lys Ser
                405                 410                 415

Arg Trp Gln Glu Gly Asn Val Phe Ser Cys Ser Val Met His Glu Ala
            420                 425                 430

Leu His Asn His Tyr Thr Gln Lys Ser Leu Ser Leu Ser Leu Gly Lys
        435                 440                 445

<210>  30
<211>  216
<212>  PRT
<213>  Artificial

<220>
<223>  11755 - Tweak xLC K147(Wt)/ K213(Wt)

<400>  30

Asp Ile Gln Met Thr Gln Ser Pro Ser Ser Val Ser Ala Ser Val Gly
1               5                   10                  15

Asp Arg Val Thr Ile Thr Cys Gln Ala Ser Gln Asn Ile Tyr Ser Asn
            20                  25                  30

Leu Ala Trp Tyr Gln Gln Lys Pro Gly Lys Ala Pro Lys Leu Leu Ile
        35                  40                  45

Tyr Thr Ala Ser Tyr Leu Ala Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Thr Ala Tyr Tyr Asn Ser Arg
                85                  90                  95

Pro Asp Thr Val Ala Phe Gly Gly Gly Thr Lys Val Glu Ile Lys Ser
            100                 105                 110

Ser Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly
    210                 215

<210>  31
<211>  451
<212>  PRT
<213>  Artificial

<220>
<223>  11754 - Tweak xHC E123(Wt)/ Q124(Wt)

<400>  31

Gln Val Gln Leu Val Gln Ser Gly Ala Glu Val Lys Lys Pro Gly Ser
1               5                   10                  15

Ser Val Lys Val Ser Cys Lys Ala Ser Gly Phe Asp Phe Ser Thr Tyr
            20                  25                  30

Tyr Met Ser Trp Val Arg Gln Ala Pro Gly Gln Gly Leu Glu Trp Met
        35                  40                  45

Gly Thr Val Tyr Val Arg Gln Gly Thr Thr Tyr Tyr Ala Ser Trp Leu
    50                  55                  60

Asn Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Asn Tyr Asp Asp Ala Phe Val Ile Trp Gly Gln
            100                 105                 110

Gly Thr Leu Val Thr Val Ser Ser Ala Ser Val Ala Ala Pro Ser Val
        115                 120                 125

Phe Ile Phe Pro Pro Ser Asp Glu Gln Leu Lys Ser Gly Thr Ala Ser
    130                 135                 140

Val Val Cys Leu Leu Asn Asn Phe Tyr Pro Arg Glu Ala Lys Val Gln
145                 150                 155                 160

Trp Lys Val Asp Asn Ala Leu Gln Ser Gly Asn Ser Gln Glu Ser Val
                165                 170                 175

Thr Glu Gln Asp Ser Lys Asp Ser Thr Tyr Ser Leu Ser Ser Thr Leu
            180                 185                 190

Thr Leu Ser Lys Ala Asp Tyr Glu Lys His Lys Val Tyr Ala Cys Glu
        195                 200                 205

Val Thr His Gln Gly Leu Ser Ser Pro Val Thr Lys Ser Phe Asn Arg
    210                 215                 220

Gly Glu Cys Pro Pro Cys Pro Pro Cys Pro Ala Pro Glu Phe Glu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser Gln
            260                 265                 270

Glu Asp Pro Glu Val Gln Phe Asn Trp Tyr Val Asp Gly Val Glu Val
        275                 280                 285

His Asn Ala Lys Thr Lys Pro Arg Glu Glu Gln Phe Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Gly Leu Pro Ser Ser Ile
                325                 330                 335

Glu Lys Thr Ile Ser Lys Ala Lys Gly Gln Pro Arg Glu Pro Gln Val
            340                 345                 350

Tyr Thr Leu Pro Pro Cys Gln Glu Glu Met Thr Lys Asn Gln Val Ser
        355                 360                 365

Leu Trp Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Arg Leu Thr Val
                405                 410                 415

Asp Lys Ser Arg Trp Gln Glu Gly Asn Val Phe Ser Cys Ser Val Met
            420                 425                 430

His Glu Ala Leu His Asn His Tyr Thr Gln Lys Ser Leu Ser Leu Ser
        435                 440                 445

Leu Gly Lys
    450

<210>  32
<211>  219
<212>  PRT
<213>  Artificial

<220>
<223>  17843 - IL17 LC Q124K

<400>  32

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asp Thr Tyr Phe His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Thr
                85                  90                  95

Thr His Ala Pro Phe Thr Phe Gly Gln Gly Thr Lys Leu Glu Ile Lys
            100                 105                 110

Arg Thr Val Ala Ala Pro Ser Val Phe Ile Phe Pro Pro Ser Asp Glu
        115                 120                 125

Lys Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  33
<211>  448
<212>  PRT
<213>  Artificial

<220>
<223>  17844 - IL17 HC K147E

<400>  33

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Leu Asp Ser Tyr
            20                  25                  30

Gly Val His Trp Val Arg Gln Ala Thr Gly Lys Gly Leu Glu Trp Val
        35                  40                  45

Ser Val Ile Trp Ser Asp Gly Thr Thr Thr Tyr Asn Ser Ala Leu Lys
    50                  55                  60

Ser Arg Phe Thr Ile Ser Arg Glu Asn Ala Lys Asn Ser Leu Tyr Leu
65                  70                  75                  80

Gln Met Asn Ser Leu Arg Ala Gly Asp Thr Ala Val Tyr Tyr Cys Ala
                85                  90                  95

Arg Asp Thr His Tyr Arg Leu Tyr Tyr Tyr Ala Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Cys Ser Arg Ser Thr Ser Glu Ser Thr Ala
    130                 135                 140

Ala Leu Gly Cys Leu Val Glu Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Lys Thr Tyr Thr Cys Asn Val Asp His
        195                 200                 205

Lys Pro Ser Asn Thr Lys Val Asp Lys Arg Val Glu Ser Lys Tyr Gly
    210                 215                 220

Pro Pro Cys Pro Pro Cys Pro Ala Pro Glu Phe Glu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser Gln Glu Asp Pro
            260                 265                 270

Glu Val Gln Phe Asn Trp Tyr Val Asp Gly Val Glu Val His Asn Ala
        275                 280                 285

Lys Thr Lys Pro Arg Glu Glu Gln Phe Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Cys Thr Leu
            340                 345                 350

Pro Pro Ser Gln Glu Glu Met Thr Lys Asn Gln Val Ser Leu Ser Cys
        355                 360                 365

Ala Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Val Ser Arg Leu Thr Val Asp Lys Ser
                405                 410                 415

Arg Trp Gln Glu Gly Asn Val Phe Ser Cys Ser Val Met His Glu Ala
            420                 425                 430

Leu His Asn His Tyr Thr Gln Lys Ser Leu Ser Leu Ser Leu Gly Lys
        435                 440                 445

<210>  34
<211>  448
<212>  PRT
<213>  Artificial

<220>
<223>  17845 - IL17 HC K213E

<400>  34

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Leu Asp Ser Tyr
            20                  25                  30

Gly Val His Trp Val Arg Gln Ala Thr Gly Lys Gly Leu Glu Trp Val
        35                  40                  45

Ser Val Ile Trp Ser Asp Gly Thr Thr Thr Tyr Asn Ser Ala Leu Lys
    50                  55                  60

Ser Arg Phe Thr Ile Ser Arg Glu Asn Ala Lys Asn Ser Leu Tyr Leu
65                  70                  75                  80

Gln Met Asn Ser Leu Arg Ala Gly Asp Thr Ala Val Tyr Tyr Cys Ala
                85                  90                  95

Arg Asp Thr His Tyr Arg Leu Tyr Tyr Tyr Ala Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Cys Ser Arg Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Lys Thr Tyr Thr Cys Asn Val Asp His
        195                 200                 205

Lys Pro Ser Asn Thr Lys Val Asp Glu Arg Val Glu Ser Lys Tyr Gly
    210                 215                 220

Pro Pro Cys Pro Pro Cys Pro Ala Pro Glu Phe Glu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser Gln Glu Asp Pro
            260                 265                 270

Glu Val Gln Phe Asn Trp Tyr Val Asp Gly Val Glu Val His Asn Ala
        275                 280                 285

Lys Thr Lys Pro Arg Glu Glu Gln Phe Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Cys Thr Leu
            340                 345                 350

Pro Pro Ser Gln Glu Glu Met Thr Lys Asn Gln Val Ser Leu Ser Cys
        355                 360                 365

Ala Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Val Ser Arg Leu Thr Val Asp Lys Ser
                405                 410                 415

Arg Trp Gln Glu Gly Asn Val Phe Ser Cys Ser Val Met His Glu Ala
            420                 425                 430

Leu His Asn His Tyr Thr Gln Lys Ser Leu Ser Leu Ser Leu Gly Lys
        435                 440                 445

<210> 35
<211> 219
<212> PRT
<213> Artificial

<220>
<223> 17846 - IL17 LC E123K

<400> 35

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asp Thr Tyr Phe His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Thr
                85                  90                  95

Thr His Ala Pro Phe Thr Phe Gly Gln Gly Thr Lys Leu Glu Ile Lys
            100                 105                 110

Arg Thr Val Ala Ala Pro Ser Val Phe Ile Phe Pro Pro Ser Asp Lys
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  36
<211>  219
<212>  PRT
<213>  Artificial

<220>
<223>  17847 - IL17 LC E123R / Q124K

<400>  36

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asp Thr Tyr Phe His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile

65                  70                  75                  80

Ser Arg Val Glu Ala Glu Asp Val Gly Val Tyr Tyr Cys Ser Gln Thr
                85                  90                  95

Thr His Ala Pro Phe Thr Phe Gly Gln Gly Thr Lys Leu Glu Ile Lys
            100                 105                 110

Arg Thr Val Ala Ala Pro Ser Val Phe Ile Phe Pro Pro Ser Asp Lys
        115                 120                 125

Arg Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  37
<211>  448
<212>  PRT
<213>  Artificial

<220>
<223>  17848 - IL17 HC K147E / K213E

<400>  37

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Leu Asp Ser Tyr
            20                  25                  30

Gly Val His Trp Val Arg Gln Ala Thr Gly Lys Gly Leu Glu Trp Val
        35                  40                  45

Ser Val Ile Trp Ser Asp Gly Thr Thr Thr Tyr Asn Ser Ala Leu Lys
    50                  55                  60

Ser Arg Phe Thr Ile Ser Arg Glu Asn Ala Lys Asn Ser Leu Tyr Leu
65                  70                  75                  80

Gln Met Asn Ser Leu Arg Ala Gly Asp Thr Ala Val Tyr Tyr Cys Ala
                85                  90                  95

Arg Asp Thr His Tyr Arg Leu Tyr Tyr Tyr Ala Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Cys Ser Arg Ser Thr Ser Glu Ser Thr Ala
    130                 135                 140

Ala Leu Gly Cys Leu Val Glu Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Lys Thr Tyr Thr Cys Asn Val Asp His
        195                 200                 205

Lys Pro Ser Asn Thr Lys Val Asp Glu Arg Val Glu Ser Lys Tyr Gly
    210                 215                 220

Pro Pro Cys Pro Pro Cys Pro Ala Pro Glu Phe Glu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser Gln Glu Asp Pro
            260                 265                 270

Glu Val Gln Phe Asn Trp Tyr Val Asp Gly Val Glu Val His Asn Ala
        275                 280                 285

Lys Thr Lys Pro Arg Glu Glu Gln Phe Asn Ser Thr Tyr Arg Val Val
    290                 295                 300

Ser Val Leu Thr Val Leu His Gln Asp Trp Leu Asn Gly Lys Glu Tyr

305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Cys Thr Leu
            340                 345                 350

Pro Pro Ser Gln Glu Glu Met Thr Lys Asn Gln Val Ser Leu Ser Cys
        355                 360                 365

Ala Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Val Ser Arg Leu Thr Val Asp Lys Ser
                405                 410                 415

Arg Trp Gln Glu Gly Asn Val Phe Ser Cys Ser Val Met His Glu Ala
            420                 425                 430

Leu His Asn His Tyr Thr Gln Lys Ser Leu Ser Leu Ser Leu Gly Lys
        435                 440                 445

<210>  38
<211>  448
<212>  PRT
<213>  Artificial

<220>
<223>  17849 - IL17 HC K147E / K213D

<400>  38

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Leu Asp Ser Tyr
            20                  25                  30

Gly Val His Trp Val Arg Gln Ala Thr Gly Lys Gly Leu Glu Trp Val
        35                  40                  45

Ser Val Ile Trp Ser Asp Gly Thr Thr Thr Tyr Asn Ser Ala Leu Lys
    50                  55                  60

Ser Arg Phe Thr Ile Ser Arg Glu Asn Ala Lys Asn Ser Leu Tyr Leu
65                  70                  75                  80

Gln Met Asn Ser Leu Arg Ala Gly Asp Thr Ala Val Tyr Tyr Cys Ala
                85                  90                  95

Arg Asp Thr His Tyr Arg Leu Tyr Tyr Tyr Ala Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Cys Ser Arg Ser Thr Ser Glu Ser Thr Ala
    130                 135                 140

Ala Leu Gly Cys Leu Val Glu Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Lys Thr Tyr Thr Cys Asn Val Asp His
        195                 200                 205

Lys Pro Ser Asn Thr Lys Val Asp Asp Arg Val Glu Ser Lys Tyr Gly
    210                 215                 220

Pro Pro Cys Pro Pro Cys Pro Ala Pro Glu Phe Glu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser Gln Glu Asp Pro
            260                 265                 270

Glu Val Gln Phe Asn Trp Tyr Val Asp Gly Val Glu Val His Asn Ala
        275                 280                 285

Lys Thr Lys Pro Arg Glu Glu Gln Phe Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr

325                 330                 335

Ile Ser Lys Ala Lys Gly Gln Pro Arg Glu Pro Gln Val Cys Thr Leu
            340                 345                 350

Pro Pro Ser Gln Glu Glu Met Thr Lys Asn Gln Val Ser Leu Ser Cys
        355                 360                 365

Ala Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Val Ser Arg Leu Thr Val Asp Lys Ser
                405                 410                 415

Arg Trp Gln Glu Gly Asn Val Phe Ser Cys Ser Val Met His Glu Ala
            420                 425                 430

Leu His Asn His Tyr Thr Gln Lys Ser Leu Ser Leu Ser Leu Gly Lys
        435                 440                 445

<210>  39
<211>  451
<212>  PRT
<213>  Artificial

<220>
<223>  17850 - Tweak xHC Q124E

<400>  39

Gln Val Gln Leu Val Gln Ser Gly Ala Glu Val Lys Lys Pro Gly Ser
1               5                   10                  15

Ser Val Lys Val Ser Cys Lys Ala Ser Gly Phe Asp Phe Ser Thr Tyr
            20                  25                  30

Tyr Met Ser Trp Val Arg Gln Ala Pro Gly Gln Gly Leu Glu Trp Met
        35                  40                  45

Gly Thr Val Tyr Val Arg Gln Gly Thr Thr Tyr Tyr Ala Ser Trp Leu
    50                  55                  60

Asn Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Tyr Asn Tyr Asp Asp Ala Phe Val Ile Trp Gly Gln
            100                 105                 110

Gly Thr Leu Val Thr Val Ser Ser Ala Ser Val Ala Ala Pro Ser Val
        115                 120                 125

Phe Ile Phe Pro Pro Ser Asp Glu Glu Leu Lys Ser Gly Thr Ala Ser
    130                 135                 140

Val Val Cys Leu Leu Asn Asn Phe Tyr Pro Arg Glu Ala Lys Val Gln
145                 150                 155                 160

Trp Lys Val Asp Asn Ala Leu Gln Ser Gly Asn Ser Gln Glu Ser Val
                165                 170                 175

Thr Glu Gln Asp Ser Lys Asp Ser Thr Tyr Ser Leu Ser Ser Thr Leu
            180                 185                 190

Thr Leu Ser Lys Ala Asp Tyr Glu Lys His Lys Val Tyr Ala Cys Glu
        195                 200                 205

Val Thr His Gln Gly Leu Ser Ser Pro Val Thr Lys Ser Phe Asn Arg
    210                 215                 220

Gly Glu Cys Pro Pro Cys Pro Pro Cys Pro Ala Pro Glu Phe Glu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser Gln
            260                 265                 270

Glu Asp Pro Glu Val Gln Phe Asn Trp Tyr Val Asp Gly Val Glu Val
        275                 280                 285

His Asn Ala Lys Thr Lys Pro Arg Glu Glu Gln Phe Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Gly Leu Pro Ser Ser Ile
                325                 330                 335

Glu Lys Thr Ile Ser Lys Ala Lys Gly Gln Pro Arg Glu Pro Gln Val

            340                 345                 350

Tyr Thr Leu Pro Pro Cys Gln Glu Glu Met Thr Lys Asn Gln Val Ser
        355                 360                 365

Leu Trp Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Arg Leu Thr Val
                405                 410                 415

Asp Lys Ser Arg Trp Gln Glu Gly Asn Val Phe Ser Cys Ser Val Met
            420                 425                 430

His Glu Ala Leu His Asn His Tyr Thr Gln Lys Ser Leu Ser Leu Ser
        435                 440                 445

Leu Gly Lys
    450